# EUROPEAN PATENT APPLICATION

(11) **EP 4 657 067 A2**
(43) Date of publication of application: **03.12.2025**
(21) Application number: 25198633.7
(22) Date of filing: 15.11.2019
(51) Int. Cl.: G01N 33/50

(54) **MOLECULES AND METHODS FOR IMPROVED IMMUNODETECTION OF SMALL MOLECULES, SUCH AS HISTAMINE**

(30) Priority: 16.11.2018 US 201862768479 P
(62) Divisional of application: 19883655.3
(71) Applicant: President And Fellows Of Harvard College, Cambridge, Massachusetts 02138 (US)
(72) Inventor: JOLLY, Pawan, Boston, 02215 (US); MUSTAFAOGLU, Nur, Boston, 02120 (US); HENRY, Olivier Yves Frederic, 29900 Conconmam (FR); INGBER, Donald E., Boston, 02118 (US)
(74) Representative: Brand Murray Fuller LLP

(57) **Abstract**

Embodiments of various aspects described herein are directed to methods, compositions, kits for detecting a target molecule in a sample. In particular, there is described herein a multivalent approach which provides an efficient method for detection of small molecules and screening of binding molecules (e.g., antibodies). The multivalent approach uses two or more small molecules in a domain that is attached to a substrate through a linking group. The multivalent domain is free to extend, e.g., into a solution, for presentation to a binding compound such as an antibody.

## Description

### RELATED APPLICATIONS

This application claims benefit under 35 U.S.C. § 119(e) of the U.S. Provisional Application No. 62/768,479 filed November 16, 2019, the content of which is incorporated herein by reference in its entirety.

### TECHNICAL FIELD

Described herein relates generally to methods, compositions, and kits for detecting a target entity in a sample. In some embodiments, methods and compositions for detecting small molecules in a test sample, including bodily fluids such as blood and tissues of a subject, food, water, and environmental surfaces are also provided herein.

### BACKGROUND

Detection of small molecules (< 1000 Da) is a challenging field because it is difficult for an antibody to recognize and bind to a small number of functional groups. These antibodies are typically raised by conjugating the small molecule target to a larger protein carrier followed by inoculation of the animal. Such an approach has been found to be successful for a number of targets that contain multiple functional groups (for example cortisol, testosterone). However, very small molecules, such as histamine (111.14 g/mol), which consists of an imidazole ring and a short carbon chain terminated with a primary amine, still pose a challenge due in part to their limited functionalities. This is an important challenge because antibody-based diagnostics that can detect small molecules such as histamine with high specificity and sensitivity have great potential value for medical diagnostics (e.g., for allergy and anaphylaxis), early detection of diseases, and food safety applications.

Antibodies against small molecules such as histamine are typically raised by conjugating the small molecule to a large immunogenic protein carrier, such as bovine serum albumin (BSA) or ovalbumin (OVA). Consequently, only a portion of the small molecules will be exposed to the lymphocytes, which commonly results in the generation of antibodies that specifically recognizes the protein-bound small molecule and not the free floating small molecule. For example, in the case of histamine, only the imidazole will be exposed to lymphocytes, which results in generation of protein-bound histamine specific antibodies having only limited affinity and sensitivity for free histamine. These antibodies typically perform poorly in the development of immunoassays for the target small molecule released in a free form from tissues, which is often most clinically relevant. Thus, there is a need to design ways to overcome this lack of specificity of currently available antibodies targeted to small molecules.

Most of the studies on histamine detection are based on the use of either protein-conjugated histamine molecule bound via its primary amine group, or chemically modified histamine, both for antibody development and as specific competitive inhibitors of binding in immunoassays. For instance, Morel *et al.* developed antibodies using chemical derivatization where an acylating reagent was synthesized to raise monoclonal antibodies against acylated histamine [Morel, A.M. and Delaage, M.A., 1988, "Immunoanalysis of histamine through a novel chemical derivatization," Journal of allergy and clinical immunology, 82(4), pp.646-654]. As a result, the antibodies produced showed greater affinity towards the derivatized histamine than free histamine. Buckler et al. describe various type of histamine derivatives for antibody production [Buckler, et al., U.S. Patent 5,112,738]. Nearly all of the haptens presented in this publication involves different ways to attach the histamine molecule (either from the carbon tail or the imidazole ring) to a carrier or a terminal functional group. The study showed that a hapten produced by conjugating histamine to a protein carrier was the most efficient way to produce monoclonal antibodies against histamine. More recently, Mattsson *et al.* presented a detailed study on the development of a histamine assay using commercial antibodies [Mattsson, L., Doppler, S. and Preininger, C., 2017, "Challenges in Developing a Biochip for Intact Histamine Using Commercial Antibodies," Chemosensors, 5(4), p.33*.*]*.* The research group tested six commercial antibodies out of which only two showed affinities towards free histamine. However, even these two antibodies demonstrated poor sensitivity in the µg/mL range for the histamine molecule.

In addition to low sensitivity and selectivity, the reported methods are inefficient, often requiring incubation times of more than an hour to evaluate the presence of small molecules. Hense, there remains a need for the development of more sensitive immunoassays and surpass the limitations of currently available inefficient low affinity small molecule antibodies. There also is a need to generate antibodies that are specifically designed to recognize the free (non-conjugated) form of the small molecule. Importantly, there is a need for molecular design approaches that can be used to both generate more specific antibodies and create more specific binding assays with available antibodies for small molecule targets.

### SUMMARY

Embodiments of various aspects described herein, include development of a molecular design approach that can be used to both select more specific antibodies and create more specific and efficient binding assays with available antibodies for small molecule targets. This approach also can be used to generate antibodies that are specifically designed to recognize the free (non-conjugated) form of the small molecule. Some embodiments inclue a novel competitor molecule that can be used to develop more sensitive immunoassays and surpass the limitations of currently available low affinity anti-histamine antibodies.

In one aspect, provided herein is a compound comprising (i) a substrate binding domain; (ii) a branching domain comprising a plurality of small molecules each small molecule linked to a branch of a branch-point; and (iii) a linker linking the substrate binding domain and the branching domain. Optionally the small molecules independently have a molecular weight of between 50 and 600 g/mol (Da), such a molecular weight less than 1000 Da. In some embodiments the small molecule is selected from the group consisting of amino acids, amino acid dimers, nucleosides, saccharides, steroids, hormones, pharmaceutically derived drugs, or derivatives and conjugates thereof. For example, the small molecule can be selected from histidine, a histadine-phenylalanine dimer, or dinitrophenol (DNP). Optionally the branching domain comprises from 2 to 20 small molecules linked to the branch-point. In some embodiments the linker comprises a polyethylene glycol (PEG) having a molecular weight of less than about 2000 Da (e.g., less than about 1,000 Da). Optionally the linker comprises PEG having from 2 to about 45 repeat units (e.g., between about 2 to 20 repeat units, between about 4 and 10 repeat units, between 4 to 6 repeat units).

In some embodiments the branch-point comprises at least one lysine and optionally at least one small molecule is linked to the alpha-amino group of the at least one lysine and at least one small molecule is linked to the epsilon-amino group of the at least one lysine. In some embodiments, the branch-point comprises a first lysine linked to a second lysine, and wherein the carboxyl group of the first lysine is linked to the epsilon-amino group of second lysine. In some embodiments, the branch-point comprises a first lysine, a second lysine and a third lysine, and wherein the carboxyl group of the first lysine is linked to the epsilon-amino group of the second lysine, and the carboxyl group of the third lysine is linked to the alpha-amino group of the first or second lysine. In some embodiments, the branch-point comprises n lysines, wherein n is an integer greater than three (e.g., between about 3 and 100), wherein the carboxyl group of a first lysine (n=1 lysine) is linked to the epsilon-amino group of a second lysine (n=2 lysine), the carboxyl group of a third lysine (n=3 lysine) is linked to the alpha-amino group of the first or second lysine, the carboxyl group of the fourth lysine is linked to the alpha-amino group of the first, second, or third lysine, and the carboxylic group of the n^{th} lysine is linked to the alpha-amino group any one of the lysines up to the (n-1)^{th} lysine. Optionally, a small molecule can be linked to any available amino group in the branch point comprising lysine(s).

In some embodiments, the branch-point is selected from the group consisting of: 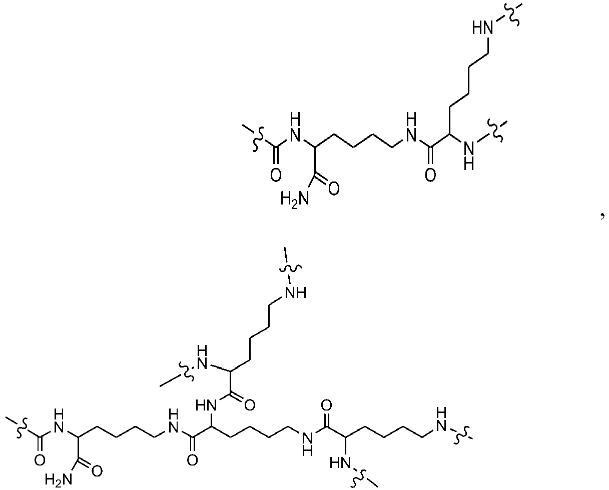 , and

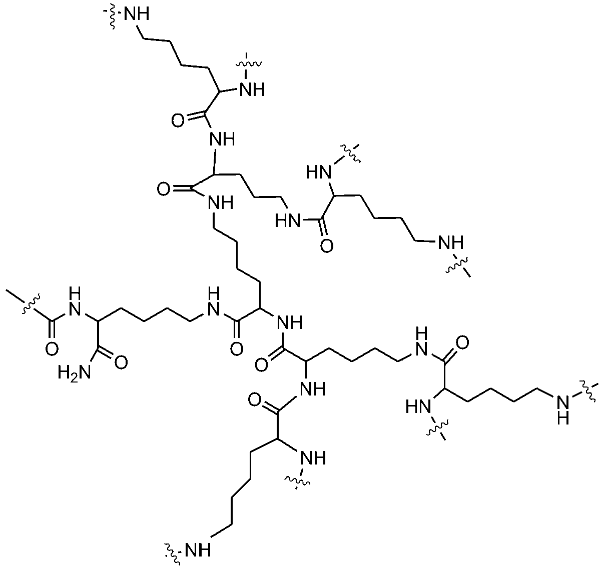

In some embodiments the branching domain comprises: 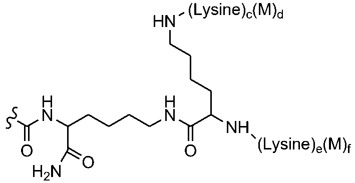 wherein, d + f ≥ 2 (e.g., between about 2 and 100), d ≥ c, and e ≥ f, wherein c, d, e and f are integers and each M is a small molecule such as histidine or dinitrophenol.
In some embodiments the branching domain is selected from the group consisting of: 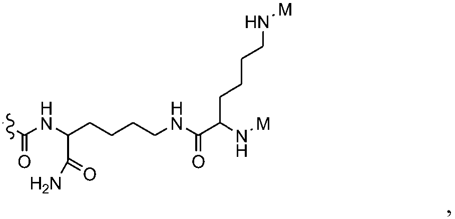 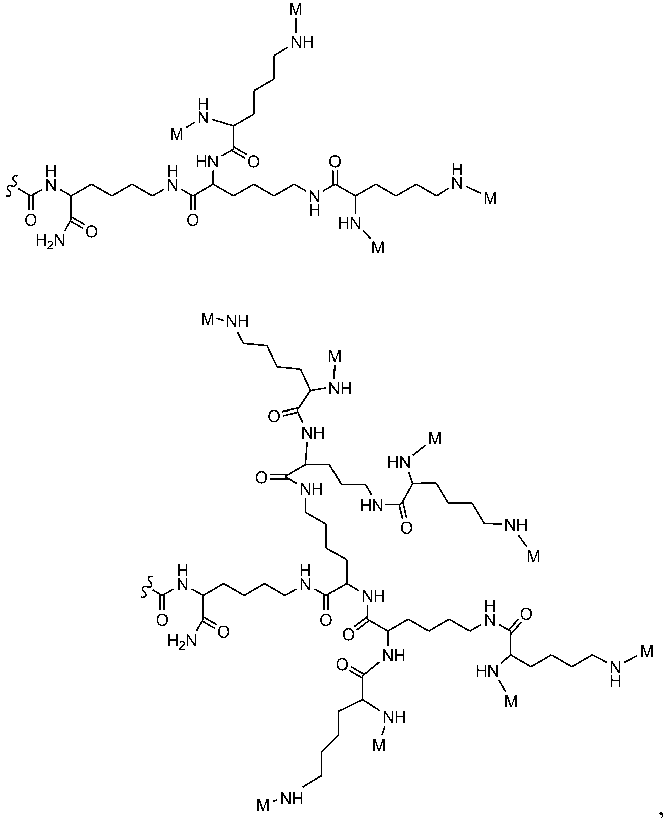 , and wherein each M is a small molecule. Optionally, the branching domain is selected from the group consisting of: 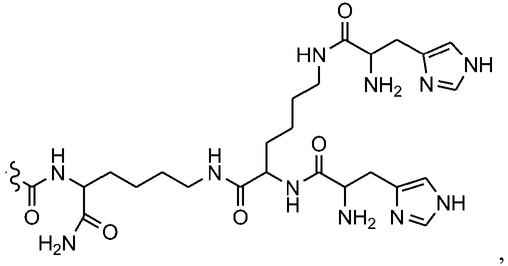 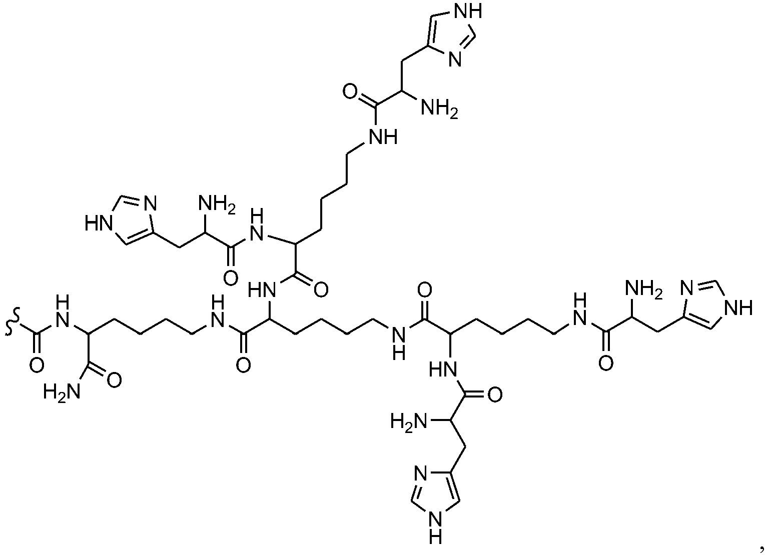 and

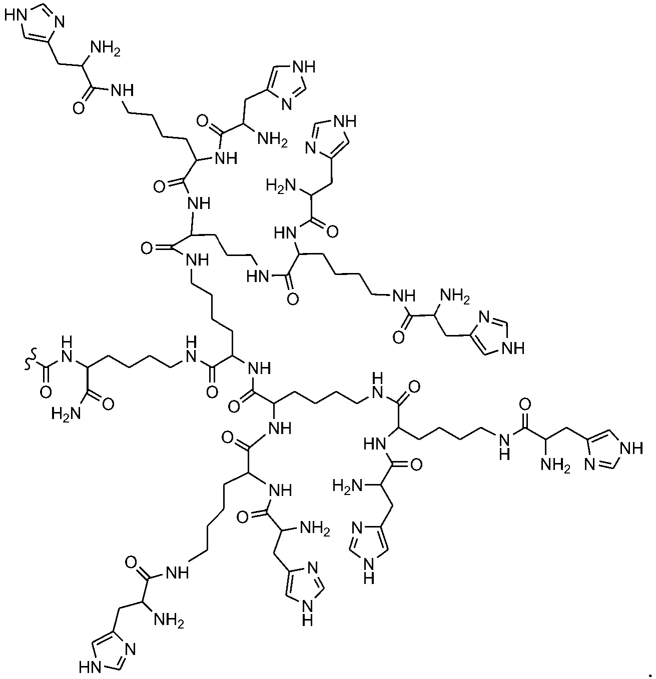

In some embodiments the branching domain is selected from the group consisting of: 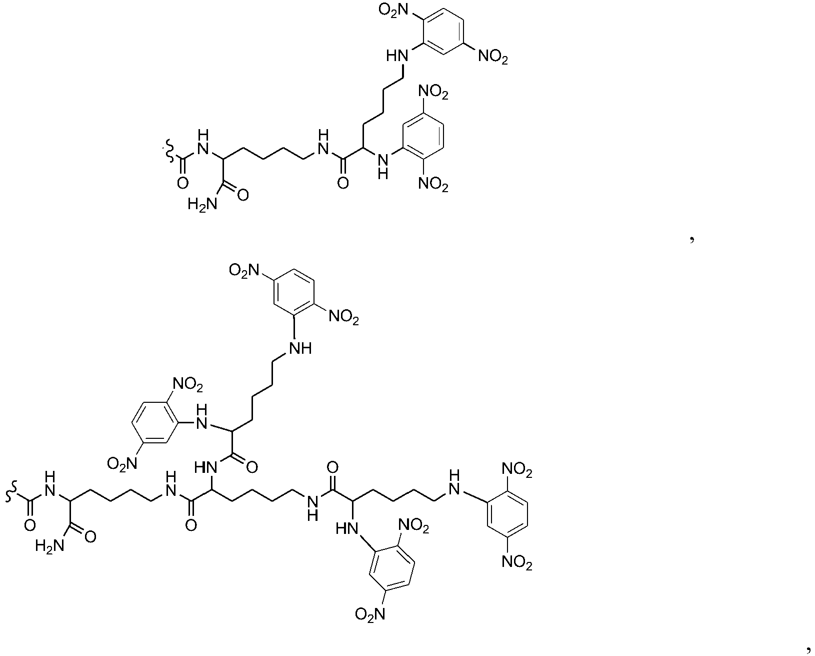 and

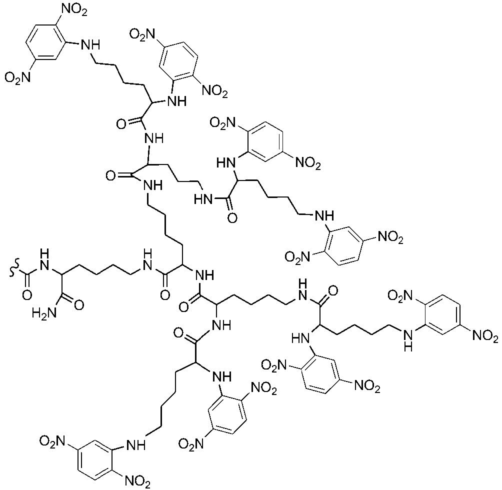

In some embodiments, the branching domain has the formula C(x)ₐM_{b}, wherein: C is a sub unit of the branching domain having a maximum of possible x branches, and the branch-point comprises one or more sub unit C and at least one subunit C is attached to the linker through a branch; M is a small molecule attached to the subunit C through a branch; a is an integer ≥ 1; and b is an integer ≥2, provided that b≤ (a)(x-2) +1.

In some embodiments the substrate binding domain comprises a reactive group or one member of a binding pair. As used herein a "reactive group" relates to moiety, such as a functional group or part of a molecule of a first member of a binding pair that can form a bond to a complementary moiety of a second member of a binding pair. The bond can be any kind of bond including one or more of a covalent, ionic, hydrophobic, hydrogen or dative bond. For example, optionally the reactive group is selected from the group consisting of alkyl halide, aldehyde, amino, bromo or iodoacetyl, carboxyl, hydroxyl, epoxy, ester, silane, thiol, and the like. Optionally the binding pair is biotin-avidin, biotin-streptavidin, complementary oligonucleotide pairs capable of forming nucleic acid duplexes, a thiol-maleimide pair, a first molecule that is negatively charged and a second molecule that is positively charged. Optionally the substrate binding domain comprises a thiol group or a biotin molecule.

In some embodiments the compound is linked to a substrate via the substrate binding domain. Optionally the substrate is a nucleic acid scaffold, a protein scaffold, a lipid scaffold, a dendrimer, a microparticle or a microbead, a nanotube, a microtiter plate, an electrode, a medical apparatus or implant, a microchip, a filtration device, a membrane, a diagnostic strip, a dipstick, an extracorporeal device, a microscopic slide, a hollow fiber, a hollow fiber cartridge, an electrode surface or any combinations thereof. For example, optionally the substrate is a microparticle or a microbead, a microtiter plate, an electrode surface, a membrane, a diagnostic strip, a dipstick, an ELISA plate, or a microscopic slide. Optionally, the substrate is a cross-linked and denatured protein, for example, wherein the denatured protein is a denatured BSA which is cross-linked with glutaraldehyde.

In some embodiments, the substrate is an ELISA plate. In some embodiments, a surface of the ELISA plate can be coated to reduce or inhibit nonspecific binding. Without limitations, such a coating can also allow for high concentration of the compounds described herein. Any coating known in the art for reducing or inhibiting non-specific binding of molecule to a surface can be used. In some embodiments an antibody is attached to an ELISA plate and the detecting molecule is attached to a particle, a detectable label, or a particle with a detectable label. For example, a small molecule specific antibody is attached to the ELISA plate and the detecting molecule includes a small molecule (e.g., linked to a branch of a branch point).

In some embodiments, the ELISA plate comprises a proteinaceous material coated on at least a part of a surface of the ELISA plate. The proteinaceous material can be reversibly or non-reversibly denatured. In some embodiments, the proteinaceous material can be non-reversibly denatured. Optinally, the proteinaceous material can be cross-linked. For example, the proteinaceous material can be cross-linked with glutaraldehyde. In some embodiments, the surface of the ELISA plate is at least partially coated with BSA, which can be reversibly or non-reversibly denatured and/or cross-linked. In some embodiments, the ELISA plate comprises a mixture of a particulate material and a proteinaceous material coated on at least a part of a surface of the ELISA plate.

Another aspect provided herein relates to a method for detecting presence of an analyte in a sample, the method comprising: (i) contacting a sample suspected of comprising an analyte with the compound comprising a substrate binding domain, a branching domain and linker; and (ii) detecting binding of an analyte binding molecule to the compound of claim 1. Optionally the analyte binding molecule is an antibody. Optionally the detecting of step (ii) comprises producing a chromogenic, fluorescence or electrochemical signal. Also optionally the analyte binding molecule comprises a detectable label. Optionally the detecting step comprises contacting the sample from (i) with a molecule capable of binding with the analyte binding molecule and comprises a detectable label. Optionally the analyte is a small molecule such as histamine or dinitrophenol.

Another aspect relates to methods wherein the compound comprising a binding domain, a branching domain and a linker; is used in an electrochemical method for detecting presence of an analyte in a sample. In these embodiments the compound can be linked to an electrode surface by the substrate binding domain and the analyte binding molecule includes an electroactive component. The analyte binding molecule is detected by the electrode when the electroactive component is proximate to the electrode. Optionally the electrode detects the analyte binding molecule by direct redox reaction with the electroactive component or by a sacrificial redox active species. In alternative embodiments the analyte binding molecule is an antibody specific to the analyte, and the electroactive component is a biotinylated detection antibody conjugated to streptavidin-polyHRP and the electrode detects the sacrificial redox active agent 3,3',5,5'-Tetramethylbenzidine (TMB).

In another aspect, there is provided a method for selecting a ligand capable of binding a small molecule comprising; (i) contacting a test ligand with the compound comprising a binding domain, a branching domain and a linker, and (ii) detecting binding of the test ligand with the compound of claim 1 in the presence and in the absence of the small molecule, and selecting the test ligand having reduced binding in the presence of the small molecule. Optionally the test ligand is selected from the group consisting of antibodies, adnectins, ankyrins, antibody mimetics and other protein scaffolds, aptamers, nucleic acid (e.g., an RNA or DNA aptamer), proteins, peptides, oligosaccharides, polysaccharides, lipopolysaccharides, cellular metabolites, cells, viruses, subcellular particles, haptens, pharmacologically active substances, alkaloids, steroids, vitamins, amino acids, avimers, peptidomimetics, hormone receptors, cytokine receptors, synthetic receptors, sugars and molecularly imprinted polymer. For example, the test ligand can be an antibody. Optionally the detecting of step (ii) comprises producing a chromogenic, fluorescence or electrochemical signal. Also optionally the ligand comprises a detectable label, for example wherein the detectable label is a chromogenic, fluorescent or redox active group. Optionally the detecting step comprises contacting the ligand from step (i) with a molecule capable of binding with the ligand and comprises a detectable label.

Other aspects include a method for raising antibodies specific to a small molecule, the method comprising contacting T cells with the compound comprising a binding domain, a branching domain and a linker.

### BRIEF DESCRIPTION OF THE DRAWINGS

This patent or application file contains at least one drawing executed in color. Copies of this patent or patent application publication with color drawings will be provided by the Office upon request and payment of the necessary fee.
**FIG. 1** is a representative drawing of a multivalent detecting molecule on a protein carrier.
**FIG. 2** shows a schematic of an ELISA protocol for BSA-Histamine Conjugate surface chemistry
**FIG. 3A** shows a bar graph for the screening of antibodies on surfaces modified with BSA-histamine and BSA only. **FIG. 3B** shows a bar graph for a competitive assay performed on surfaces modified with BSA-histamine and BSA only in the presence of 271 nM free histamine.
**FIG. 4** is a representative drawing of a Histamine-Protein carrier conjugate.
**FIG. 5A** and **5B** are the results from an optimization study for the development of competitive assay using BSA-histamine conjugate surface chemistry. **FIG. 5A** is a bar graph showing the effect of changing BSA-histamine conjugate concentration on the signal change with 271.6 nM free histamine. **FIG. 5B** The effect of anti-histamine antibody by keeping BSA-histamine conjugate concentration constant (0.02 µg/mL) on the signal change with 271.6 nM free histamine.
**FIG. 6** is a semi-log plot showing a calibration curve with optimized BSA-Histamine and Anti-Histamine antibody.
**FIG. 7A** shows a conjugate synthesized using histamine as compared to **FIG. 7B** which show a conjugate synthesized using histidine, where R a linker and X is a functional end group.
**FIG. 8** shows a schematic of ELISA protocol with PEG-linkers.
**FIG. 9** shows a bar graph for the screening of antibodies with BSA histamine and PEG-mono-histidine.
**FIG. 10** shows a plot of the data from a competitive assay obtained with BSA-histamine and PEG-histidine.
**FIG. 11** shows a semi-log plot of the data from a competitive assay obtained with PEG-mono histidine and PEG-dual histidine.
**FIG. 12A** shows a schematic structure of Electrochemical Biosensor for Histamine. **FIG. 12B** is a plot showing a calibration curve obtained using electrochemical chips.
**FIG. 13** shows a schematic for the synthesis of Biotin-PEG-mono-histamine.
**FIG. 14** shows a schematic for the synthesis of Biotin-PEG-mono-histidine.
**FIG. 15** shows a semi-log plot for comparison data of PEG-mono histamine and PEG-mono histidine using streptavidin coated ELISA plates for the detection of histamine.
**FIG. 16** shows plotted data of the effect of anti-histamine antibody incubation times on assay absorbance.
**FIG. 17A** shows plotted data for a comparison of multivalent linkers using absolute absorbance values. **FIG. 17B** shows the same information with normalized absorbance values.
**FIG. 18A** shows plotted data for a comparison of PEG-Mono histidine and PEG-Dual histidine using absolute absorbance values. **FIG. 18B** shows the same information with normalized absorbance values.
**FIG. 19** shows a schematic for an assay design using direct anti-histamine antibody labelled with HRP.
**FIG. 20A** shows a semi-log plot for histidine using a PEG-mono-histidine conjugate. **FIG. 20B** shows a semi-log plot for histidine using both a PEG-mono histidine and PEG-Dual histidine conjugate.
**FIG. 21** shows a semi-log plot of fitted calibration curve data points using a Hill equation.
**FIG. 22** shows a schematic for an assay protocol using a BSA-PEG-mono-Histidine conjugate.
**FIG. 23** shows a semi-log plot of calibration data for a histidine 5 min assay using BSA modified with PEG-mono Histidine and anti-histamine antibody labelled with HRP.
**FIG. 24** shows a semi-log plot of calibration data obtained using electrochemical chips using PEG-Dual histidine linker in spiked plasma.
**FIG. 25** is a plot showing ELISA results with Mono-DNP linker (blue) versus PEG-Dual- DNP linker (red).
**FIG. 26** is a plot of ELISA results illustrating the affinity of anti-histamine antibody conjugated to HRP to different histamine conjugate linkers.
**FIG. 27** is a plot show a comparison of PEG-mono histidine linker with PEG-Phenyl-Histidine Linker in a 5 min test.
   **FIG. 28** is a plot of ELISA plate data using denatured protein coating (blue) versus a plate with traditional blocking (red).

### DETAILED DESCRIPTION OF THE INVENTION

Embodiments of various aspects described herein relate to methods, compositions and kits using a multivalent detecting molecule. The inventors have discovered *inter alia* that a multivalent approach provides an efficient (e.g., sensitive and rapid) detection of small molecules, screening of binding molecules (e.g., antibodies), and methods for producing antibodies. The multivalent approach uses two or more small molecules in a domain that is attached to a substrate through a linking group. The multivalent domain is free to extend, e.g., into a solution, for presentation to a binding compound such as an antibody.

In some embodiments the invention includes a compound comprising at least a substrate binding domain, a branching domain comprising a plurality of small molecules each small molecule linked to a branch point and a linker linking the substrate binding domain and the branching domain.

As used herein, the term "small molecules" refers to natural or synthetic molecules including, but not limited to, amino acids, amino acid dimers, amino acid trimers, peptides, peptidomimetics, polynucleotides, aptamers, nucleotide analogs, organic or inorganic compounds (i.e., including heterorganic and organometallic compounds), saccharides (e.g., mono, di, tri and polysaccharides), steroids, hormones, pharmaceutically derived drugs (e.g., synthetic or naturally occurring), lipids, derivatives of these (e.g., esters and salts of these), fragments of these, and conjugates of these. In some embodiments the small molecules have a molecular weight less than about 10,000 Da, organic or inorganic compounds having a molecular weight less than about 5,000 Da, organic or inorganic compounds having a molecular weight less than about 1,000 Da, organic or inorganic compounds having a molecular weight less than about 500 Da. In some embodiments the small molecule has a molecular weight of less than about 1000 Da.

In some embodiments the small molecules comprise any amino acid or nucleoside that has been modified. For example, without limitation, amino acid and nucleoside modifiations can include acetylation, glycosylation, amidation, hydroxylation, methylation, ubiquitylation, pyrrolidon carboxylic acid, sulfation, racemization, isomerization, phosphorylation, cyclization, sumoylation, formation of disulfide bridges, deamidation, deamination, eliminylation, oxidation, reduction, pegylation, and combinations of these.

Is some embodiments the small molecule is the amino acid histamine or histidine. In other optional embodiments the small molecule is a substituted aromatic compound such as dinitrophenol (e.g., 2, 4-dinitrophenol).

In some embodiments the small molecules comprise an amino acid dimer or an amino acid trimer. As used herein, an amino acid dimer is an oligomer of two amino acids that are bonded through a peptide bond, and an amino acid trimer is an oligomer of three amino acids that are bonded thorugh a peptide bond. In some embodiments the small molecule is an amino acid dimer or trimer wherein at least one of the amino acids is a histadine. In some embodiments the amino acid dimer or trimer includes at least one of either a glutamine or a phenylalanine, for example, wherein the small molecule is a histadine-phenylalanine dimer or a histadine-gluatamine dimer.

As used herein "branching domain" referes to a molecular structure that can include an inert portion and active portion. The inert portion provides a structure such as a core to which the active portions is attached external to at least a portion of the core. The branching domaine can have any shape, including spherical, elliptical, a rod, a single long polymer chain, a polymer combe structure, a random coil, and have large pores (e.g., > 1nm) or include no pores or openings (e.g., <1nm). The linker group is also attached to the branching domain so that the branching domain can be tethered to a substrate, but where the tether can allow the branching domain to be extened away from the substrate. The terms "active" and "inert" are relative terms and depend on the branching domain environment. For example, the active portion can include functional groups, polymers or molecules that bind or interact with an antigen, molecule or polymer, while the inert portion does not directly bind or interact with the antigen, molecule or polymer. The activity can be based on the nature of the material making the inert portion and active portion, or it can be based on special considerations (e.g., accessibility to an antigen, molecule or polymer). For example, in some embodiments small molecules form at least part of the active portion of the branching domain.

In some embodiments the branching domain includes only one type of small molecule. For example, two or more, such as a plurality of the small molecules having the same structure/composition, each linked to a branch point of a branching domain. As used herein, a "branch-point" is an atom or molecule that can be linked to a linker and to the small molecules. Small molecules are linked to the branch point by a "branch" which can be a bond such as a covalent or dative bond. In some embodiments, the branch comprises at least part of the inert portion of the branching domain.

The branching domain can be represented by the formula C(x)ₐM_{b} where C is a subunit of the branching domain having a maximum possible branches equal to x. Each branch can be linked to another C or to small molecule M. In some embodiments, not all the branches are linked (to either C or M) and are left as open or unoccupied. The integers a and b are constrained as follows: a is an integer ≥1 and b is an integer ≥2, provided that b≤ (a)(x-2) +1. It is understood that if a =1, then the single subunit C is equal to the branch-point. **Structure 10** shows an embodiment where x = 4, a = 2, and b= 4. **Structure 11** shows an embodiment where x =3, a = 5 and b =5. The "L" refers to a linker group, which is not part of the branching domain, and the unit "B" refers to an open branch point e.g., a non-occupied site not bound to M or L. **Structure 10** exemplifies an embodiment where all the possible branch points are used for binding to either L or M. **Structure 11** exemplifies an embodiment where x, the maximum possible branch points, is not used for bonding to M or L and therefore one position "B" is left open. In some embodiments more than one linker can be attached to the branching domain, while in other embodiments as shown by **Structure 10** and **11,** only one linker is attached. In some embodiments, the inert portion of the branching domain comprises at least a portion of C ans the active portion of the branching domain includes at least a portion of one or more of M.

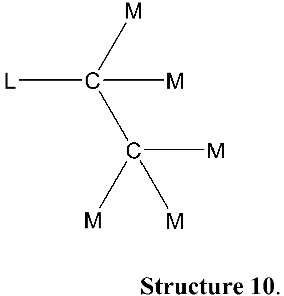

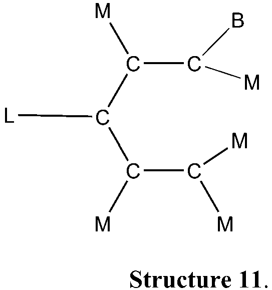

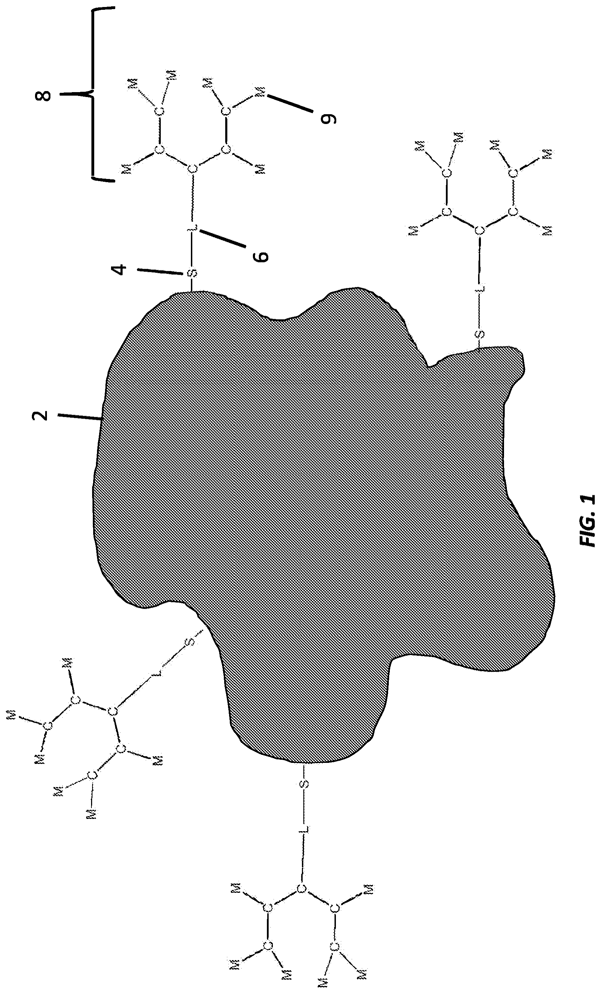

Some embodiments, as depicted by **FIG. 1****,** include a protein carrier **2** to which the "detecting molecule" including a substrate binding domain **4,** a linker group **6** and a branching domain **8** are attached. As shown by **FIG. 1****,** the branching domain can be "multi-valent" with respect to small molecules (**9**) attached to branch points of the branching domain. As used herein multivalent refers to two or more of the small molecules in the branching domain. The protein carrier can be modified to bind the substrate binding domain, for example through reaction of surface carboxylic acids with maleimide groups and reaction with a thiol containing substrate binding domain on the detecting molecule. The density of detecting molecules on the surface can be varied. In some embodiments, the density is determined at least partially by the amount of available functional e.g., carboxylic acid, groups on the surface. For example, some commercial BSA protein carries have specific amounts of functionalization, such as 46 (average) groups per protein carrier. Therefore, the maximum amount of detecting molecule on these carriers is 46.

In some embodiments, the small molecule e.g., M in **FIG. 1****,** is selected to have the same structure as a small molecule target of a method for detecting the small molecule using the detecting molecule as described herein. In some embodiments functional groups such as amino, carboxyl, thiol, hydroxyl that are part of the target small molecule, that is the small molecule that is the analyte to be detected, are used for forming a link to a branch in the branching domain. In other embodiments functional groups such as amino, carboxyl, thiol, hydroxyl that are part of the target small molecule are not usef for forming a link to a branch in the branching domain.

Some embodiments include a particle, a detectable label or a particle including a detectable label to which the detecting molecule including a substrate binding domain **4,** a linker group **6** and a branching domain **8** are attached. Some embodiments include a surface such to which a substrate binding domain **4,** a linker group **6** and a branching domain **8** are attached. Some embodiments include a gold surface and the substrate binding domaine can include a thiol group which binds to the gold. Some embodiments include a silane modified surface and the substrate binding domain includes a silane reative groups such as an amine which bindes to the silane functionalized group, e.g., by a silane-amine coupling. In some embodiments the detecting molecule forms a monolayer on a surface.

As used herein the term "linking" and "linked" refers to forming a direct or indirect attachment or connection between at least two atoms or molecules. The attachment can be by a direct chemical bond between the two atoms or molecules or by an intermediate atom or molecule. For example, F can be linked to H directly, e.g., with a covelent or other bond "-", to form the structure "F-H" or it can be linked indirectly through G by the structure "F-G-H." The intermediate can include, for example, an atom, a small molecule, a polymer, a protein, or a functional group. The term "linker" refers to a molecular entity that can directly or indirectly connect two parts of a composition, e.g., at least one branching domain and one substrate binding domain. In some embodiments, the linker can directly or indirectly link one branching domain and one substrate binding domain.

Linkers can be configured according to a specific need, e.g., based on at least one of the following characteristics. By way of example only, in some embodiments, linkers can be configured to have a sufficient length and flexibility such that it can allow for a branching domain to orient accordingly with respect to a receptor site of a large molecule such as an antigen-binding site of an antibody. In some embodiments the linker can include flexible structure units such polyethylene, poly ethylene glycol or poly propylene glycol groups. In some embodiments the linking groups have a medium to high solubility in aqueous solutions. Without being bound by any specific theory, this solubility, or affinity for water, allows the linker to extend into an aqueous solution rather than self-associate. In some other embodiments, a linker can be selected to be compatible with non-aqueous solutions, such as hydrocarbons and fluorocarbons, e.g., thereby extending into these solutions rather than self associating. In some embodiments the linker is non-toxic. In some embodiments the linker does not react or bind to a sensing antibody or components of a patient sample such as blood, plasma, semen, mucus and other biological fluids. In some embodiments the linker can be any linking group as described in US patent 5,112,738 which is hereby incorporated by reference. For example, the linker can be linear or branched alkenes comprising from 1 to as many as 40 (e.g as many as 30 or 20), or 2, 6, 8, 10 to as many as 20, (i.e., methylene, ethylene, n-propylene, iso-propylene, n-butylene, and so forth). In addition, such alkylenes can contain other substituent groups such as cyano, amino (including substituted amino), acylamino, halogen, thiol, hydroxyl, carbonyl groups, carboxyl (including substituted carboxyls such as esters, amides, and substituted amides). The linker can also contain or consist of substituted or unsubstituted aryl, aralkyl, or heteroaryl groups (e.g., phenylene, phenethylene, and so forth). Additionally, such linkers can contain one or more heteroatoms selected from nitrogen, sulfur and oxygen in the form of ether, ester, amido, amino, thio ether, amidino, sulfone, or sulfoxide. Also, such linkers can include unsaturated groupings such as olefinic or acetylenic bonds, imino, or oximino groups. In some embodiments the linker will be a chain, such as aliphatic comprising between 6 and about 60 atoms excluding hydrogen, between 6 and 50, between 6 and 40, between 6 and 30, between 6 and 20, between 6 and 10, of which between 0 and 60 atm % (e.g., 0 and 50 atm%, 0 and 40 atm %, 10 and 40 atm%) are heteroatoms selected from nitrogen, oxygen, and sulfur.

In some embodiments the linking group comprises a polyethylene glycol with between about 2 and 45 repeat units (e.g., between about 2 and 30 repeat units, between about 2 and 20 repeat units, between about 4 and 10 repeat units). As used herein Poly(ethylene glycol) (PEG), polyethylene glycol, poly(oxyethylene) or poly(ethylene oxide) (PEO), are used interchangeably. Where PEG(x) is used, x is the approximate molecular weight of the linker group. In some other embodiments the linking group comprises polypropylene groups with between 2 and 45 repeat units (e.g., between about 2 and 30 repeat units, between about 2 and 20 repeat units, between about 4 and 10 repeat units) Optionally the linker length is greater than about 5 and less than about 200 Å (e.g., greater than 5 Å and less than about 180 Å, greater than about 7 Å and less than about 157.5 Å, between about 7 Å and about 100 Å).

In some other embodiments, without limitations, the linker comprises is a polyamide, polyimide, polytetrafluoroethylene, polyurethane, polyesters, polyols, polysaccharides, peptides, polyacrylonitrile, RNA, DNA or a fragment comprising between 2 and 30 repeat units of these polymers (e.g., a dimer, trimer or oligomer).

In some embodiments, the linker can be branched. For branched linkers, the linker can link together at least one (e.g., one, two, three, four, five, six, seven, eight, nine, ten or more) surface binding domain and at least one (e.g., one, two, three, four, five, six, seven, eight, nine, ten or more) branching domain.

In some embodiments the linker can be a polymer chain (branched or linear). In some embodiments, chemical linkers can comprise a direct bond or an atom such as oxygen or sulfur, a unit such as NH, C(O), C(O)NH, SO, SO2, SO2NH, or a chain of atoms, such as substituted or unsubstituted C1-C6 alkyl, substituted or unsubstituted C2-C6 alkenyl, substituted or unsubstituted C2-C6 alkynyl, substituted or unsubstituted C6-C12 aryl, substituted or unsubstituted C5-C12 heteroaryl, substituted or unsubstituted C5-C12 heterocyclic, substituted or unsubstituted C3-C12 cycloalkyl, where one or more methylenes can be interrupted or terminated by O, S, S(O), SO2, NH, or C(O). In some embodiments the one or more O, S, S(O), SO2, NH, or C(O) are part of the substrate binding domain, for example the substrate-binding domain can comprise at least one amino group attached to the linker and that can non-covalently or covalently couple with functional groups on the surface of the substrate. For example, the primary amines of the amino acid residues (e.g., lysine or cysteine residues) at the N-terminus or in close proximity to the N-terminus of the substrate binding domains can be used to couple with functional groups on the substrate surface. In some embodiments the one or more O, S, S(O), SO2, NH, or C(O) are part of the linking group forming a link to the branching domain. For example, an ester bond (-NHC(O)-) formed by the reaction of an amino group on a PEG based linker with a carboxylic acid from a branching domain.

A "binding pair", "coupling molecule pair" and "coupling pair" are used interchangeably and without limitation herein to refer to the first and second molecules or functional groups that specifically bind to each other. For example, the binding can be through one or more of a covalent bond, a hydrogen bond, an ionic bond, and a dative bond. In some embodiments one member of the binding pair is conjugated with a solid substrate while the second member is conjugated with the substrate-binding domain. A binding pair can be used for linking the linker to the substrate domain and for linking the linker to the branching domain.

Exemplary coupling molecule pairs also include, without limitations, any haptenic or antigenic compound in combination with a corresponding antibody or binding portion or fragment thereof (e.g., digoxigenin and anti-digoxigenin; mouse immunoglobulin and goat antimouse immunoglobulin) and nonimmunological binding pairs (e.g., biotin-avidin, biotin-streptavidin), hormone (e.g., thyroxine and cortisol-hormone binding protein), receptor-receptor agonist, receptor-receptor antagonist (e.g., acetylcholine receptor-acetylcholine or an analog thereof), IgG-protein A, lectin-carbohydrate, enzyme-enzyme cofactor, enzyme-enzyme inhibitor, and complementary oligonucleotide pairs capable of forming nucleic acid duplexes). The coupling molecule pair can also include a first molecule that is negatively charged and a second molecule that is positively charged.

One example of using coupling pair conjugation is the biotin-avidin or biotin-streptavidin conjugation. In this approach, one of the members of the coupling pair (e.g., a portion of the engineered microbe-targeting molecule such as substrate-binding domain, or a substrate) is biotinylated and the other (e.g., a substrate or the engineered microbe-targeting molecule) is conjugated with avidin or streptavidin. Many commercial kits are also available for biotinylating molecules, such as proteins. For example, an aminooxy-biotin (AOB) can be used to covalently attach biotin to a molecule with an aldehyde or ketone group. In one embodiment, AOB is attached to the substrate-binding domain (e.g., comprising AKT oligopeptide) of the engineered microbe-targeting molecule.

One non-limiting example of using conjugation with a coupling molecule pair is the biotin-sandwich method. See, e.g., Davis et al., 103 PNAS 8155 (2006). The two molecules to be conjugated together are biotinylated and then conjugated together using tetravalent streptavidin. In addition, a peptide can be coupled to the 15-amino acid sequence of an acceptor peptide for biotinylation (referred to as AP; Chen et al., 2 Nat. Methods 99 (2005)). The acceptor peptide sequence allows site-specific biotinylation by the E. coli enzyme biotin ligase (BirA; Id.). An engineered microbe surface-binding domain can be similarly biotinylated for conjugation with a solid substrate. Many commercial kits are also available for biotinylating proteins. Another example for conjugation to a solid surface would be to use PLP -mediated bioconjugation. See, e.g., Witus et al., 132 JACS 16812 (2010).

Still another example of using coupling pair conjugation is double-stranded nucleic acid conjugation. In this approach, one of the members of the coupling pair (e.g., a portion of the engineered microbe-targeting molecule such as substrate-binding domain, or a substrate) can be conjugated with a first strand of the double-stranded nucleic acid and the other (e.g., a substrate) is conjugated with the second strand of the double-stranded nucleic acid. Nucleic acids can include, without limitation, defined sequence segments and sequences comprising nucleotides, ribonucleotides, deoxyribonucleotides, nucleotide analogs, modified nucleotides and nucleotides comprising backbone modifications, branchpoints and nonnucleotide residues, groups or bridges.

Other examples for forming a coupling pair include click chemistry. As used herein "click chemistry" referes to a class of small molecule reactions which can be used for the linking of a binding pair and is not a single specific reaction but rather describes the method of generating products by mimicing nature which produces substance by joining of small modular units. Although useful for biochemical reactions, click chemistry is not limited to biological conditions. Click reactions are efficient and easy to used, occurring in one pot without any special precaustions against water and air, do not produce offensive (e.g., not toxic) byproducts, and, because they are characterized by a high thermodynamic driving force that drives the reaction quickly to a single reaction product, require minimal or no final isolation and purification. Examples of click chemistry includes the copper-catalyzed reaction of an azide with an alkyne to form a 5-membered heteroatom ring (e.g., a Cu(I)-catalyzed azide-alkyne cycloaddition), the thiol-Michael Addition reaction such as reaction of of a thiol group with a maleimide group, , strain-promoted azide-alkyne cycloaddition, strain-promoted alkyne-nitrone cycloaddition, reactions of strained alkenes, alkene and azide [3+2] cycloaddition, alkene and tetrazine inverse-demand Diels-Alder, and alkene and tetrazole photoclick reaction. In some embodiements, a coupling pair is formed using the reaction of a thiol group with a malamide group, forming a thiol-malamide link.

In other embodiments condensation reactions such as amide bond formation between and amine and carboxylic acids can be used to link the linker to the substrate bonding domain or to the branching domain. In still other embodiments the coupling pair can include adsorption such as adsorption of a thiol to a gold surface. Embodiments can also include the reaction of alkyl halide, aldehyde, amino, bromo or iodoacetyl, carboxyl, hydroxyl, epoxy, ester, silane, thiol, and the like, wherein these groups can be one part of the binding pair. Other embodiments include ionic-boding wherein a positive and negative pair combine.

In some embodiments, the substrate-binding domain can comprise at least one, at least two, at least three or more oligopeptides. The length of the oligonucleotide can vary from about 2 amino acid residues to about 10 amino acid residues, or about 2 amino acid residues to about 5 amino acid residues. Determination of an appropriate amino acid sequence of the oligonucleotide for binding with different substrates is well within one of skill in the art. For example, an oligopeptide comprising an amino acid sequence of Alanine-Lysine-Threonine (AKT), which provides a single biotinylation site for subsequent binding to streptavidin-coated substrate.

As used herein the "substrate" can be any material that can be linked to the substrate binding domain. The substrate can be a solid, semi-solid, or polymer and can be homogenous or heterogeneous. For example, and without limitation, a substrate can include a metal, ceramic or polymer surface. For example, the substrate can be the surface of a microbead, a silicon chip, a well plate surface. The surface can also include a coating such as a polymer or protein coating which can be functionalized, e.g., for reaction with the substrate binding domain. Some examples of a substrate include a nucleic acid scaffold, a protein scaffold, a lipid scaffold, a dendrimer, a microparticle or a microbead, a nanotube, a microtiter plate, an electrode, a medical apparatus or implant, a microchip, a filtration device, a membrane, a diagnostic strip, a dipstick, an extracorporeal device, a microscopic slide, a hollow fiber, a hollow fiber cartridge, an electrode surface or any combinations thereof. In some embodiments, the substrate includes ELISA plates. In some embodiments the substrate is a plate such as a microtiter plate that has been modified with hydrophilic groups. For example, high binding ELISA plates, which range from hydrophilic to very hydrophilic, and are commercially available from Thermo Fisher Scientific (Waltham, MA).

In some embodiments, a surface of the substrate can be coated to reduce non-specific binding. For example, a surface of the substrate, e.g., ELISA plate can be coated with a blocking agent. In some embodiments, the substrate e.g., ELISA plate comprises a mixture of a particulate material and a proteinaceous material coated on at least a part of a surface of the substrate. The proteinaceous material in the coarting can be reversibly or non-reversibly denatured. In some embodiments, the proteinaceous material can be non-reversibly denatured. In some embodiments, the proteinaceous material can be cross-linked. For example, the proteinaceous material can be cross-linked with glutaraldehyde.

Exemplary coatings are described in International Application No. PCT/US2018/044076, the content of which is herein incorporated by reference. In some embodiments, the proteinaceious material does not include a particulate material, for example, where no allotrope of carbon is used. In some embodiments, the coating comprises a mixture of an allotrope of carbon having atoms arranged in a hexagonal lattice and a proteinaceous material. For example, the coating is a nanocomposite coating comprising carbon nanotubes, graphene and/or reduced graphene oxide mixed with a proteinaceous material such as BSA, where the proteinaceous material can optionally be reversibly or non-reversibly denatured and/or cross-linked.

As used herein "proteinaceous" material includes proteins and peptides, functionalized proteins, copolymers including proteins, natural and synthetic variants of these, and mixtures of these. For example, proteinaceous material can be Bovine Serum Albumin (BSA).

As used herein, "to cross link" means to form one or more bonds between polymer chains so as to form a network structure such as a gel or hydrogel. The polymers are then "cross-linked" polymers. The bonding can be through hydrogen bonding, covalent bonding or electrostatic. The "cross linking agent" can be a bridging molecule or ion, or it can be a reactive species such as an acid, a base or a radical producing agent.

For molecular cross linking agents, the cross linking agents contain at least two reactive groups that are reactive towards numerous groups, including primary amines, carboxyls, sulfhydryls, carbohydrates and carboxylic acids. Proteins and peptide molecules have many of these functional groups and therefore proteins and peptides can be readily conjugated and cross linked using these cross linking agents. Cross linking agents can be homobifunctional, having two reactive ends that are identical, or heterobifunctional, having two different reactive ends. In some embodiments the cross linking agent is a molecule such as glutaraldehyde, dimethyl adipimidate (DMA), dimethyl suberimidate (DMS), Bissulfosuccinimidyl suberate, formaldehyde, p-azidobenzoyl hydrazide; n-5-azido-2-nitrobenzoyloxysuccinimide; n-[4-(p-azidosalicylamido)butyl]-3'-(2'-pyridyldithio) propionamide; p-azidophenyl glyoxal monohydrate; bis [b-(4-azidosalicylamido)ethyl]disulfide; bis [2-(succinimidooxycarbonyloxy)ethyl] sulfone; 1,4-di [3'-(2'-pyridyldithio)propionamido] butane; dithiobis(succinimidyl propionate); disuccinimidyl suberate; disuccinimidyl tartrate; 3,3'-dithiobis(sulfosuccinimidyl propionate); 3,3'-dithiobis(sulfosuccinimidyl propionate) 1-ethyl-3-[3-dimethylaminopropyl]carbodiimide hydrochloride; Ethylene Glycol bis(succinimidyl succinate); N-(E-maleimidocaproic acid hydrazide); [N-(E-maleimidocaproyloxy)-succinimide ester]; N-Maleimidobutyryloxysuccinimide ester; Hydroxylamine.HCl; Maleimide-PEG-succinimidyl carboxy methyl; m-Maleimidobenzoyl-N-hydroxysuccinimide Ester; N-Hydroxysuccinimidyl-4-azidosalicylic acid; N-(p-Maleimidophenyl isocyanate); N-Succinimidyl(4-iodoacetyl) Aminobenzoate; Succinimidyl-4-(N-maleimidomethyl)cyclohexane-1-carboxylate; Succinimidyl 4-(p-maleimidophenyl) Butyrate; Sulfo Disulfosuccinimidyl Tartrate; [N-(E-maleimidocaproyloxy)-sulfo succinimide ester; N-Maleimidobutyryloxysulfosuccinimide ester; N-Hydroxysulfosuccinimidyl-4-azidobenzoate; m-Maleimidobenzoyl-N-hydroxysulfosuccinimide Ester; Sulfosuccinimidyl (4-azidophenyl)-1,3 dithio propionate; Sulfosuccinimidyl 2-(m-azido-o-nitrobenzamido)-ethyl-1,3'-dithio propionate; Sulfosuccinimidyl 6-(4'-azido-2'-nitrophenylamino) hexanoate; Sulfosuccinimidyl-2-(p-azidosalicylamido)ethyl-1,3-dithiopropionate; N-(Sulfosuccinimidyl(4-iodoacetyl)Aminobenzoate); Sulfosuccinimidyl-4-(N-maleimidomethyl)cyclohexane- 1-carboxylate; Sulfo succinimidyl 4-(p-maleimidophenyl) Butyrate; and mixtures of these. In some embodiments the cross linking agent is mone- or poly-ethylene glycol diglycidil ether. In some embodiments the cross linker is a homobifunctional cross linking agent such as glutaraldehyde.

As used herein, "denaturing" is the process of modifying the quaternary, tertiary and secondary molecular structure of a protein from its natural, original or native state. For example, such as by breaking weak bonds (e.g., hydrogen bonds), which are responsible for the highly ordered structure of the protein in its natural state. The process can be accomplished by, for example: physical means, such as by heating, sonication or shearing; by chemical means such as acid, alkali, inorganic salts and organic solvents (e.g., alcohols, acetone or chloroform); and by radiation. A denatured protein, such as an enzyme, losses its original biological activity. In some instances, the denaturing process is reversible, such that the protein molecular structure is regained by the re-forming of the original bonding interactions at least to the degree that the original biological function of the protein is restored. In other instances, the denaturing process is irreversible or non-reversible, such that the original and biological function of the protein is not restored. Cross-linking, for example after denaturing, can reduce or eliminate the reversibility of the denaturing process.

The degree of denaturing can be expressed as a percent of protein molecules that have been denatured, such as a mole percent. Some methods of denaturing can be more efficient than others. For example, under some conditions, sonication applied to BSA can denature about 30-40% of the protein and the denaturing is reversible. When BSA is denatured it undergoes two structural stages. The first stage is reversible whilst the second stage is irreversible (e.g., non-reversible) but does not necessarily result in a complete destruction of the ordered structure. For example, heating up to 65°C can be regarded as the first stage, with subsequent heating above that as the second stage. At higher temperatures, further transformations are seen. In some embodiments, BSA is denatured by heating above about 65°C (e.g., above about 70°C, above about 80°C, above about 90°C, above about 100°C, above about 110°C, above about 120°C), below about 200°C (below about 190°C, 180°C, 170°C, 160°C, 150°C), and for at least about 1 minute (e.g., at least about 2, 3, 4, 5, 10 or 20 minutes) but less than about 24 hours (e.g., less than about 12, 10, 8, 6, 4, 2 1 hour). Embodiments include any ranges herein described, for example heating above about 90°C but below about 150°C and for at least 2 minutes but less than one hour.

In some embodiments the proteinaceous material used in the compositions and structures described herein are at least about 20% to about 100% (e.g., 20%, 30%, 40%, 50%, 60%, 70%, 80%, 90%, or 100%) denatured. In some embodiments, less than 50% of the denatured protein reverts back to its natural state (e.g., less than 40%, less than 30%, less than 20%, less than 10%, less than 1%). Therefore, the reversibility of the denaturing can be described as being 50% reversible, 40% reversible (60% irreversible), 30 % reversible (70% irreversible), 20% reversible (80% irreversible), 10% reversible (90% irreversible) or even 0% reversible (100% irreversible).

In embodiments wherein the linker has a specified length, the length is the linear length from the head to tail group, wherein the head group is attached to the branching domain and the tail group is attached to the substrate domain. In some embodiments the length is the contour length, which is length of the linker in its maximally extended conformation and wherein none of the bonds are strained in length or angle from their lowest energy configuration. For example, where the polymer comprises a carbon or carbon oxygen chain, the eclipsed conformation is used. For example, the contour length for a single unit of a poly ethylene oxide (PEO) chain (e.g., -CH₂-CH₂-O-) has a contour length of 0.28 in water. It is understood that in addition to the molecular weight, the length of a linker will depend on the molecular dynamics, wherein, for example, the medium has a large contribution. One measurement of length that contrasts with the contour length is the Flory radius which is calculated using the random walk law, and applies, for the most part, in the melt. That is, when a polymer is put in solution with an organic solvent, the coil expands to a larger size than the size reflected by the Flory radius equation. Table 1 illustrates the contour length as compared to Flory radius for PEO.

**Table 1: PEG lengths**

| **Number of PEO units** | **MW (Dalton)** | **Contour length (nm)** | **Flory radius (nm)** |
|---|---|---|---|
| 2 | 88 | 0.6 | 0.5 |
| 11 | 484 | 3.1 | 1.2 |
| 45 | 2000 | 12.7 | 2.8 |

In some embodiments, the molecule disclosed herein, i.e., a compound comprising (i) a substrate binding domain; (ii) a branching domain comprising a plurality of small molecules each small molecule linked to a branch of a branch-point; and (iii) a linker linking the substrate binding domain and the branching domain, can be immobilized or conjugated to a surface of various substrates. Accordingly, a further aspect provided herein is an article comprising a substrate and at least one molecule, i.e., a compound comprising (i) a substrate binding domain; (ii) a branching domain comprising a plurality of small molecules each small molecule linked to a branch of a branch-point; and (iii) a linker linking the substrate binding domain and the branching domain, described herein, wherein the substrate comprises on its surface at least one, including at least two, at least three, at least four, at least five, at least ten, at least 25, at least 50, at least 100, at least 250, at least 500, or more of the molecules. In some embodiments, the substrate can be conjugated or coated with at least one compound as described herein, using any of conjugation methods described herein or any other art-recognized methods. For example, the compound comprising (i) a substrate binding domain; (ii) a branching domain comprising a plurality of small molecules each small molecule linked to a branch of a branch-point; and (iii) a linker linking the substrate binding domain and the branching domain can be linked to immobilized or conjugated to a surface of a substrate via the substrate binding domain.

The substrate can be made from a wide variety of materials and in a variety of formats. For example, the solid substrate can be utilized in the form of beads (including polymer microbeads, magnetic microbeads, and the like), filters, fibers, screens, mesh, tubes, hollow fibers, scaffolds, plates, channels, other substrates commonly utilized in assay formats, and any combinations thereof. Examples of substrates include, but are not limited to, nucleic acid scaffolds, protein scaffolds, lipid scaffolds, dendrimers, microparticles or microbeads, nanotubes, microtiter plates, medical apparatuses (e.g., needles or catheters) or implants, dipsticks or test strips, microchips, filtration devices or membranes, diagnostic strips, hollow-fiber reactors, microfluidic devices, living cells and biological tissues or organs, extracorporeal devices, mixing elements (e.g., spiral mixers).

The substrate can be made of any material, including, but not limited to, metal, metal alloy, polymer, plastic, paper, glass, fabric, packaging material, biological material such as cells, tissues, hydrogels, proteins, peptides, nucleic acids, and any combinations thereof. The substrate can be a solid, semi-solid, or polymer and can be homogenous or heterogeneous. For example, and without limitation, a substrate can include a metal, ceramic or polymer surface. For example, the substrate can be a microbead, a silicon chip, or a well plate surface. The surface can also include a coating such as a polymer or protein coating which can be functionalized, e.g., for reaction with the substrate binding domain. For example, in some embodiments the substrate can be gold. In some embodiments the substrate can be a silane functionalized surface such as a silica (e.g., glass) surface or a resin bead.

The particular format and/or material of the substrate depend on the application such as separation/detection methods employed in an assay. In some embodiments, the format and/or material of the substrate can be chosen or modified to maximize signal-to-noise ratios, *e.g.,* to minimize background binding, and/or for ease of separation of reagents and cost. For example, the surface of the substrate can be treated or modified with surface chemistry to minimize chemical agglutination and non-specific binding. In some embodiments, at least a portion of the substrate surface can be treated to become less adhesive to any molecules (including microbes, if any) present in a test sample. By way of example only, the substrate surface in contact with a test sample can be silanized or coated with a polymer such that the substrate surface is inert to the molecules present in the test sample, including but not limited to, cells or fragments thereof (including blood cells and blood components), proteins, nucleic acids, peptides, small molecules, therapeutic agents, microbes, microorganisms and any combinations thereof. In other embodiments, a substrate surface can be treated with an omniphobic layer. In other embodiments, a solid substrate surface can be modified or overlaid with a repellant or slippery surface. For example, a solid substrate surface can comprise a nano/microstructured substrate layer infused with a lubricating fluid, where the lubricating fluid is substantially immobilized on the substrate layer to form a repellant or slippery surface. In some embodiments, the repellant or slippery surface is known as Slippery Liquid-Infused Porous Surface (SLIPS), which is described in Wong T.S. et al., "Bioinspired self-repairing slippery surfaces with pressure-stable omniphobicity." (2011) Nature 477 (7365): 443-447, and International Application Nos. PCT/US12/21928 and PCT/US12/21929, the contents of which are incorporated herein by reference.

In some embodiments, the substrate can be fabricated from or coated with a biocompatible material. As used herein, the term "biocompatible material" refers to any material that does not deteriorate appreciably and does not induce a significant immune response or deleterious tissue reaction, *e.g.*, toxic reaction or significant irritation, over time when implanted into or placed adjacent to the biological tissue of a subject, or induce blood clotting or coagulation when it comes in contact with blood. Suitable biocompatible materials include, for example, derivatives and copolymers of polyimides, poly(ethylene glycol), polyvinyl alcohol, polyethyleneimine, and polyvinylamine, polyacrylates, polyamides, polyesters, polycarbonates, and polystyrenes. In some embodiments, biocompatible materials can include metals, such as titanium and stainless steel, or any biocompatible metal used in medical implants. In some embodiments, biocompatible materials can include paper substrate, *e.g.,* as a substrate for a diagnostic strip. In some embodiments, biocompatible materials can include peptides or nucleic acid molecules, *e.g.,* a nucleic acid scaffold such as a 2-D DNA sheet or 3-D DNA scaffold.

Additional material that can be used to fabricate or coat a substrate include, without limitations, polydimethylsiloxane, polyimide, polyethylene terephthalate, polymethylmethacrylate, polyurethane, polyvinylchloride, polystyrene polysulfone, polycarbonate, polymethylpentene, polypropylene, polyvinylidine fluoride, polysilicon, polytetrafluoroethylene, polysulfone, acrylonitrile butadiene styrene, polyacrylonitrile, polybutadiene, poly(butylene terephthalate), poly(ether sulfone), poly(ether ketones), poly(ethylene glycol), styrene-acrylonitrile resin, poly(trimethylene terephthalate), polyvinyl butyral, polyvinylidenedifluoride, poly(vinyl pyrrolidone), and any combination thereof.

In various embodiments, the substrate can be functionalized with various coupling molecules as described earlier.

As used herein, by the "coating" or "coated" is generally meant a layer of molecules or material formed on an outermost or exposed layer of a substrate surface. With respect to a coating of the compounds disclosed herein, e.g., a compound comprising (i) a substrate binding domain; (ii) a branching domain comprising a plurality of small molecules each small molecule linked to a branch of a branch-point; and (iii) a linker linking the substrate binding domain and the branching domain, on a substrate, the term "coating" or "coated" refers to a layer of the molecules formed on an outermost or exposed layer of a substrate surface.

The amount of the molecules described herein conjugated to or coating on a substrate surface can vary with a number of factors such as a substrate surface area, conjugation/coating density, types of molecules, and/or binding performance. A skilled artisan can determine the optimum density of molecules on a substrate surface using any methods known in the art. By way of example only, for magnetic microparticles (including nanoparticles) as a substrate (as discussed in detail later), the amount of the molecules described herein used for conjugating to or coating magnetic microbeads can vary from about 1 wt % to about 30 wt %, or from about 5 wt % to about 20 wt%. In some embodiments, the amount of the molecules described herein used for conjugating to or coating magnetic microbeads can be higher or lower, depending on a specific need.

Exemplary substrates include, but are not limited to, a nucleic acid scaffold, a protein scaffold, a lipid scaffold, a dendrimer, a microparticle or a microbead, a nanotube, a microtiter plate, an electrode, a medical apparatus or implant, a microchip, a filtration device, a membrane, a diagnostic strip, a dipstick, an extracorporeal device, a microscopic slide, a hollow fiber, a hollow fiber cartridge, an electrode surface or any combinations thereof.

In some embodiments, the substrate includes ELISA plates. In some embodiments the substrate is a plate such as a microtiter plate that has been modified with hydrophilic groups. For example, high binding ELISA plates, which range from hydrophilic to very hydrophilic, and are commercially available from Thermo Fisher Scientific (Waltham, MA).

In some embodiments, the substrate is an electrode.

In some embodiments, the substrate is a particle. Without limitations, the particle can be a microparticle or a nanoparticle. The term "microparticle" as used herein refers to a particle having a particle size of about 0.001 µm to about 100 µm, about 0.005 µm to about 50 µm, about 0.01 µm to about 25 µm, about 0.05 µm to about 10 µm, or about 0.05 µm to about 5 µm. In one embodiment, the microparticle has a particle size of about 0.05 µm to about 1 µm. In one embodiment, the microparticle is about 0.09 µm - about 0.2 µm in size. The term "nanoparticle" as used herein generally refers to a bead or particle having a size ranging from about 1 nm to about 1000 nm, from about 10 nm to about 500 nm, from about 25 nm to about 300 nm, from about 40 nm to about 250 nm, or from about 50 nm to about 200 nm.

In some embodiments, the substrate is magnetic, e.g., a magnetic particle. For example, the substrate can be ferromagnetic, paramagnetic or super-paramagnetic.

In some embodiments, the substrate is a dipstick and/or a test strip for detection of small molecules. For example, a dipstick and/or a test strip can include at least one test area containing one or more molecules described herein.

As described herein, the compounds described herein, i.e., a compound comprising (i) a substrate binding domain; (ii) a branching domain comprising a plurality of small molecules each small molecule linked to a branch of a branch-point; and (iii) a linker linking the substrate binding domain and the branching domain, can be used to develop assays for rapid detection of an analyte, e.g., a small molecule, in a sample. Accordingly, kits and assays for detecting the presence or absence of an analyte, e.g., a small molecule, in a test sample are also provided herein. Exemplary assays include, but are not limited to enzyme-linked immunosorbent assay (ELISA), fluorescent linked immunosorbent assay (FLISA), immunofluorescent microscopy, fluorescence in situ hybridization (FISH), or any other radiological, chemical, enzymatic or optical detection assay.

In some aspects, described herein is a method for detecting the presence or absence of an analyte, e.g., a small molecule in sample. Generally, the method comprises contacting a test sample with a compound described herein; and detecting binding of an analyte binding ligand to the compound described herein. A decrease in binding relative to binding in absence of the test sample, indicates the small molecule is present in the sample. The small molecules linked to the branching domain of the compound and the analyte can be structurally similar and/or they can bind competitively with the analyte biding molecule.

In some embodiments, analyte is histamine or dinitrophenol.

In some embodiments, analyte is histamine and the small molecules linked to the branching domain of the compound are histidine.

Without limitations, any molecule capable of binding with the analyte and/or the small molecules linked to the branching domain can be used as an analyte binding ligand. Exemplary analyte binding ligands can include, but are not limited to, antibodies, antigen binding fragments of antibodies, aptamers, cell-surface receptors and the like. In some embodiments, the analyte binding ligand is an antibody.

In some embodiments, the analyte binding ligand comprises a detectable label.

In some embodiments, labeling molecules that can bind with the analyte binding ligand can be used for detecting the binding. As used herein, a "labeling molecule" refers to a molecule that comprises a detectable label and can bind with an analyte binding ligand. Labeling molecules can include, but are not limited to, antibodies, antigen binding fragments of antibodies, aptamers, cell-surface receptors and the like.

Also provided herein is a method for selecting a ligand capable of binding a small molecule. Generally, the method comprises contacting a test ligand with a compound described herein; and detecting binding of the test ligand with the compound described herein in the presence and absence of a free small molecule. The small molecules linked to the branching domain of the compound and the free small molecule can be structurally similar or they can bind competitively with the test ligand. A test ligand having reduced binding to the compound in the presence of the small molecule can be selected as a ligand capable of binding the small molecule. In some embodiments, the small molecule is a histamine or dinitrophenol.

The binding of the test ligand to the compound described herein can be detected by any means available. For example, the test ligand can comprise a detectable label. Alternatively, or in addition, a labeling molecule, comprising a detectable label, that can bind with the test ligand can be used for detecting the binding.

A detection component, device or system can be used to detect and/or analyze the binding of the analyte binding ligand to the compound described herein, for example, by spectroscopy, electrochemical detection, polynucleotide detection, fluorescence anisotropy, fluorescence resonance energy transfer, electron transfer, enzyme assay, magnetism, electrical conductivity, isoelectric focusing, chromatography, immunoprecipitation, immunoseparation, aptamer binding, filtration, electrophoresis, use of a CCD camera, immunoassay, ELISA, immunostaining, microscopy, immunofluorescence, western blot, polymerase chain reaction (PCR), RT-PCR, fluorescence *in situ* hybridization, sequencing, mass spectroscopy, or substantially any combination thereof.

As used herein, the term "detectable label" refers to a composition capable of producing a detectable signal indicative of the presence of a target. Detectable labels include any composition detectable by spectroscopic, photochemical, biochemical, immunochemical, electrical, optical or chemical means. Suitable labels include fluorescent molecules, radioisotopes, nucleotide chromophores, enzymes, substrates, chemiluminescent moieties, bioluminescent moieties, and the like. As such, a label is any composition detectable by spectroscopic, photochemical, biochemical, immunochemical, electrical, optical or chemical means needed for the methods and devices described herein.

A wide variety of fluorescent reporter dyes are known in the art. Typically, the fluorophore is an aromatic or heteroaromatic compound and can be a pyrene, anthracene, naphthalene, acridine, stilbene, indole, benzindole, oxazole, thiazole, benzothiazole, cyanine, carbocyanine, salicylate, anthranilate, coumarin, fluorescein, rhodamine or other like compound.

Other exemplary detectable labels include luminescent and bioluminescent markers (*e.g.,* biotin, luciferase (*e.g.,* bacterial, firefly, click beetle and the like), luciferin, and aequorin), radiolabels (*e.g.,* 3H, 125I, 35S, 14C, or 32P), enzymes (*e.g.,* galactosidases, glucorinidases, phosphatases (*e.g.,* alkaline phosphatase), peroxidases (*e.g.,* horseradish peroxidase), and cholinesterases), and calorimetric labels such as colloidal gold or colored glass or plastic (*e.g.,* polystyrene, polypropylene, and latex) beads. Patents teaching the use of such labels include U.S. Pat. Nos. 3,817,837, 3,850,752, 3,939,350, 3,996,345, 4,277,437, 4,275,149, and 4,366,241, each of which is incorporated herein by reference.

Means of detecting such labels are well known to those of skill in the art. Thus, for example, radiolabels can be detected using photographic film or scintillation counters, fluorescent markers can be detected using a photo-detector to detect emitted light. Enzymatic labels are typically detected by providing the enzyme with an enzyme substrate and detecting the reaction product produced by the action of the enzyme on the enzyme substrate, and calorimetric labels can be detected by visualizing the colored label.

In some embodiments, the detectable label is a fluorophore or a quantum dot. Without wishing to be bound by a theory, using a fluorescent reagent can reduce signal-to-noise in the imaging/readout, thus maintaining sensitivity. Accordingly, in some embodiments, prior to detection, the microbes isolated from or remained bound on the microbe-targeting substrate can be stained with at least one stain, *e.g.,* at least one fluorescent staining reagent comprising a microbe-binding molecule, wherein the microbe-binding molecule comprises a fluorophore or a quantum dot. Examples of fluorescent stains include, but are not limited to, any microbe-targeting element (*e.g.,* microbe-specific antibodies or any microbe-binding proteins or peptides or oligonucleotides) typically conjugated with a fluorophore or quantum dot, and any fluorescent stains used for detection as described herein.

Any method known in the art for detecting the particular label can be used for detection. Exemplary methods include, but are not limited to, spectrometry, fluorometry, microscopy imaging, immunoassay, and the like.

In particular embodiments, binding can be detected through use of one or more enzyme assays, *e.g.,* enzyme-linked assay (ELISA). Numerous enzyme assays can be used to provide for detection. Examples of such enzyme assays include, but are not limited to, beta-galactosidase assays, peroxidase assays, catalase assays, alkaline phosphatase assays, and the like. In some embodiments, enzyme assays can be configured such that an enzyme will catalyze a reaction involving an enzyme substrate that produces a fluorescent product. Enzymes and fluorescent enzyme substrates are known and are commercially available (*e.g.,* Sigma-Aldrich, St. Louis, Mo.). In some embodiments, enzyme assays can be configured as binding assays that provide for detection of microbe. For example, in some embodiments, a labeling molecule can be conjugated with an enzyme for use in the enzyme assay. An enzyme substrate can then be introduced to the one or more immobilized enzymes such that the enzymes are able to catalyze a reaction involving the enzyme substrate to produce a detectable signal.

In some embodiments, an enzyme-linked assay (ELISA) can be used to detect signals from the analyte binding ligand or the labeling molecule. In ELISA, the analyte binding ligand or the labeling molecule can comprise an enzyme as the detectable label. Each analyte binding ligand or labeling molecule can comprise one or more (*e.g.,* 1, 2, 3, 4, 5, 6, 7, 8, 9, 10 or more) enzymes.

For the ELISA, a variety of enzymes can be used, with either colorimetric or fluorogenic substrates. In some embodiments, the reporter-enzyme produces a calorimetric change which can be measured as light absorption at a particular wavelength. Exemplary enzymes include, but are not limited to, beta-galactosidases, peroxidases, catalases, alkaline phosphatases, and the like.

One of skill in the art can readily recognize an appropriate enzyme substrate for any art-recognized enzymes used for colorimetric detection. By way of example only, an exemplary substrate for alkaline phosphatase can include BCIP/NBT or PNPP (p-Nitrophenyl Phosphate, Disodium Salt); exemplary substrates for horseradish peroxidase can include TMB (3,3',5,5'-tetramethylbenzidine) and chromogen.

In some embodiments the detectable label can be redox active and is directly detected by the electrode. For example, electrochemical methods, systems and compositions as described in application PCT/US18/44076 incorporated herein by reference can be used. Without wishing to be bound by a theory, the electroactive label enables detection by a change in the concentration of the label at the electrode in the presence of the analyte in the sample. For example, a decrease in the concentration of the label at the electrode due to displacement in a competition assay. The redox active label can be detected by electrochemical means. Without limitations, electrochemical means include methods that rely on a change in the potential, charge or current to characterize the analyte's concentration. Some examples include potentiometry, controlled current coulometry, controlled-potential coulometry, amperometry, stripping voltammetry, hydrodynamic voltammetry, polarography, stationary electrode voltammetry, pulsed polarography, electrochemical impedance spectroscopy and cyclic voltammetry. The signals are detected using an electrode (e.g., a working, counter and reference can be used) or electrochemical sensors coupled to circuits and systems for collection, manipulation and analysis of the signals.

In some embodiments the detectable label can be directly electroacitively detectable. For example, label can include redox active compounds such as metal particles (e.g., silver nanoparticles), metal complexes (e.g., ferrocene derivatives) and organic compounds (e.g., polyaniline, viologens).

In some other embodiments the electrochemical label can be detected indirectly by electrochemical means. For example, the electrochemical label can be detected by reacting with a sacrificial redox active molecule which deposits on the electrode surface that then is detected electrochemically. For example, the antibody or secondary antibody can be conjugated with a redox catalyst and the sacrificial redox active molecule can be oxidized or reduced and precipitated onto the electrode surface. In some embodiments the redox active catalyst is a peroxidase such as horseradish peroxidase (HRP) and the sacrificial redox active molecule is 3,3'-Diaminobenzidine (DMB); 2,2'-azino-bis(3-ethylbenzothiazoline-6-sulphonic acid) (ABTS); o-orthophenylenediamine (OPD); AmplexRed; 3,3'-Diaminobenzidine (DAB); 4-chloro-1-naphthol (4CN); AEC; 3,3',5,5'-Tetramethylbenzidine (TMB); homovanilllic acid; lumininol; Nitro blue tetrazolium (NBT); Hydroquinone; benzoquinone; mixtures of these; or mixtures of these. Embodiments include known immunoassays or modifications of these to be detectable by electrochemistry. Optionally, the sacrificial molecule can also be detected by fluorescence.

In some embodiments of the methods described herein, the compound comprising (i) a substrate binding domain; (ii) a branching domain comprising a plurality of small molecules each small molecule linked to a branch of a branch-point; and (iii) a linker linking the substrate binding domain and the branching domain is conjugated to a surface of a substrate.

In some embodiments a blocking agent can be used in the methods described herein. As used herein a "blocking agent" or "molecular blockers" are compounds used to prevent non-specific interactions. The blocking agent can be a coating on a surface, e.g., of the substrate, that prevents non-specific interactions or fouling of the surface when it is contacted with the test sample. In some embodiments the compound comprising (i) a substrate binding domain; (ii) a branching domain comprising a plurality of small molecules each small molecule linked to a branch of a branch-point; and (iii) a linker linking the substrate binding domain and the branching domain is directly attached to the surface of the substrate. In some embodiments the compound comprising (i) a substrate binding domain; (ii) a branching domain comprising a plurality of small molecules each small molecule linked to a branch of a branch-point; and (iii) a linker linking the substrate binding domain and the branching domain is attached to the blocking agent. In some embodiments, the substrate can be pretreated with the blocking agent, prior to contacting with the test sample and/or the analyte binding ligand. In some embodiments, the substrate can be contacted concurrently with a blocking agent and a test sample.

Non-specific interactions can include any interaction that is not desired between the target molecule and the surface, or between other components in solution. The blocking agent can be a protein, mixture of proteins, fragments of proteins, peptides or other compounds that can passively absorb to the surface in need of blocking. For example, proteins (e.g., BSA and Casein), poloxamers (e.g., pluronics), PEG-based polymers and oligomers (e.g., diethylene glycol dimethyl ether), cationic surfactants (e.g., DOTAP, DOPE, DOTMA). Some other examples include commercially available blocking agent or components therein that are available from, for example, Rockland Inc. (Limeric, PA) such as : BBS Fish Gel Concentrate; PBS Fish Gel Concentrate; TBS Fish Gel Concentrate; Blocking Buffer for Fluorescent Western Blotting; BLOTTO; Bovine Serum Albumin (BSA); ELISA Microwell Blocking Buffer; Goat Serum; IPTG (isopropyl beta-D-thiogalactoside) Inducer; Normal Goat Serum (NGS); Normal Rabbit Serum; Normal Rat Serum; Normal Horse Serum; Normal Sheep Serum; Nitrophenyl phosphate buffer (NPP); and Revitablot^{™} Western Blot Stripping Buffer.

In some embodiments, the methods described herein comprise a step of separating the compound comprising (i) a substrate binding domain; (ii) a branching domain comprising a plurality of small molecules each small molecule linked to a branch of a branch-point; and (iii) a linker linking the substrate binding domain and the branching domain from the test sample after contact.

The separating step can be accomplished by any means known in the art. For example, the sample mixture can be drained/poured from the substrate and then flushed with a liquid, for example one or more of the preprocessing solutions described herein. This can be repeated. In some embodiments the flushing liquid includes a composition including an analyte binding ligand. Alternatively, the flushing liquid can be added to the mixture to dilute the mixture, optionally with removal of any excess liquid as the volume is increased. Accordingly, the substrate can optionally be washed or flushed any number (e.g., 1, 2, 3, 4, 5 or more) of times before detection (e.g., fluorometric, electrochemical, colorimetric detection). Without wishing to be bound by a theory, such washing can reduce and or eliminate any contaminants from the test sample, such as components in a biological fluid, that can be problematic during incubation or detection. In one embodiment, the detecting molecule- substrate after isolated from the solution and/or the test sample can be washed with a buffer (e.g., but not limited to, TBST) for at least about 1-3 times.

In some embodiments, the methods described herein comprise a step of separating the compound comprising (i) a substrate binding domain; (ii) a branching domain comprising a plurality of small molecules each small molecule linked to a branch of a branch-point; and (iii) a linker linking the substrate binding domain and the branching domain from any unbound analyte binding ligand.

Any art-recognized wash buffer that does not affect function/viability of the binding molecule-substrate and does not interfere with binding of the analyte binding ligand with the components (e.g., analyte or the small molecule conjugated to the branching domain) can be used to washing. Examples of a wash buffer can include, but are not limited to, phosphate-buffered saline, Tris-buffered saline (TBS), and a combination thereof. In some embodiments, a wash buffer can include a mixture of TBS, 0.1% Tween and 5 mM Ca²⁺. In some embodiments, the processing buffer and/or wash buffer can exclude calcium ions and/or include a chelator, e.g., but not limited to, EDTA.

In embodiments where substrate is magnetic, e.g., a magnetic bead, a magnet can be employed in the separating step. The skilled artisan is well aware of methods for carrying out magnetic separations. Generally, a magnetic field or magnetic field gradient can be applied to direct the magnetic beads. Optionally, the substrate can be washed with a buffer to remove any leftover sample and unbound components.

In some embodiments where the substrate is a magnetic particle, magnetic particles larger than the substrate magnetic particles can be added to the test sample. The larger magnetic particles (optionally conjugated with a compound comprising (i) a substrate binding domain; (ii) a branching domain comprising a plurality of small molecules each small molecule linked to a branch of a branch-point; and (iii) a linker linking the substrate binding domain and the branching domain) can act as local magnetic field gradient concentrators, thereby attracting the smaller magnetic particles to the larger magnetic particles and forming an aggregate, which in turn can be immobilized in the presence of a magnetic field gradient more readily than individual smaller magnetic particles. Thus, addition of magnetic particles that are larger than the substrate magnetic particles can reduce loss of smaller magnetic particles to a fluid during each wash and/or magnetic separation. This concept of using larger magnetic particles to act as local magnetic field gradient concentrators can be extended to magnetic separations and is described in U.S. Provisional Appl. No. 61/772,436, filed March 4, 2013, entitled "Methods for Magnetic Capture of a Target Molecule," the content of which is incorporated herein by reference. In some embodiments, the magnetic field gradient can be generated by a magnetic field gradient generator described in the U.S. Provisional Application No. 61/772,360, filed March 4, 2013, entitled "Magnetic Separator."

Without limitations, if substrate does not possess a magnetic property, the separating step can be carried out by non-magnetic means, e.g., centrifugation, and filtration. In some embodiments where the substrate is in form of a dipstick or membrane, the detecting dipstick or membrane can be simply removed from the test sample.

In some embodiments, analyte (e.g., small molecule) detection can be performed by flowing a test sample through a device comprising (i) a chamber with an inlet and an outlet, (ii) at least one compound comprising (i) a substrate binding domain; (ii) a branching domain comprising a plurality of small molecules each small molecule linked to a branch of a branch-point; and (iii) a linker linking the substrate binding domain and the branching domain disposed in the chamber between the inlet and outlet.

In some embodiments of any one of the methods for detecting small molecules as described herein is modified and used for the detection of a ligand capable of binding the small molecule. In this method the test sample includes the target small molecule and the test ligands. For example, the test ligand can be any compounds to be tested for binding to the small molecule. For example, the test ligand can include antibodies, adnectins, ankyrins, antibody mimetics and other protein scaffolds, aptamers, nucleic acid (e.g., an RNA or DNA aptamer), proteins, peptides, oligosaccharides, polysaccharides, lipopolysaccharides, cellular metabolites, cells, viruses, subcellular particles, haptens, pharmacologically active substances, alkaloids, steroids, vitamins, amino acids, avimers, peptidomimetics, hormone receptors, cytokine receptors, synthetic receptors, sugars and molecularly imprinted polymer. The test sample is contacted/mixed using any of the methods, compositions, reagents and equipment described herein for detecting a small molecule. Optimization for detection by changing both the concentration of the small molecule and the ligand can be done by a designed experiment as is known in the art. Comparisons of different ligands and their accuracy and precision for detecting the small molecules can be tabulated and compared. In some embodiments, the method includes screening of known commercial antibodies to determine which antibodies bind more strongly to a target small molecule.

Any processes or steps described herein can be performed by a module or device. While these are discussed as discrete processes, one or more of the processes or steps described herein can be combined into one system for carrying out the assays of any aspects described herein.

In some embodiments, the assay or process described herein can be adapted for use in a high-throughput platform, e.g., an automated system or platform.

In some embodiments, the structures, compositions and methods described herein can be useful for a rapid assay, for example, for testing for the presence of a small molecule. As used herein the term "rapid" refers to methods that take less time for detecting the molecule than previous comparable methods. A comparable method refers to test for the same target and providing a similar precision and sensitivity (e.g., wherein similar here means ±10%). The comparable method can include the same methods and compositions as the instant test without use of the compound comprising compound comprising (i) a substrate binding domain; (ii) a branching domain comprising a plurality of small molecules each small molecule linked to a branch of a branch-point; and (iii) a linker linking the substrate binding domain and the branching domain described herein. For example, where a known ELISA method for detecting a small molecule in a sample takes T1 time to perform, the methods describe herein can be used to detect the small molecule in the sample in time T2, where T2 is less than T1 (e.g., with T2 is a third or less than T1, T2 half or less of T1, T2 is at least an order of magnitude less than T2). The rapidity can be, for example, determined by one rate limiting process, such as an incubation time. Without being bound by a specific theory, in some embodiments, the methods described herein can detect a small molecule more rapidly than comparative methods because the sensitivity to the small molecule is higher and less time is required for a detectable signal (e.g., above noise) to be acquired. In some embodiments, the assay can detect a small molecule through the elimination of a step used in the comparative test. For example, in a competitive ELISA assay, a test can include incubating with an analyte binding ligand to allow the competition to be established. Typically, a labeling molecule with a detectable label is then added to allow detection of the analyte binding ligand. By tethering conjugating a detectable label to the analyte binding ligand, the step of adding the labeling molecule is eliminated. In some embodiments the compositions and structures described herein can be used for the detection of a small molecule in less than one hour, e.g., less than 40 min, less than 20 min, less than 10 min or even less than 5 min.

In some embodiments the assay for a small molecule (e.g. a histamine or a DNP assay) includes the immobilization of conjugates to the solid support. In some embodiments the assay includes the immobilization of the detecting molecule (e.g., anti-histamine antibody, or anti-DNP antibody) on the solid support.

### Test sample

In accordance with various embodiments described herein, a test sample, including any fluid or specimen (processed or unprocessed) that is intended to be evaluated for the presence of a small molecule can be subjected to methods, compositions, kits and systems described herein. The test sample or fluid can be liquid, supercritical fluid, solutions, suspensions, gases, gels, slurries, and combinations thereof. The test sample or fluid can be aqueous or non-aqueous.

In some embodiments, the test sample can be an aqueous fluid. As used herein, the term "aqueous fluid" refers to any flowable water-containing material that is suspected of comprising an analyte such as a target small molecule.

In some embodiments, the test sample can include a biological fluid obtained from a subject. Exemplary biological fluids obtained from a subject can include, but are not limited to, blood (including whole blood, plasma, cord blood and serum), lactation products (e.g., milk), amniotic fluids, sputum, saliva, urine, semen, cerebrospinal fluid, bronchial aspirate, perspiration, mucus, liquefied stool sample, synovial fluid, lymphatic fluid, tears, tracheal aspirate, and any mixtures thereof. In some embodiments, a biological fluid can include a homogenate of a tissue specimen (e.g., biopsy) from a subject. In one embodiment, a test sample can comprise a suspension obtained from homogenization of a solid sample obtained from a solid organ or a fragment thereof.

In some embodiments, the test sample can include a fluid or specimen obtained from an environmental source. For example, the fluid or specimen obtained from the environmental source can be obtained or derived from food products or industrial food products, food produce, poultry, meat, fish, beverages, dairy products, water (including wastewater), surfaces, ponds, rivers, reservoirs, swimming pools, soils, food processing and/or packaging plants, agricultural places, hydrocultures (including hydroponic food farms), pharmaceutical manufacturing plants, animal colony facilities, and any combinations thereof.

In some embodiments, the test sample can include a fluid or specimen collected or derived from a biological culture. For example, a biological culture can be a cell culture. Examples of a fluid or specimen collected or derived from a biological culture includes the one obtained from culturing or fermentation, for example, of single- or multi-cell organisms, including prokaryotes (e.g., bacteria) and eukaryotes (e.g., animal cells, plant cells, yeasts, fungi), and including fractions thereof. In some embodiments, the test sample can include a fluid from a blood culture. In some embodiments, the culture medium can be obtained from any source, e.g., without limitations, research laboratories, pharmaceutical manufacturing plants, hydrocultures (e.g., hydroponic food farms), diagnostic testing facilities, clinical settings, and any combinations thereof.

In some embodiments, the test sample can include a media or reagent solution used in a laboratory or clinical setting, such as for biomedical and molecular biology applications. As used herein, the term "media" refers to a medium for maintaining a tissue, an organism, or a cell population, or refers to a medium for culturing a tissue, an organism, or a cell population, which contains nutrients that maintain viability of the tissue, organism, or cell population, and support proliferation and growth.

In some embodiments, the test sample can be a non-biological fluid. As used herein, the term "non-biological fluid" refers to any fluid that is not a biological fluid as the term is defined herein. Exemplary non-biological fluids include, but are not limited to, water, salt water, brine, buffered solutions, saline solutions, sugar solutions, carbohydrate solutions, lipid solutions, nucleic acid solutions, hydrocarbons (e.g. liquid hydrocarbons), acids, gasolines, petroleum, liquefied samples (e.g., liquefied samples), and mixtures thereof

It can be necessary or desired that a test sample, such be preprocessed prior to small molecule detection as described herein, e.g., with a preprocessing reagent. Even in cases where pretreatment is not necessary, preprocess optionally can be done for mere convenience (e.g., as part of a regimen on a commercial platform). A preprocessing reagent can be any reagent appropriate for use with the methods described herein.

The sample preprocessing step generally comprises adding one or more reagents to the sample. This preprocessing can serve a number of different purposes, including, but not limited to, hemolyzing cells such as blood cells, dilution of sample, etc. The preprocessing reagents can be present in the sample container before sample is added to the sample container or the preprocessing reagents can be added to a sample already present in the sample container. When the sample is a biological fluid, the sample container can be a VACUTAINER^{®}, e.g., a heparinized VACUTAINER^{®} .

The preprocessing reagents include, but are not limited to, surfactants and detergents, salts, cell lysing reagents, anticoagulants, degradative enzymes (e.g., proteases, lipases, nucleases, lipase, collagenase, cellulases, amylases and the like), and solvents, such as buffer solutions.

After the optional preprocessing step, the sample can be optionally further processed by adding one or more processing reagents to the sample. These processing reagents can degrade unwanted molecules present in the sample and/or dilute the sample for further processing. These processing reagents include, but are not limited to, surfactants and detergents, salts, cell lysing reagents, anticoagulants, degradative enzymes (e.g., proteases, lipases, nucleases, lipase, collagenase, cellulases, amylases, heparanases, and the like), and solvents, such as buffer solutions. Amount of the processing reagent to be added can depend on the particular sample to be analyzed, the time required for the sample analysis, identity of the small molecule to be detected or the amount of small molecule present in the sample to be analyzed.

It is not necessary, but if one or more reagents are to be added they can present in a mixture (e.g., in a solution, "processing buffer") in the appropriate concentrations. Amount of the various components of the processing buffer can vary depending upon the sample, small molecule to be detected, concentration of the small molecule in the sample, or time limitation for analysis.

Reagents and treatments for processing blood before assaying are also well known in the art, e.g., as used for assays on Abbott TDx, AxSYM^{®}, and ARCHITECT^{®} analyzers (Abbott Laboratories), as described in the literature (see, e.g., Yatscoff et al., Abbott TDx Monoclonal Antibody Assay Evaluated for Measuring Cyclosporine in Whole Blood, Clin. Chem. 36: 1969-1973 (1990), and Wallemacq et al., Evaluation of the New AxSYM Cyclosporine Assay: Comparison with TDx Monoclonal Whole Blood and EMIT Cyclosporine Assays, Clin. Chem. 45: 432-435 (1999)), and/or as commercially available. Additionally, pretreatment can be done as described in U.S. Pat. No. 5,135,875, European Pat. Pub. No. 0 471 293, U.S. Provisional Pat. App. 60/878,017, filed Dec. 29, 2006, and U.S. Pat. App. Pub. No. 2008/0020401, content of all of which is incorporated herein by reference. It is to be understood that one or more of these known reagents and/or treatments can be used in addition to or alternatively to the sample treatment described herein.

After addition of the processing reagents, the sample can be incubated for a period of time, e.g., for at least one minute, at least two minutes, at least three minutes, at least four minutes, at least five minutes, at least ten minutes, at least fifteen minutes, at least thirty minutes, at least forty-five minutes, or at least one hour. Such incubation can be at any appropriate temperature, e.g., room-temperature (e.g., about 16°C to about 30°C), a cold temperature (e.g. about 0°C to about 16°C), or an elevated temperature (e.g., about 30°C to about 95°C). In some embodiments, the sample is incubated for less than about 10 minutes at room temperature (e.g., less than about 8 minutes, less than about 5 minutes).

### Antibody production

Standard methods for antibody as known in the art can be modified to use the detecting molecule described herein.

Briefly, antibody production relies on the *in vivo* humoral response to injected foreign antigens. For example, the antibody production can be made in a mammal such as a mouse, pig or human. Simple immunizations of foreign molecules, viruses or cells can elicit a strong antibody response, but some substances fail to induce a strong response.

The immune system can be manipulated to increase the response by modifying either the antigen or the host. In some embodiments the antigen includes detecting molecule described herein.

Proteins, peptides, carbohydrates, nucleic acids, lipids and many other naturally occurring or synthetic compounds can act as successful immunogens. Peptides, non-protein antigens such as small molecules usually need to be conjugated to a carrier protein (bovine serum albumin or keyhole limpet hemocyanin) to become good immunogens. The conjugation to the carrier provides the required class II T receptor binding sites. In some embodiments the detecting molecule comprising a branching domain linked to a carrier protein is used as the immunogen.

Additionally, immunogens may need to be administered with an adjuvant to ensure a high quality/quantity response. Adjuvants are non-specific stimulators of the immune response. They allow smaller doses of antigen to be used to elicit a persistent antibody response.

Polyclonal antibodies can be made by immunizing with a compound comprising the detecting molecule conjugated to a protein carrier. Repeated immunizations of this antigen at intervals of several weeks stimulates specific B cells to produce large amounts of the anti-antigen. In this embodiment, the blood will contain a variety of antibodies, each to a different epitope on the antigen. The immune-sera can be used in its crude form, where high levels of specific antibodies are present, or the specific antibodies can be isolated from sera components by affinity purification.

To produce monoclonals the same immunization protocol is used but all antibody-forming cells (e.g. B cells) are removed. These are fused with immortal tumor cells to become hybridomas, which are screened for antibody production and performance. The hybridomas that produce antibodies are given clone names, which are uniquely assigned to permit identification. The antibody producing hybridoma cells are cloned by isolation and cultivated using tissue culture. Alternatively, genes coding for antibody production can be cloned into transfection vectors to produce recombinant antibodies. Unlike polyclonal antibodies, monoclonal antibodies are homogenous with defined specificity to one epitope. The antibody secreted by the cells into the culture media can be harvested and used in its crude form, or it can be purified by affinity chromatography.

Without being bound to any specific theory it is believed that antibody production using the detecting molecule described herein can be advantageous because of the multivalent presentation to the T-Cell receptor site.

### Kits comprising a composition described herein

A kit comprising at least one composition described herein is also provided.

In some embodiments, the detecting molecule can be affixed to a solid substrate. Non-limiting examples of the first or the solid substrate includes, but is not limited to, a nucleic acid scaffold, a protein scaffold, a lipid scaffold, a dendrimer, microparticle or a microbead, a nanotube, a microtiter plate, a medical apparatus or implant, a microchip, a filtration device, a membrane, a diagnostic strip, a dipstick, an extracorporeal device, a mixing element (e.g., a spiral mixer), a microscopic slide, a hollow fiber, a hollow fiber cartridge, and any combination thereof.

In some embodiments, the kit can further comprise a detecting molecule capable of detecting a first plurality of small molecule. In some embodiments, the kit can further comprise a second detecting molecule capable of ducting second plurality, different from the first plurality, of small molecules.

The kits can include any of the preprocessing reagents as described herein.

In addition to the above mentioned components, any embodiments of the kits described herein can include informational material. The informational material can be descriptive, instructional, marketing or other material that relates to the methods described herein and/or the use of the aggregates for the methods described herein. For example, the informational material can describe methods for using the kits provided herein to perform an assay for detection of a target entity, e.g., a small molecule. The kit can also include an empty container and/or a delivery device, *e.g.,* which can be used to deliver or prepare a test sample to a test container.

The informational material of the kits is not limited in its form. In many cases, the informational material, *e.g.,* instructions, is provided in printed matter, *e.g.,* a printed text, drawing, and/or photograph, *e.g.,* a label or printed sheet. However, the informational material can also be provided in other formats, such as Braille, computer readable material, video recording, or audio recording. In another embodiment, the informational material of the kit is a link or contact information, *e.g.,* a physical address, email address, hyperlink, website, or telephone number, where a user of the kit can obtain substantive information about the formulation and/or its use in the methods described herein. Of course, the informational material can also be provided in any combination of formats.

In some embodiments, the kit can contain separate containers, dividers or compartments for each component and informational material. For example, each different component can be contained in a bottle, vial, or syringe, and the informational material can be contained in a plastic sleeve or packet. In other embodiments, the separate elements of the kit are contained within a single, undivided container. For example, a collection of the magnetic particles is contained in a bottle, vial or syringe that has attached thereto the informational material in the form of a label.

*Embodiments of various aspects described herein can be defined in any of the following numbered paragraphs:*
1. A compound comprising:
   (i) a substrate binding domain;
   (ii) a branching domain comprising a plurality of small molecules each small molecule linked to a branch of a branch-point; and
   (iii) a linker linking the substrate binding domain and the branching domain.
2. The compound of paragraph 1, wherein the small molecules independently have a molecular weight of less than 1,000 Da.
3. The compound of any of paragraphs 1 or 2, wherein the small molecules independently have a molecular weight of higher than 50 g/mol.
4. The compound of any of paragraphs 1-3, wherein the small molecules independently have a molecular weight of between about 50 and 600 g/mol.
5. The compound of any of paragraphs 1-4, wherein the small molecule is selected from the group consisting of amino acids, nucleosides, saccharides, steroids, hormones, pharmaceutically derived drugs, or derivatives and conjugates thereof.
6. The compound of any of paragraphs 1-5, wherein the small molecules are histidine, a histadine-phenylalanine dimer, or dinitrophenol (DNP).
7. The compound of any of paragraphs 1-6, wherein the linker has a length between 5 and 200 angstroms.
8. The compound of any of paragraphs 1-7, wherein the linker comprises a polyethylene glycol (PEG) having a molecular weight of less than 2,000 Da.
9. The compound of any of paragraphs 1-8, wherein the linker comprises a PEG having from 2 to 45 repeat units.
10. The compound of any of paragraphs 1-9, wherein the branch-point comprises at least one lysine.
11. The compound of any of paragraphs 1-10, wherein at least one small molecule is linked to the alpha-amino group the at least one lysine and at least one small molecule is linked to the epsilon-amino group of the at least one lysine.
12. The compound of any of paragraphs 1-11, wherein the branch-point comprises a first lysine linked to a second lysine, and wherein the carboxyl group of the first lysine is linked to the epsilon-amino group of second lysine.
13. The compound of any of paragraphs 1-12, wherein the branch-point comprises a first lysine, a second lysine and a third lysine, and wherein the carboxyl group of the first lysine is linked to the epsilon-amino group of the second lysine, and the carboxyl group of the third lysine is linked to the alpha-amino group of the first or second lysine.
14. The compound of any of paragraphs 1-13, wherein the branch-point is selected from the group consisting of 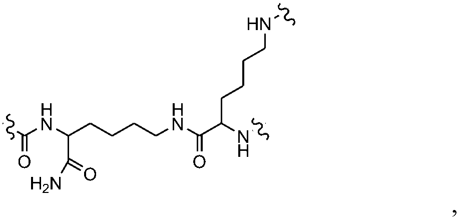 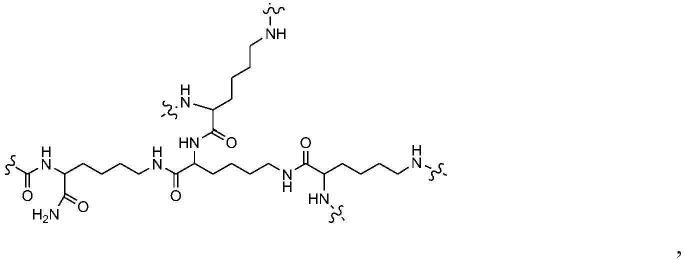 and 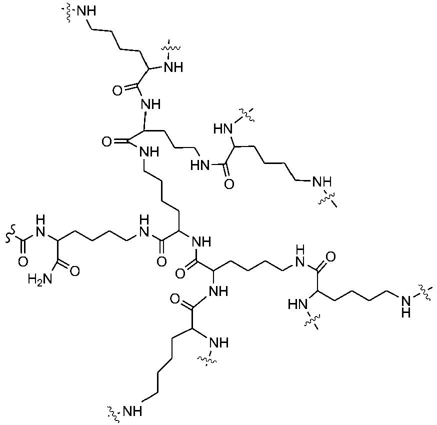
15. The compound of any of paragraphs 1-14, wherein the branching domain comprises from 2 to 20 small molecules linked to the branch-point.
16. The compound of any of paragraphs 1-15, wherein the branching domain is selected from the group consisting of: 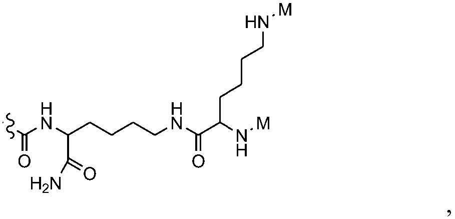 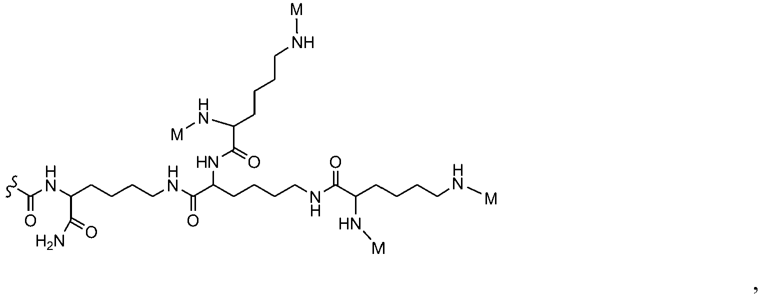 and 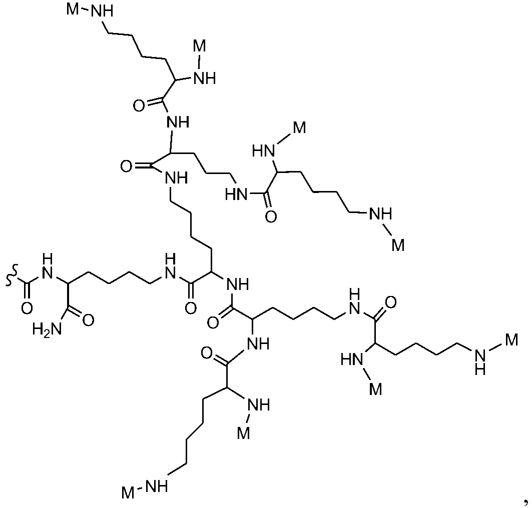 wherein each M is a small molecule.
17. The compound of any of paragraphs 1-16, wherein the branching domain is selected from the group consisting of: 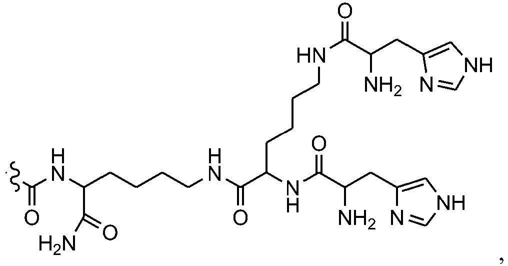 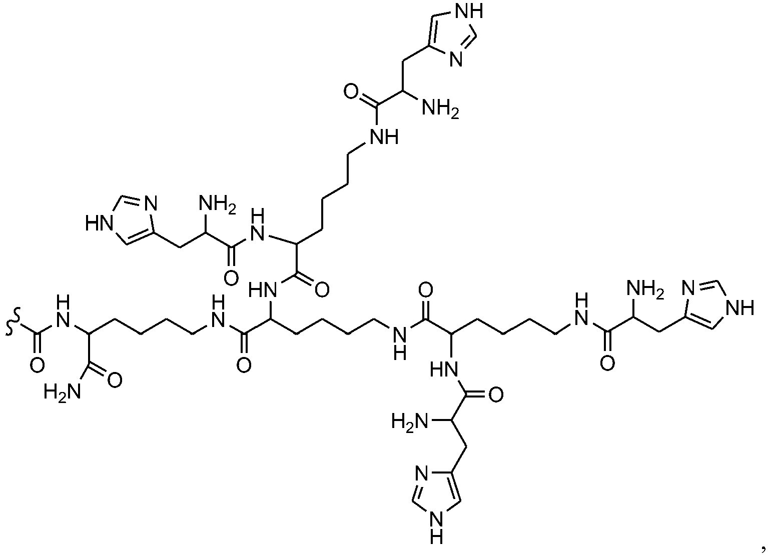 and 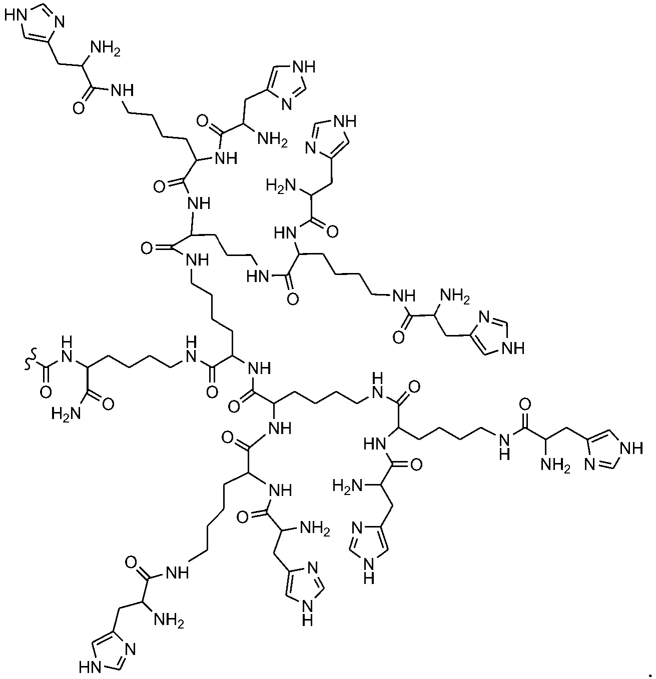
18. The compound of any of paragraphs 1-17, wherein the branching domain is selected from the group consisting of: 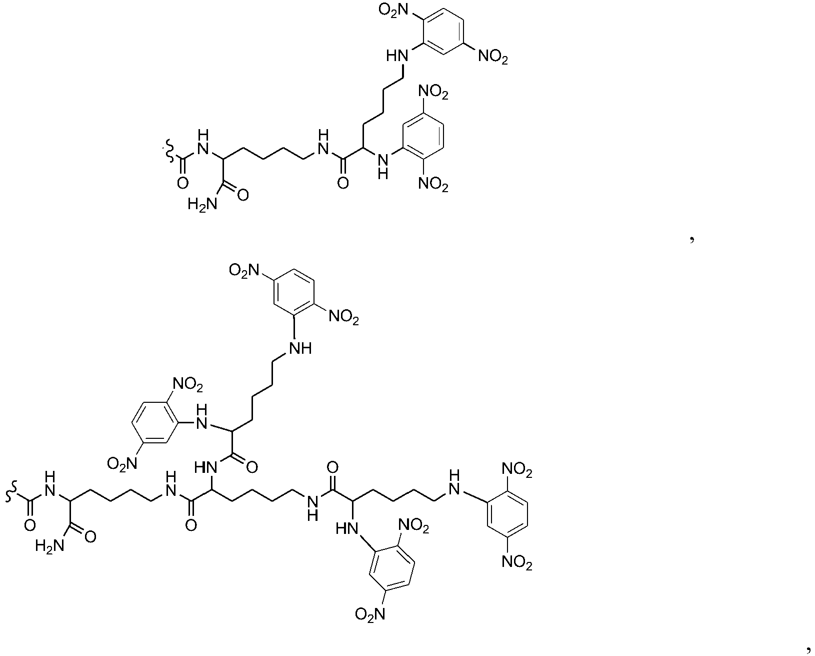 and 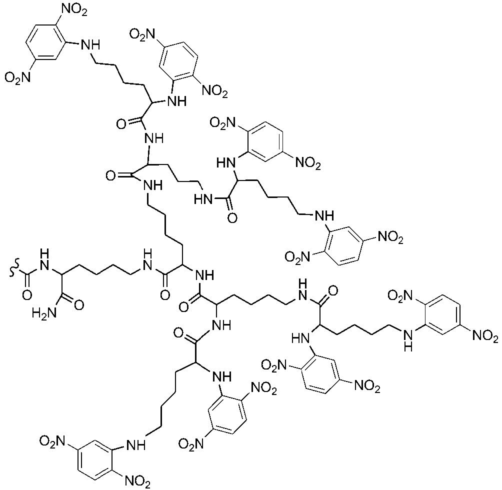
19. The compound of any of paragraphs 1-18, wherein the substrate binding domain comprises a reactive group or one member of a binding pair.
20. The compound of paragraph 19, wherein the reactive group is selected from the group consisting of alkyl halide, aldehyde, amino, bromo or iodoacetyl, carboxyl, hydroxyl, epoxy, ester, silane, thiol, and the like.
21. The compound of paragraph 19 or 20, wherein the binding pair is biotin-avidin, biotin-streptavidin, complementary oligonucleotide pairs capable of forming nucleic acid duplexes, a thiol-maleimide pair, a first molecule that is negatively charged and a second molecule that is positively charged.
22. The compound of any of paragraphs 1-21, wherein the substrate binding domain comprises a thiol group or a biotin molecule.
23. The compound of any of paragraphs 1-22, wherein the branching domain comprises: 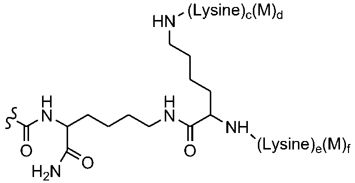 wherein,
   d + f ≥ 2 (e.g., between about 2 and 100), d ≥ c, and e ≥ f, wherein c, d, e and f are integers and each M is a small molecule.
24. The compound of paragraph 23, wherein M is histidine or dinitrophenol.
25. The compound of any of paragraphs 1-24, wherein the branching domain has the formula C(x)ₐM_{b},
   wherein:
   C is a sub unit of the branching domain having a maximum of possible x branches, and the branch-point comprises one or more sub unit C and at least one subunit C is attached to the linker through a branch;
   M is a small molecule attached to the subunit C through a branch;
   a is an integer ≥ 1; and
   b is an integer ≥ 2, provided that b ≤ (a)(x-2) + 1.
26. The compound of any of paragraphs 1-25, wherein the compound is linked to a substrate via the substrate binding domain.
27. The compound of paragraph 26, wherein the substrate is a nucleic acid scaffold, a protein scaffold, a lipid scaffold, a dendrimer, a microparticle or a microbead, a nanotube, a microtiter plate, an electrode, a medical apparatus or implant, a microchip, a filtration device, a membrane, a diagnostic strip, a dipstick, an extracorporeal device, a microscopic slide, a hollow fiber, a hollow fiber cartridge, an electrode surface, an ELISA plate or any combinations thereof.
28. The compound of any of paragraphs 26-27, wherein the substrate is a microparticle or a microbead, a microtiter plate, an electrode surface, a membrane, a diagnostic strip, a dipstick, an ELISA plate or a microscopic slide.
29. The compound of paragraph 26-28, wherein a surface of the substrate is coated with a proteinaceous material, wherein the proteinaceous material can optionally be reversibly or non-reversibly denatured and/or cross-linked.
30. The compound of paragraph 29, wherein the proteinaceous material is denatured BSA which is cross-linked with glutaraldehyde.
31. A method for detecting presence of an analyte in a sample, the method comprising:
   (i) contacting a sample suspected of comprising an analyte with a compound of any of paragraphs 1-28; and
   (ii) detecting binding of an analyte binding molecule to the compound.
32. The method of paragraph 31, wherein the analyte binding molecule is an antibody.
33. The method of any of paragraphs 31 or 32, wherein said detecting of step (ii) comprises producing a chromogenic, fluorescence or electrochemical signal.
34. The method of any of paragraphs 31-33, wherein the analyte binding molecule comprises a detectable label.
35. The method of any of paragraphs 31-34, wherein said detecting step comprises contacting the sample from (i) with a molecule capable of binding with the analyte binding molecule and comprises a detectable label.
36. The method of any of paragraphs 31-35, wherein the analyte is histamine or dinitrophenol.
37. The method of any of paragraphs 31-36, wherein the compound selected from any one of paragraphs 1-30 is linked to an electrode surface by the substrate binding domain and the analyte binding molecule includes an electroactive component, and wherein
   the analyte binding molecule is detected by the electrode when the electroactive component is proximate to the electrode.
38. The method of paragraph 37, wherein the linker length is greater than 5 Å and less than 200 Å.
39. The method of paragarph 37 or 38, wherein the electrode detects the analyte binding molecule by direct redox reaction with the electroactive component or by a sacrificial redox active species.
40. The method of any of paragraphs 37-39, wherein the analyte binding molecule is an antibody specific to the analyte, and the electroactive component is a biotinylated detection antibody conjugated to streptavidin-polyHRP and the electrode detects the sacrificial redox active agent 3,3',5,5'-Tetramethylbenzidine (TMB).
41. A method for selecting a ligand capable of binding a small molecule, the method comprising:
   (i) contacting a test ligand with a compound of any of paragraphs 1-28; and
   (ii) detecting binding of the test ligand with the compound in the presence and in the absence of the small molecule, and
   selecting the test ligand having reduced binding in the presence of the small molecule.
42. The method of paragraph 41, wherein the test ligand is selected from the group consisting of antibodies, adnectins, ankyrins, antibody mimetics and other protein scaffolds, aptamers, nucleic acid (e.g., an RNA or DNA aptamer), proteins, peptides, oligosaccharides, polysaccharides, lipopolysaccharides, cellular metabolites, cells, viruses, subcellular particles, haptens, pharmacologically active substances, alkaloids, steroids, vitamins, amino acids, avimers, peptidomimetics, hormone receptors, cytokine receptors, synthetic receptors, sugars and molecularly imprinted polymer.
43. The method of paragraph 41, wherein the ligand is an antibody.
44. The method of any of paragraphs 41-43, wherein said detecting of step (ii) comprises producing a chromogenic, fluorescence or electrochemical signal.
45. The method of any of paragraphs 41-44, wherein the ligand comprises a detectable label.
46. The method of paragraph 45, wherein the detectable label is a chromogenic, fluorescent or redox active group.
47. The method of any of paragraphs 41-46, wherein said detecting step comprises contacting the ligand from (i) with a molecule capable of binding with the ligand and comprises a detectable label.
48. A method for raising antibodies specific to a small molecule, the method comprising contacting T cells with the compound of any of claims 1-30.

### Some selected definitions

For convenience, certain terms employed in the entire application (including the specification, examples, and appended claims) are collected here. Unless defined otherwise, all technical and scientific terms used herein have the same meaning as commonly understood by one of ordinary skill in the art to which this invention belongs.

As used herein, the term "binding" or "bound" generally refers to a reversible binding of one agent or molecule to another agent or molecule via, e.g., van der Waals force, hydrophobic force, hydrogen bonding, and/or electrostatic force. The binding interaction between an agent or molecule and another agent or molecule can be described by a dissociation constant (K_{d}) or association constant (K), which is further described below. For example, in the presence of a higher affinity binder (e.g., a small molecule), the dectecting molecule can be displaced by the higher affinity binder (e.g., the small molecule). As used herein, the term "effective binding affinity" generally refers to an overall binding property of a first agent (e.g., a detecting molecule or small molecule) interacting with a second agent (e.g., an antibody) under a specific condition, and the overall binding property is typically dependent on intrinsic characteristics of the first agent and the second agent including, but not limited to, surface composition of the first agent and/or the second agent (e.g., but not limited to, density of target-binding molecules present on the surface of the target-binding agent as well as the surrounding/ambient condition for the binding interaction, e.g., but not limited to, concentration of the first agent and/or the second agent, and/or the presence of a third agent (e.g., a blocking agent, an interfering agent and/or a target entity) during the binding interaction between the first and the second agents. Different measures of an effective binding affinity of an agent are known in the art. In some embodiments, the effective binding affinity of a first agent for a second agent can be indicated by a dissociation constant (K_{d}) for binding of the first agent to the second agent. The dissociation constant (K_{d}) is an equilibrium constant that generally measures the propensity of a bound complex to separate (dissociate) reversibly into separate agents. In these embodiments, a higher dissociation constant indicates a lower effective binding affinity. Alternatively, the effective binding affinity of a first agent for a second agent can be indicated by an association constant (K) for binding of the first agent to the second agent. The association constant (K) is the inverse of the dissociation constant (K_{d}), i.e., a higher association constant indicates a higher effective binding affinity.

As used in this specification and the appended claims, the singular forms "a," "an," and "the" include plural references unless the context clearly dictates otherwise. Thus for example, references to "the method" includes one or more methods, and/or steps of the type described herein and/or which will become apparent to those persons skilled in the art upon reading this disclosure and so forth. Similarly, the word "or" is intended to include "and" unless the context clearly indicates otherwise.

Other than in the operating examples, or where otherwise indicated, all numbers expressing quantities of ingredients or reaction conditions used herein should be understood as modified in all instances by the term "about." The term "about" when used to described the present invention, in connection with percentages means ± 1%.

In one aspect, the present invention relates to the herein described compositions, methods, and respective component(s) thereof, as essential to the invention, yet open to the inclusion of unspecified elements, essential or not ("comprising"). In some embodiments, other elements to be included in the description of the composition, method or respective component thereof are limited to those that do not materially affect the basic and novel characteristic(s) of the invention ("consisting essentially of"). This applies equally to steps within a described method as well as compositions and components therein. In other embodiments, the inventions, compositions, methods, and respective components thereof, described herein are intended to be exclusive of any element not deemed an essential element to the component, composition or method ("consisting of").

It should be understood that this invention is not limited to the particular methodology, protocols, and reagents, etc., described herein and as such may vary. The terminology used herein is for the purpose of describing particular embodiments only, and is not intended to limit the scope of the present invention, which is defined solely by the claims.

All patents, patent applications, and publications identified are expressly incorporated herein by reference for the purpose of describing and disclosing, for example, the methodologies described in such publications that might be used in connection with the present invention. These publications are provided solely for their disclosure prior to the filing date of the present application. Nothing in this regard should be construed as an admission that the inventors are not entitled to antedate such disclosure by virtue of prior invention or for any other reason. All statements as to the date or representation as to the contents of these documents is based on the information available to the applicants and does not constitute any admission as to the correctness of the dates or contents of these

### EXAMPLES

The following examples illustrate some embodiments and aspects of the invention. It will be apparent to those skilled in the relevant art that various modifications, additions, substitutions, and the like can be performed without altering the spirit or scope of the invention, and such modifications and variations are encompassed within the scope of the invention as defined in the claims which follow. The following examples do not in any way limit the invention.

### Initial Experiments

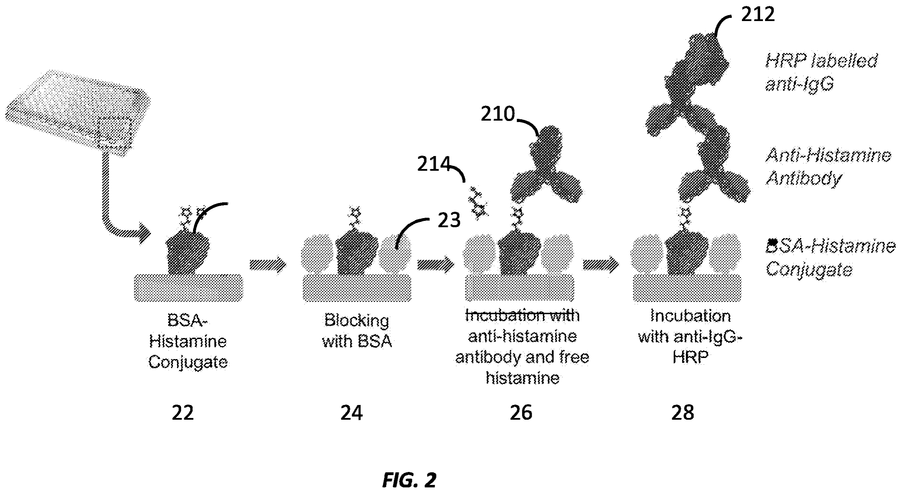

Multiple commercially available antibodies that were developed using the conventional BSA-histamine conjugate approach were screened. **FIG. 2** presents a schematic of the ELISA protocol used for antibody screening with the BSA-histamine conjugate. The BSA-histamine conjugate is a random conjugation where multiple histamine molecules are conjugated to each BSA particle in a random fashion. Briefly, high binding ELISA plates were first functionalized with BSA-histamine **21** by passive absorption step **22** and then the plates were blocked with BSA **23** to reduce non-specific binding and background signal, in a blocking step **24.** Thereafter, different commercial mouse anti-histamine antibodies **210** were incubated for 1 hour at room temperature on a shaker, step **26.** Finally, rabbit anti- mouse IgG labelled with horse radish peroxidase(HRP) **212** was used to detect the bound antibodies, step **28.** The presence of HRP was revealed using 3,3',5,5'-Tetramethylbenzidine (TMB) substrate and quantified colorimetrically at 650 nm. In an alternative protocol, histamine **214** is also added in step **26.**

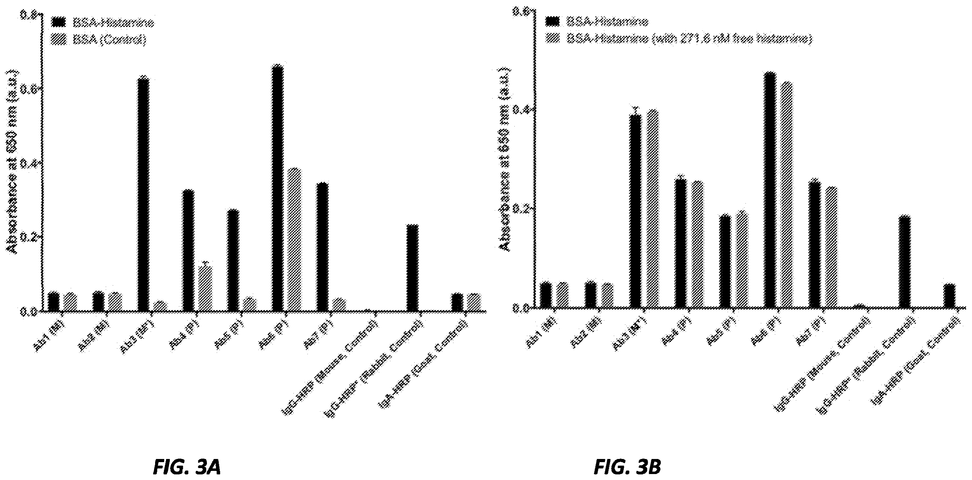

When the commercial anti-histamine antibodies were screened using BSA-histamine in a reverse phase assay strategy (**FIG. 2** without free histamine), all of the antibodies demonstrated affinity towards the immobilized BSA-histamine as shown in **FIG. 3A** for Ab1, Ab2, Ab3, Ab4, Ab5, Ab6, Ab7 and controls IgG-HRP(Mouse), IgG-HRP(Rabbit), IgA-HRP(Goat) where Ab1 (GTX 12894) and Ab2 (MAB5408) are mouse monoclonal IgA antibody from Genetex and Millipore respectively, Ab3 (MAB5408) is a IgG mouse monoclonal antibody from Cloudclone. Ab 4 (PAA927Ge01), 5 (H5080-06A), 6 (GTX12840), 7 (H7403) are polyclonal rabbit IgG antibodies from Cloudclone, US biological, Genetex and Sigma respectively. Anti- mouse IgG-HRP (115-035-008) and anti-rabbit IgG (111-035-008) were purchased from Jackson Immuno Research Laboratories while, anti-IgA HRP was purchased from Invitrogen (62-6720).

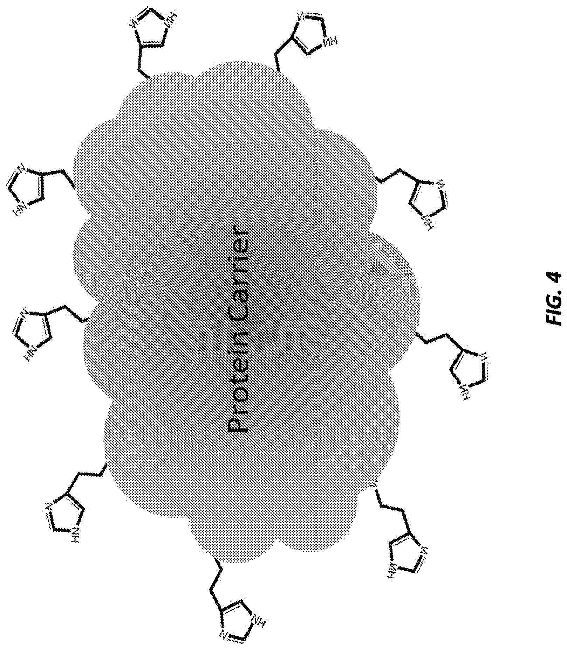

Importantly, however, most of the antibodies also showed significant binding to the BSA control, indicating a lack of binding specificity. These antibodies were then tested with 271 nM of free histamine (**FIG. 2** with free histamine), nearly all assay results were negative as the antibodies failed to detect the presence of free histamine (**FIG. 3B**). This could be attributed to the antibodies binding very strongly to the imidazole ring of the BSA-histamine conjugate and potentially to the nearby peptide domains in BSA that are exposed when histamine is cross-linked to BSA as depicted in **Fig. 4****.** As previously noted, similar poor sensitivity for free histamine when using commercially available anti-histamine antibodies in terms of specificity and sensitivity has been reported [Mattsson et al., 2017. "Challenges in Developing a Biochip for Intact Histamine Using Commercial Antibodies," Chemosensors, 5(4), p.33.

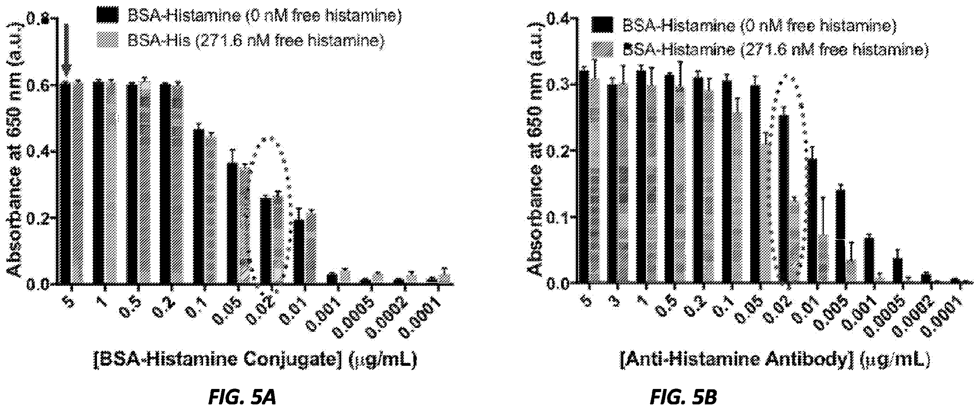

Experiments were conducted with commercial antibody (Ab3) to optimize the assay conditions. The initial study was performed on plates modified with the BSA-histamine conjugate to develop a competitive histamine ELISA assay which required that the concentrations of both the target conjugate and anti-histamine antibody be optimized. The concentration of BSA-histamine conjugate used to prepare the plates was varied between 5 µg mL⁻¹ and 0.1 ng mL⁻¹ and the assay was carried out using a constant concentration of free histamine (276.1 nM) and soluble anti-histamine antibody (5 µg mL⁻¹). When free histamine and anti-histamine antibody were incubated on the plate, the free histamine should compete with the immobilized BSA-histamine for binding to the antibody. Following a 60 min incubation step, the HRP-tagged secondary antibody was introduced to label the anti-histamine antibody bound at the plate surface (**FIG. 2**). As shown in **Fig. 5A****,** the results showed no difference in signal change when free histamine was present, indicating a lack of sensitivity for the histamine molecule itself in this assay. Thereafter, the effect of anti-histamine antibody concentration was studied keeping the BSA-histamine conjugate fixed at 0.02 µg mL⁻¹. The results presented in **Fig. 5B** show that the signal change in the absence or presence of free histamine in the sample increased as the antibody concentration is lowered. The concentration of anti-histamine antibody was fixed at 0.02 µg mL⁻¹ as this was the condition at which both the signal intensity and the difference between the two histamine concentrations tested was the highest.

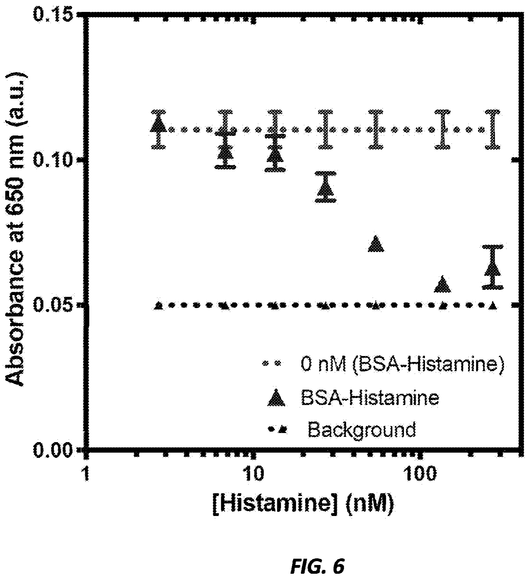

**Fig. 6** shows the calibration curve obtained using the optimized anti-histamine antibody and BSA-histamine conjugate concentrations. The intensity of the signal measured for the BSA-histamine conjugate was low (0.11 AU at 650 nm) and the signal change between the different concentrations did not differ significantly. Furthermore, the assay reached saturation at 134 nM of free histamine resulting in a narrow dynamic range. This assay was also found to exhibit poor reproducibility. Although the assay performed better than earlier versions, and could now be used to detect free histamine, the sensitivity was still lower than required for clinical applications (5-100 nM), and the antibodies still continued to exhibit stronger affinity towards the histamine conjugate.

### Exemplifications of some embodiments of the Invention

During the initial experiments (e.g., data as depicted by **FIG 3A and 3B****),** it was evident that the antibody exhibited stronger affinities towards the histamine-BSA conjugate (**FIG. 4**) than to the free histamine molecule that was the target of interest for quantification. As a result, when such antibodies were utilized in the development of competitive immunoassays, they failed to exhibit specific binding to free histamine.

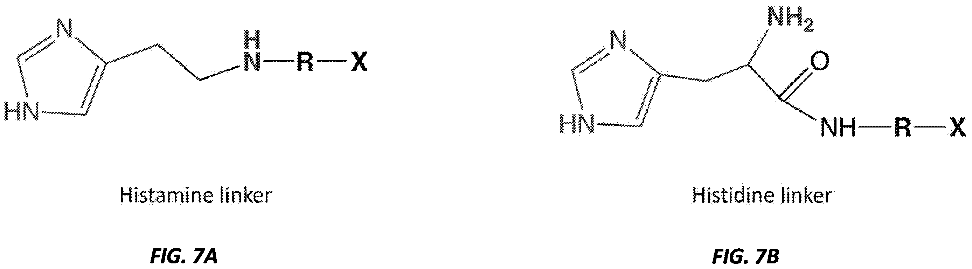

To address the lower affinity for free histamine, a new molecule was designed and synthesized containing a long linker region (X) with a histidine molecule at its end (**FIG. 7B**). Histidine, only differs from histamine by the presence of one additional carboxylate group at its end, and when its terminal amine group becomes covalently linked to the linker, it effectively exposes the remaining portion of the structure that is equivalent to the entire histamine molecule. This is in contrast to linking histamine itself directly to a linker or conjugate through its amine group, which then only exposes a portion of the histamine structure (**FIG. 7A**). The additional carboxylate group of histidine can be conjugated to a linker, such as a polyethylene glycol (PEG) polymer chain, and this results in a conjugate that mimics free histamine better as both the imidazole ring and the amine group are now available for the antibody to bind.

### I. Design and Synthesis of Histidine-Linker conjugates

Two different histidine conjugated linkers attached to a biotin were prepared having the **Structure 1** and **Structure 2** shown here. **Structure** 1 shows a monovalent Biotin-PEG-mono-histidine and **Structure 2** shows a multivalent Biotin-PEG-dual-histidine exposing two histamines per linker.

### Structure 1, Biotin-PEG-mono-histidine:

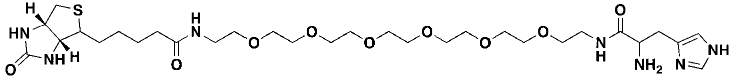

### Structure 2, Biotin-PEG-dual-histidine:

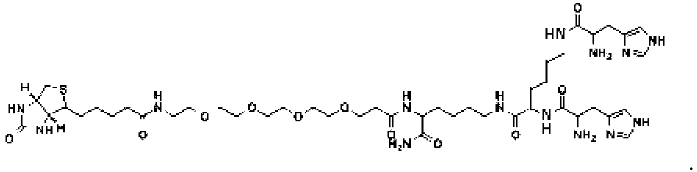

The biotin-PEG-mono-histidine linker (**Structure 1**) was synthesized in solution. Briefly, the primary amine of histidine was first protected with fluorenylmethyloxycarbonyl (FMOC) and the carboxylic acid group activated by 2-(1H-benzotriazole-1-yl)-1,1,3,3-tetramethyluronium hexafluorophosphate (HBTU), and mixed with biotinylated PEG₆-amine linker in dimethylformamide (DMF) with addition of N,N-diisopropylethylamine (DIEA) and incubated at room temperature. After the reaction was completed, the DMF was removed using a rotavapor, the FMOC protection group on the primary amine of the histidine was cleaved with 20% Piperidine, and the protection on the tertiary amine of the histidine was cleaved by Trifluoroacetic acid (TFA). The product was purified by reverse phase high-performance liquid chromatography on a C18 column and characterized by liquid chromatography-mass spectrometry (LC-MS).

To assess the potential added effects of linkers with multivalency, a dual version of the biotin conjugated histidine linker was synthesized (**Structure 2**). Biotin-PEG-dual-histidine linker was synthesized on solid phase peptide synthesis system using FMOC chemistry. First, ivDde protected lysine was attached to the rink amide resin, and then biotin-PEG₄-COOH was attached to the primary amine of the lysine. ivDde group was cleaved from epsilon amine of the lysine using 2% hydrazine in DMF, and then another FMOC protected lysine was attached to that side. Removing of FMOC groups from the lysine allowed to attach two histidine molecules per linker. FMOC protection of the histidine molecules was removed by using 20% piperidine, and the product molecule was cleaved from the resin using TFA. The final product was purified using a C18 column on RP-HPLC system, and characterized by using LC-MS. For conciseness Biotin-PEG₆-mono Histidine (**Structure 1**) and Biotin-PEG₄-dual Histidine (**Structure 2**) can be referred to herein as "PEG-mono histidine" and "PEG-dual histidine," respectively.

### II. Multivalent target approach to enhance sensitivity of competitive histamine immunoassays

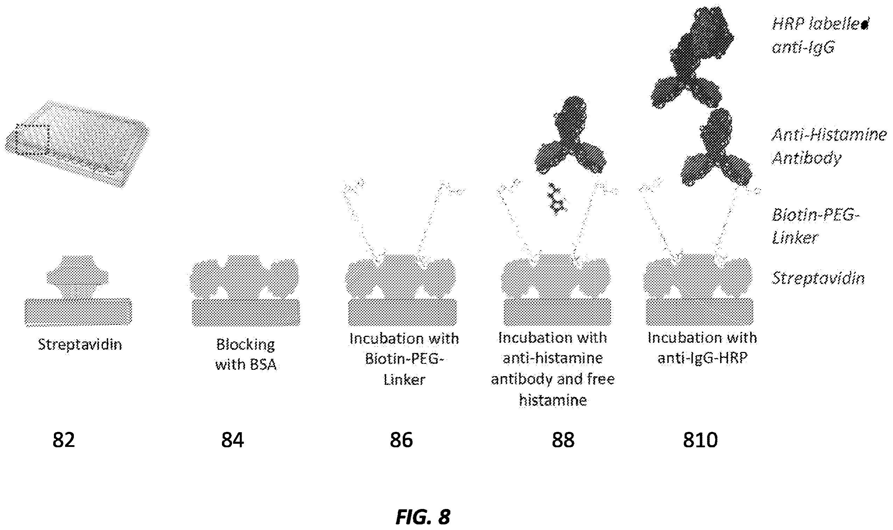

To identify an antibody that recognizes free histamine with improved (lower) binding affinities, we used the PEG-mono-histidine linker (**Structure 1**) as a target with antibodies that we previously screened with BSA-histamine, i.e., the antibodies liste in **FIG. 3A****.** Briefly, as shown in **FIG. 8****,** high binding ELISA plates were first functionalized with 5 µg ml⁻¹ streptavidin by passive absorption **82** and the resulting plates blocked with 1% BSA **84** to reduce non-specific binding and background signal. The monovalent histidine linker (25 µM) **86** was added for 1 hour, and then 5 µg ml⁻¹ anti-histamine antibody was incubated with different concentrations of soluble histamine for 1 hour at room temperature on a shaker **88.** Finally, anti-IgG labelled HRP 810 was used to detect the bound antibodies. The presence of HRP was revealed using 3,3',5,5'-Tetramethylbenzidine (TMB) substrate and quantified colorimetrically at 650 nm.

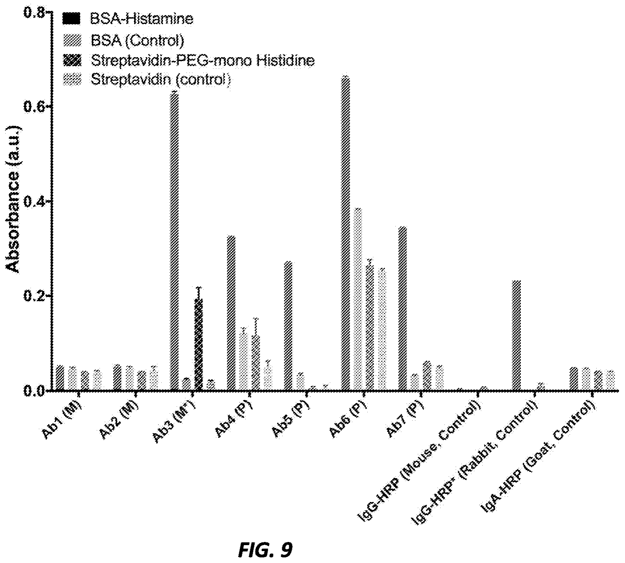

A comparison of **FIG. 8** and **FIG.2** illustrates the difference in mechanism of antibody binding to the PEG-mono histidine versus the BSA-histamine conjugate. The graph shown in **FIG. 9**-highlights that many commercial antibodies fail to recognize the PEG-mono histidine or exhibited significant binding to streptavidin, which was used as a control. As demonstrated, using the PEG-mono histidine linker, the antibodies that have affinity towards the free histamine molecule is efficiently narrowed down with minimum cross reactivity for controls to a single commercial antibody (Antibody 3).

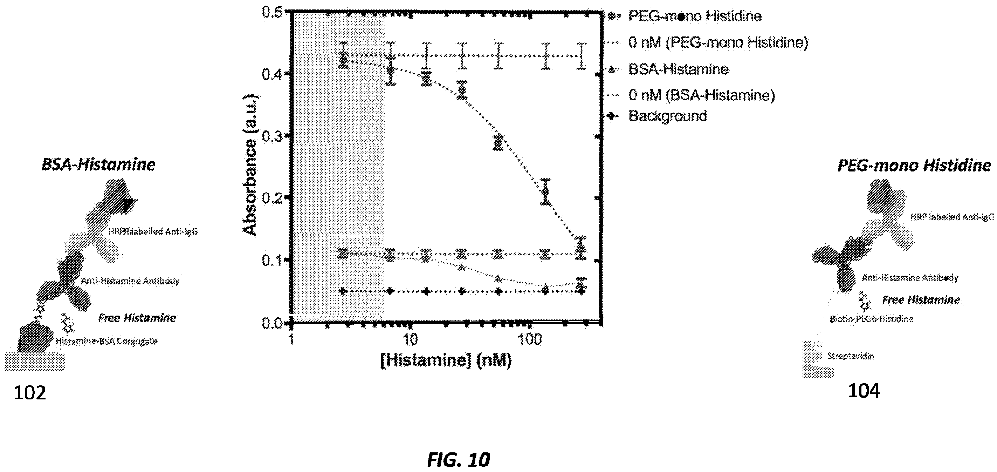

**FIG. 10** compares calibration curves obtained using free histamine as a competitor with immobilized BSA-histamine versus Biotin-PEG-mono-histidine. The PEG-histidine strategy was more versatile and was found to work over a much broader range of antibody and PEG-histidine concentrations. A BSA-Histamine -HRP labelled Anti IgG complex 102 and PEG-mono Histidine - HRP labelled Anti IgG complex **104,** both on a substrate surface and in the presense of free histmain, are shown to the left and right of the calibration curve in **FIG. 10****.** For the conditions tested, the results obtained with PEG-histidine ligand was significantly improved both in terms of signal enhancement and sensitivity; a wider dynamic range was also obtained.

Increasing the number of conjugated histidine molecules at the end of the linker can further increase the sensitivity of the assay by exposing multiple histamines and hence create a multivalent linker. Multivalent targets in assays have been previously reported; however, this was accomplished using a protein carrier modified with multiple target molecules. In contrast, the present approach creates multivalency by modifying the end of a single small molecule linker with multiple histidine moieties with the objective of enhancing sensitivity to histamine.

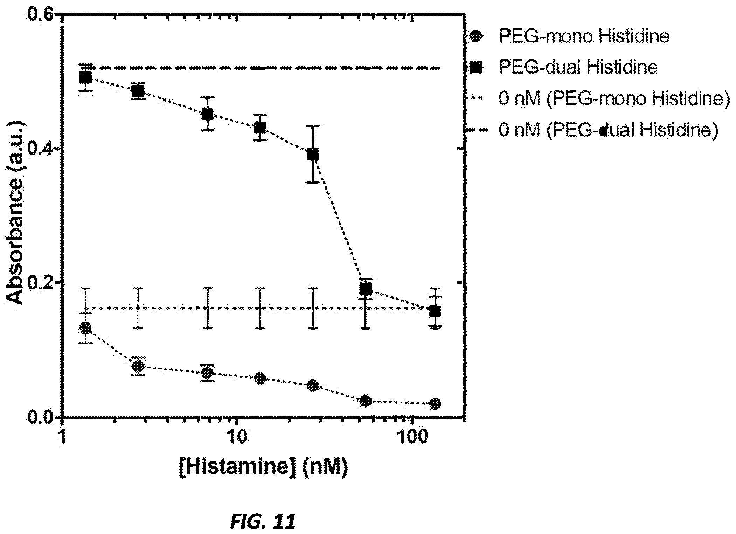

As a proof of principle, a comparison of the performance of PEG-mono-histidine and PEG-dual-histidine was made, the results of which are presented in **FIG. 11****.** The calibration curve shows a significant improvement in the antibody binding specificity and sensitivity when using the dual histidine conjugate as compared to the single histidine conjugate. Without being bound by any specific mechanism, it is proposed that this improvement in sensitivity is the result of the two full histamine molecules competing with the same epitope on the antibody therefore decreasing affinity and consequently leading to a stronger interaction with free histamine. Both linkers demonstrated sensitivity in the grey region which represents the lower limit of the clinically relevant range of sensitivity in biological fluids, such as plasma, which is <5 nM.

Further improvement of the detection sensitivity using more complex multivalent linkers can be obtained. For example, two more linkers with four and eight histidine. Structure 3 shows a Biotin-PEG-four-histidine linker and Structure 4 shows a Biotin-PEG-octo-histidine linker.

### Structure 3, Biotin-PEG-four histidine:

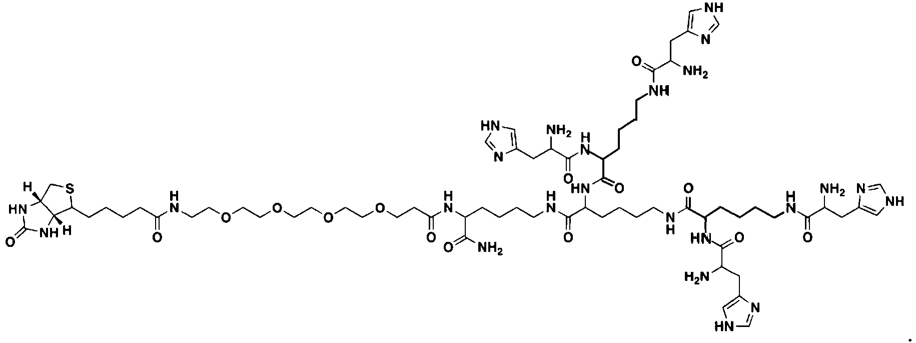

### Structure 4, Biotin-PEG-octo-histidine:

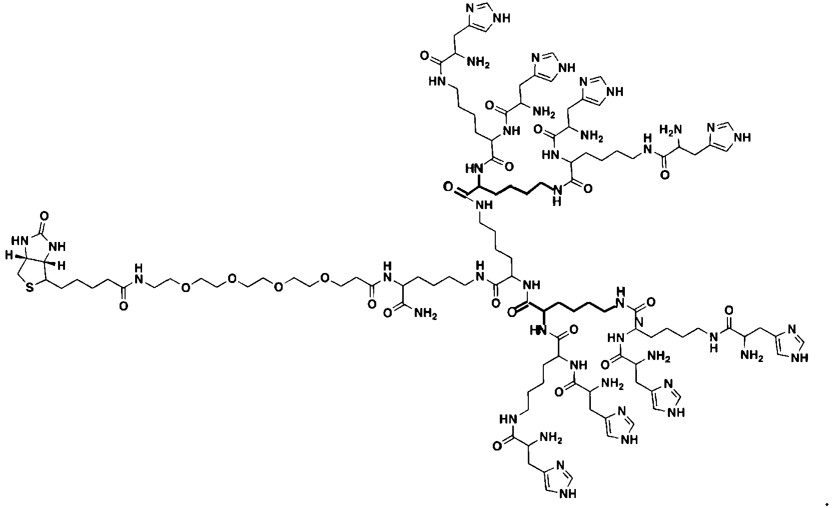

Using a multivalent approach, combined with the design of a linker chemistry that exposes the structure of the free molecule of interest upon protein conjugation, can be used to detect other small molecules, and thereby solve other challenging detection and diagnostic problems. Importantly, the same monovalent or multivalent linkers can be used as antigens for generating more specific antibodies against small molecules.

In an alternate arrangement to that shown by FIG. 8, the ELISA plates can be functionalized with an anti-histamine antibody. A detection molecule such as any one of structures 1-4 can be attached to a detection label (e.g., to a particle/or directly to a fluorophore). In this alternate arrangement the analyte to be detected, e.g., histamine, is in solution with the detection molecule.

Briefly, as shown in **FIG. 8****,** high binding ELISA plates were first functionalized with 5 µg ml⁻¹ streptavidin by passive absorption **82** and the resulting plates blocked with 1% BSA **84** to reduce non-specific binding and background signal. The monovalent histidine linker (25 µM) **86** was added for 1 hour, and then 5 µg ml⁻¹ anti-histamine antibody was incubated with different concentrations of soluble histamine for 1 hour at room temperature on a shaker **88.** Finally, anti-IgG labelled HRP **810** was used to detect the bound antibodies. The presence of HRP was revealed using 3,3',5,5'-Tetramethylbenzidine (TMB) substrate and quantified colorimetrically at 650 nm.

### III. Application of linker for the development of electrochemical biosensor.

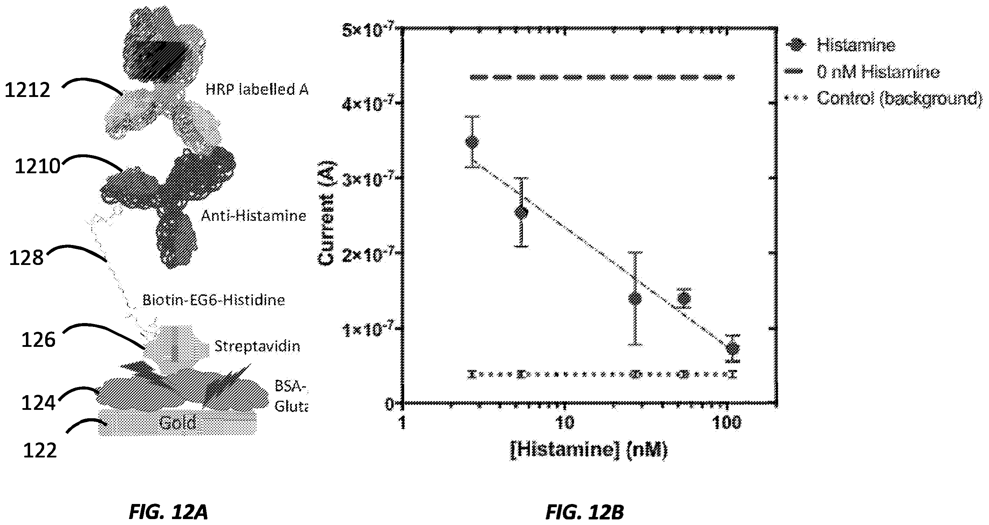

The Immuno-assay developed on the ELISA plates was translated to an electrochemical platform to provide a biosensor for free histamine detection. The lowest concentration tested with the sensor was 2.7 nM which generated a statistically significant difference from the background. **FIG. 12A** shows schematically from bottom to top a Gold electrode **122,** BSA-Glut **124,** streptavidin **126,** Biotin-PEG₆-Histidine conjugate **128,** Anti-histamine **1210** and HRP labeled detection Antibody **1212.** **FIG. 12B** is a semi-log plot showing the calibration curves for the electrochemical sensor using the structure depicted by **FIG. 12A****.** Detection occurs as free histidine competes and displaces Biotin-PEG₆-Histidine conjugate from the anti-histamine.

### IV. Comparison of PEG-mono-Histamine with PEG-mono-Histidine.

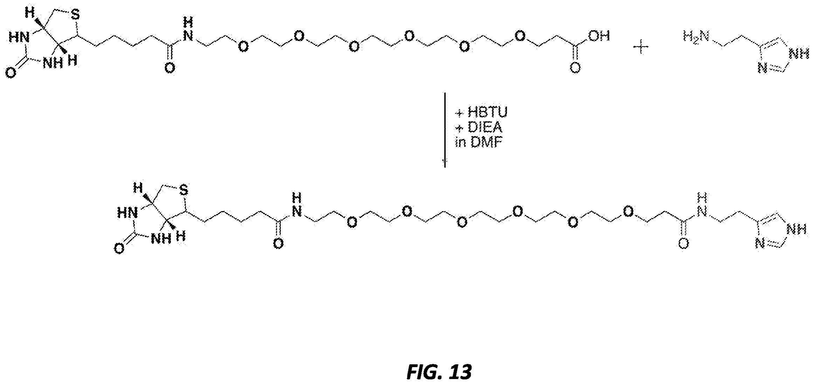

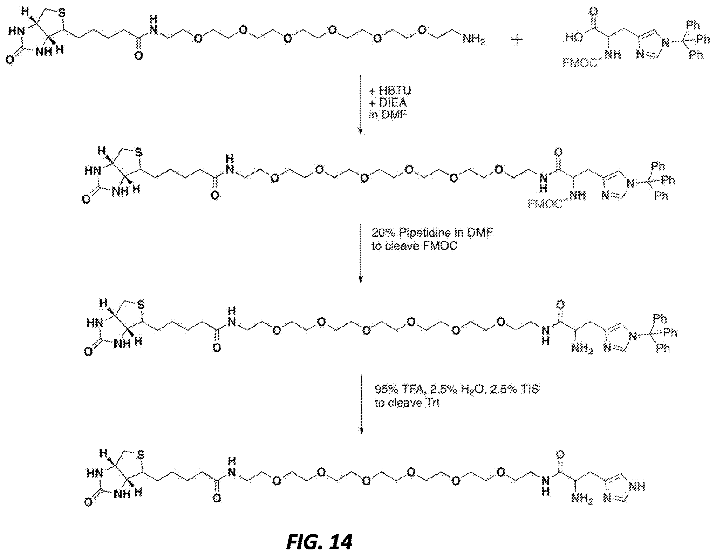

In the previous section, an assay developed using optimized conditions of commercial BSA-histamine conjugate with an assay using PEG-mono histidine linker was compared. In this section, a direct comparison study using streptavidin modified ELISA plates is made. Such a study was performed using streptavidin coated plates, followed by immobilization of either PEG-mono-histamine or PEG-mono-histidine linker as shown in **FIG. 8****.** The study was performed to highlight the effect of having a whole histamine molecule in place of histamine conjugate using the same PEG linker. **FIG. 13** and **FIG. 14** show the difference in linker structure with having histamine or histidine on a PEG linker chain. Both histidine and histamine were conjugated with biotin-PEG linker in solution. Briefly, histamine linkers (**FIG. 13**) were synthesized as follow. Biotin-EG6-COOH (0.4 mmol) was first activated with 2-(1H-benzotriazole-1-yl)-1,1,3,3-tetramethyluronium hexafluorophosphate (HBTU) (0.36 mmol) with addition of N,N-diisopropylethylamine (DIEA) in dimethylformamide (DMF). Thereafter, histamine (0.2 mmol) was added to the solution and reacted overnight at room temperature while agitating. DMF was removed using a rotavapor, and synthesized biotin-EG6-histamine molecule was purified on a C18 column on Reverse Phase High Pressure Liquid Chromatography (RP-HPLC) system and characterized by using liquid chromatography - mass spectroscopy (LC-MS). Exact mass of the Biotin-PEG-histamine molecule is 672.35.85, found 673.3. Purity of the synthesized molecule was >95%, identified using a Zorbax C18 analytical column. For synthesizing the histidine linkers (**FIG. 14**), fluorenylmethyloxycarbonyl (Fmoc) protected histidine amino acid (0.2 mmol) was activated with HBTU (0.18 mmol) with addition of DIEA in DMF. Subsequently, Biotin-EG6-amine (0.4 mmol) was added to the solution and reacted overnight at room temperature. Finally, DMF was removed using a rotavapor. This step was followed by the removing of the protection groups of the histidine amino acid as follow: Fmoc was cleaved by using 20% piperidine solution, and tripheylmethyl (Trt) group was removed using trifluoroacetic acid (TFA) cleavage cocktail (95% TFA, 2.5% water, 2.5% triisopropylsilane (TIS)). The synthesized Biotin-PEG-histidine molecule was purified on a C18 column using a RP-HPLC system and characterized by LC-MS. Exact mass of the Biotin-PEG-histidine molecule is 687.85, found 688.4. Purity of the synthesized molecule was >95%, identified using a Zorbax C18 analytical column.

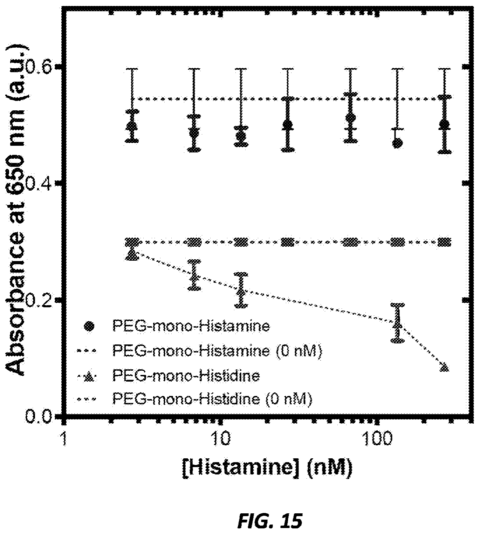

**FIG. 15** presents the data obtained for the detection of free histamine using PEG-mono-histamine and PEG-mono-histadine. PEG-mono-histidine linker was used as a positive control. A difference in terms of detecting free histamine was observed with PEG-mono-histidine as compared to PEG-mono-histamine as histamine could not be detected in the concentration range studied using PEG-mono-histamine. Without being bound by any specific mechanism it is proposed that the higher signal obtained with PEG-mono-histamine can be attributed to strong binding affinity of the antibody towards the conjugate leading to a lower sensitivity towards free histamine.

### V. Optimization of assay time and linker study

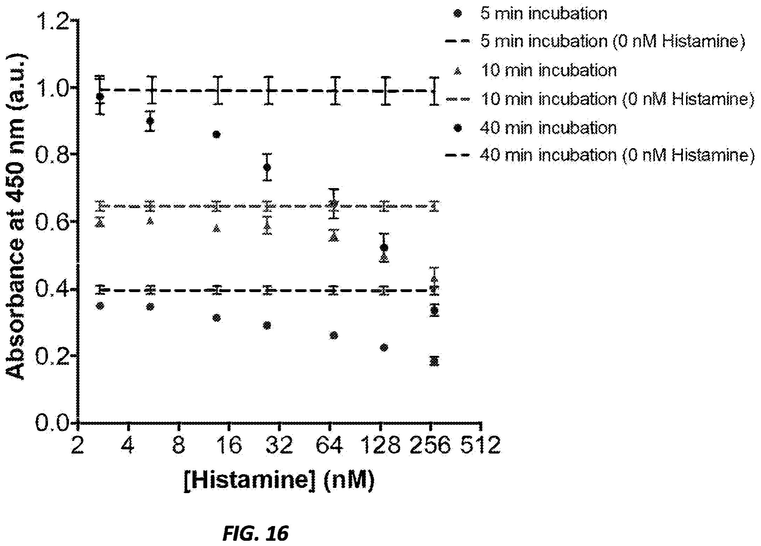

In order for the assay design to be used for clinical applications for the rapid quantification of allergy severity, reducing the assay time is one of the most challenging aspect. As per to date, no method exists to detect histamine in less than 10 minutes. To address such a challenge, the assay was further improved by designing to obtain a high starting signal. PEG-mono histidine was used for the study and incubation time was monitored for a 5 min, 10 min and 40 min followed by the usual 1 h incubation with anti-IgG HRP antibody. **FIG. 16** shows the results obtained from the study. As seen in the graph, higher starting signal was obtained from long incubation time. A lower starting signal was obtained with 5 min incubation however, the trend was similar to a 40 min incubation. The following studies were performed to decrease the assay time while retaining high detection sensitivity using the multi-valency approach for the linker designs.

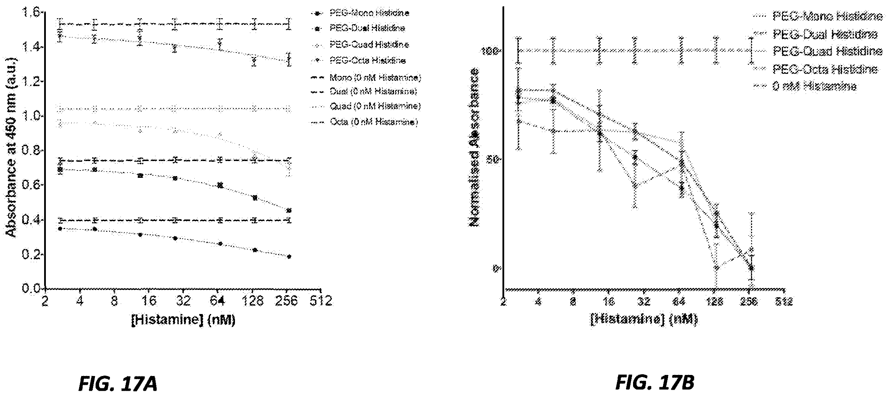

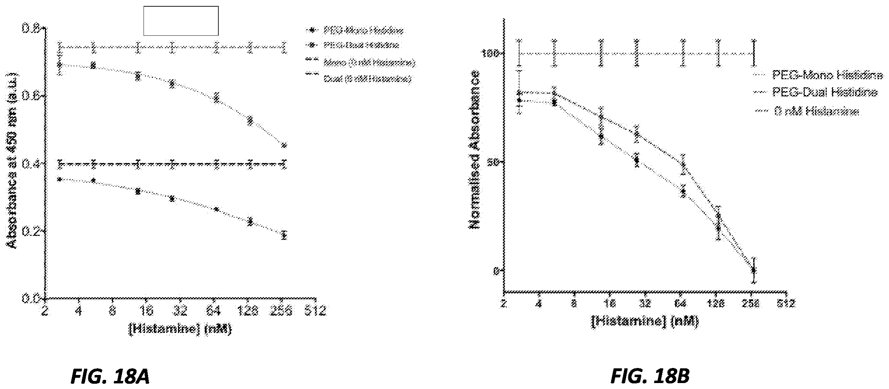

As seen in the previous experiments reported herein, using a multi-valent linker provides a higher starting (e.g., absorbance) signal as well as improving the sensitivity. Therefore, all the PEG-Histidine conjugates were compared using a 5 min incubation of anti-histamine antibody. Streptavidin modified plates were used to study mono, dual, quad, and octa-histidine linkers using the assay format depicted by **FIG. 8****.** The results obtained from all the linkers are presented in **FIG. 17A** and **17B. FIG. 17B** presents the normalized values of the data presented by **FIG. 17A****.** Although, a similar trend was seen in terms of detecting free histamine with all the linkers, quad and octa-histidine showed higher standard deviation from the trend line. This effect can be attributed to a higher affinity of the antibody towards linkers of higher valency. As the number of histidines was increased from two to four and eight, the affinity of the antibody increased towards the linker and, correspondingly, decreased towards free histamine. The data was further studied by considering only PEG-mono- and PEG-dual-histidine linker. **FIG. 18A** and **FIG. 18** **B** shows the comparison of PEG-mono and Dual Histidine, where **FIG. 18B** shows the normalized data. Both the PEG-mono- and PEG-dual-histidine linker showed a similar normalized response. However, an increased starting signal was obtained with the dual-histidine, so that the total signal in the dual-histidine case was larger. The PEG-mono and dual histidine linker conjugates were used to further refined the histamine assay.

### VI. Refinement of Histamine assay

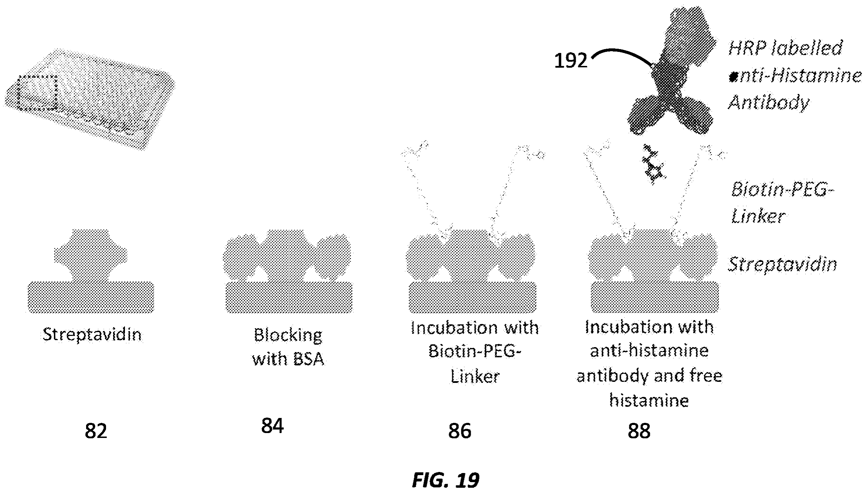

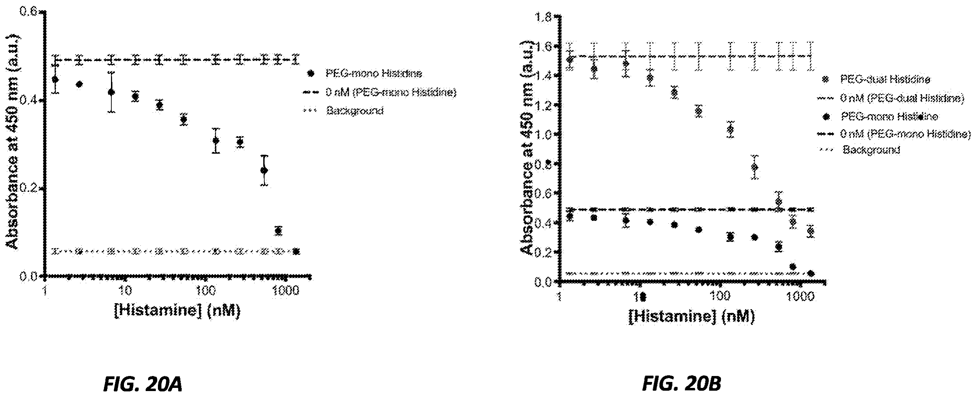

The assay was further refined to decrease the total assay time. This was achieved by conjugating the anti-histamine antibody to the detection enzyme HRP **192** as depicted by **FIG. 19****.** The resultant assay could be completed within 5 min. For the present study, only PEG-mono histidine and PEG-dual histidine were used. **FIG. 20A** and **FIG. 20B** show the results obtained from the study. **FIG. 20A** shows the plotted data for PEG-mono-histidine while **FIG. 20B** shows that plotted data for both the PEG-mono histidine and PEG-Dual histidine. The PEG-Dual histidine conjugate demonstrated improved performance in terms of higher starting signal.

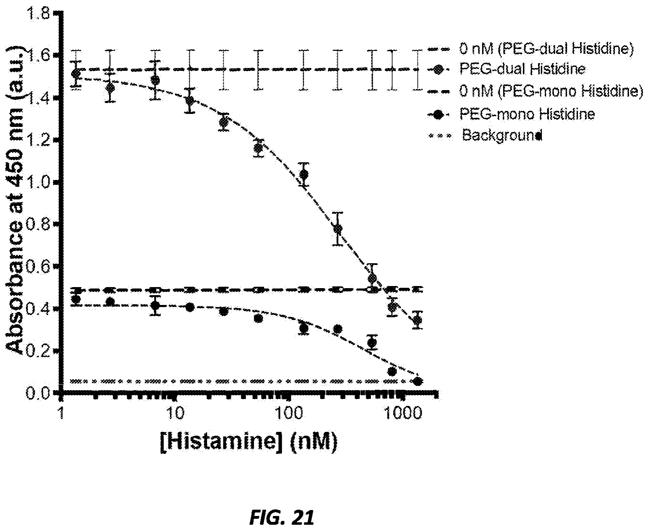

The data points obtained were fitted using a Hill equation which further assisted to characterize the linkers under study. **FIG. 21** shows the fitted values of the calibration curve without consideration of 0 nM Histamine data point. A dissociation constant (Kd) of 449 nM with an R square value of 0.95 was obtained with PEG-mono Histidine whereas, Kd of 280 nM with an R square value of 0.99 was obtained for PEG-dual Histidine. The PEG-dual histidine linker showed a broader dynamic range as compared to PEG-mono histidine.

Further experiments were made to study the use of BSA as a carrier protein (scaffold) to immobilize histidine linkers. For the study, maleimide modified BSA was used to link thiol-modified PEG-Mono-Histidine. The structure of the thiol-modified PEG-mono-Histindine is shown by **Structure 5:**

### Structure 5: thiol-modified PEG-mono-histindine:

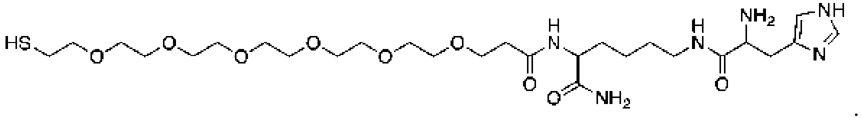

The synthesis is briefly described as follows. Thiol-PEG-mono-histidine (**Structure 5**) was synthesized on the Rink Amide LL resin (1111 mg). A 1-(4,4-dimethyl-2,6-dioxocyclohex-1-ylidene)isovaleryl (ivDde) protected Fmoc-Lys amino acid (1 mmol) was first attached to the Rink Amide LL resin using HBTU (0.9 mmol) and DIEA (100 µL). The reaction was allowed to proceed for 2 h. Thereafter, the Fmoc group of the lysine was removed and HBTU activated Thiol-EG6-COOH (0.72 mmol) was added to the resin and allowed to react for 1 h at room temperature while agitating. Next, the ivDde group on the Lys was removed using 2% hydrazine solution and the Fmoc-protected histidine amino acid (1 mmol) was reacted with the linker on the resin. After 1 h of reaction, the Fmoc group of the histidine was cleaved by using 20% piperidine in DMF, and synthesized linker was removed from the resin using TFA cleavage cocktail (92.5% TFA, 2.5% H2O, 2.5% TIS, 2.5% EDT), and collected. All organic solvents were removed before purification of the molecule on the C18 column using an RP-HPLC system. Thiol-PEG-mono-Histidine linker was characterized using LC-MS, and expected mass was 634.34; found 635.3. Purity of the synthesized molecule was >95%.

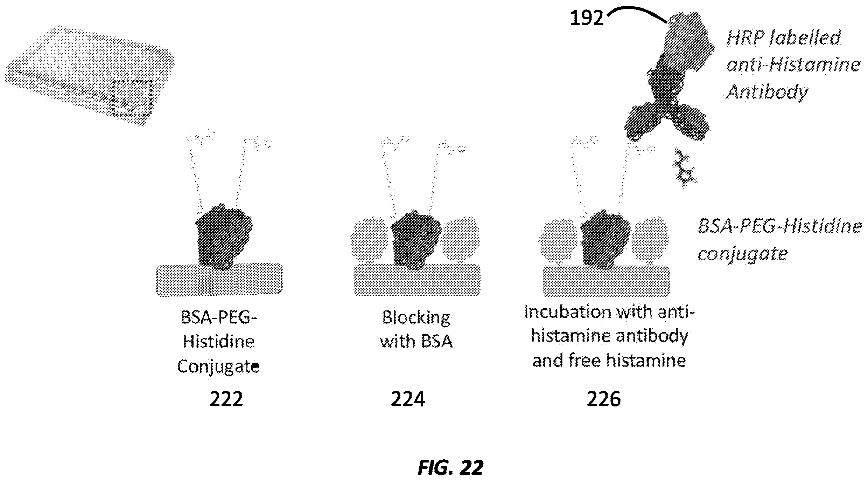

The thiol-PEG-mono Histidine linker was attached to BSA as follows. A 1 mL aliquot of maleimide conjugation buffer obtained from Thermo scientific was added to 10 mg of purified thiol-PEG-mono Histidine linker. Subsequently, the 1 mL solution containing the thiol-PEG-mono Histidine was added to 5 mg of commercially available BSA-maleimide substrate and allowed to react at room temperature for 4 h to provide the histidine-PEG-BSA conjugate. The histidine-PEG-BSA conjugate was purified using a 10K spin column. The histidine-PEG-BSA conjugate obtained was then used to modify the ELISA plate wells and assay was performed using a 5 min incubation time. **FIG. 22** shows schematically the assay design using the histidine-PEG-BSA conjugate. The steps include coating the plates with BSA-PEG-Histidine conjugate **222** , blocking with BSA **224,** and incubation with HRP labeled anti-histamine antibody and free histamine **226.**

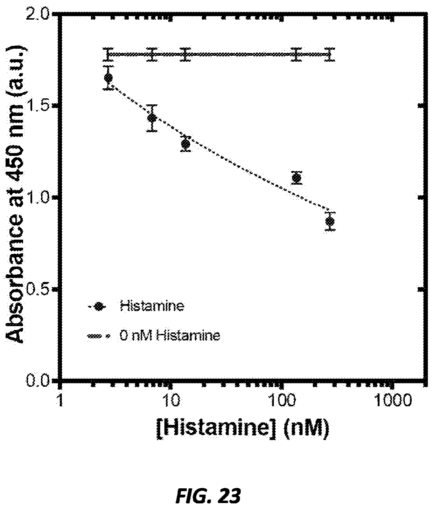

**FIG. 23** presents the results obtained and compared to BSA-histamine (commercial, **FIG. 5**). PEG-mono Histidine modified BSA showed increased sensitivity towards free histamine. The used and anti-histamine antibody labelled with HRP in a 5 min incubation time assay.

### VII. 5-minute electrochemical assay in spiked human plasma samples

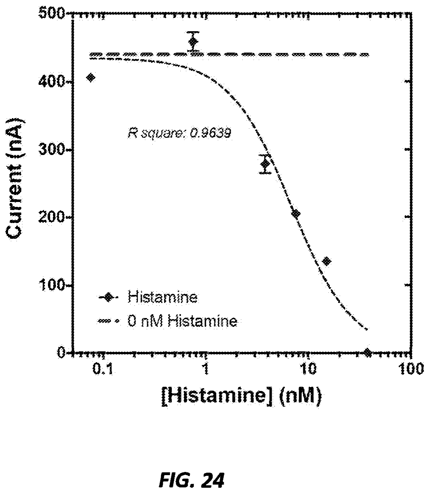

The Immuno-assay developed on the ELISA plates using PEG-dual Histidine was translated to an electrochemical platform to develop a biosensor for free histamine detection in human plasma samples. From the calibration curve obtained, 3.75 nM generated a statistically significant current change from 439 nA obtained with 0 nM histamine to 278 nA with 3.75 nM histamine (**FIG. 24**). A Hill equation was used to fit the curve without considering the 0 nM Histamine data point and a Kd value of 6.76 nM was achieved with R square value of 0.

### VIII. Examples using DNP molecules

Design of the single and multivalent linkers for the small molecule detection assays can be applicable to many small molecules. As shown above, the synthesis of histamine and histidine linkers have used HBTU chemistry to form amide bonds between carboxylic acid and amine groups of the molecules. Linkers with different functionality, such as biotinylated and thiolated linkers have also been synthesized.

To show that multivalent approach can be used for many other small molecule linkers in detection and surface modification applications, Dinitrophenol (DNP) versions of the linkers having the **Structures 6, 7, 8** and **9** shown herein can be prepared.

### Structure 6, Biotin-PEG-mono-DNP:

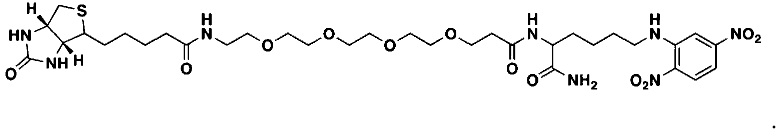

### Structure 7, Biotin-PEG-dual-DNP:

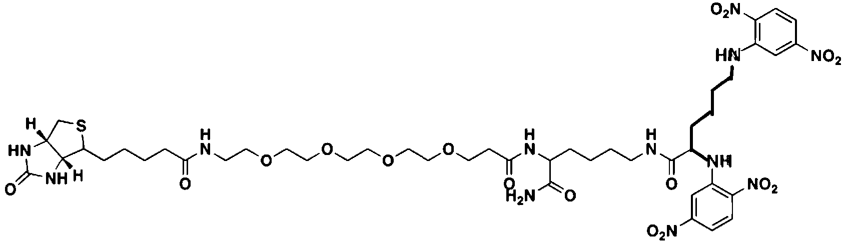

### Structure 8, Biotin-PEG-quad-DNP:

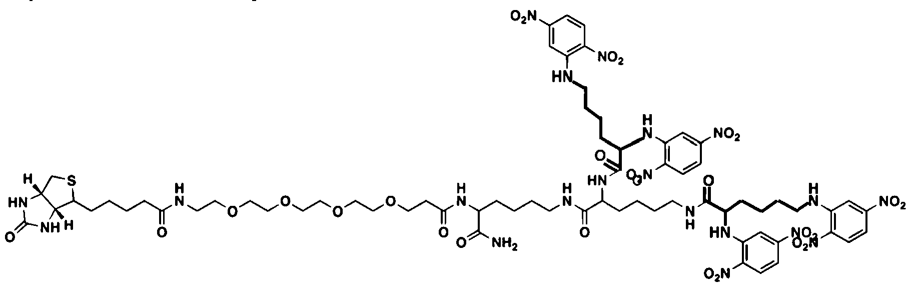

### Structure 9, Biotin-PEG-octo-DNP:

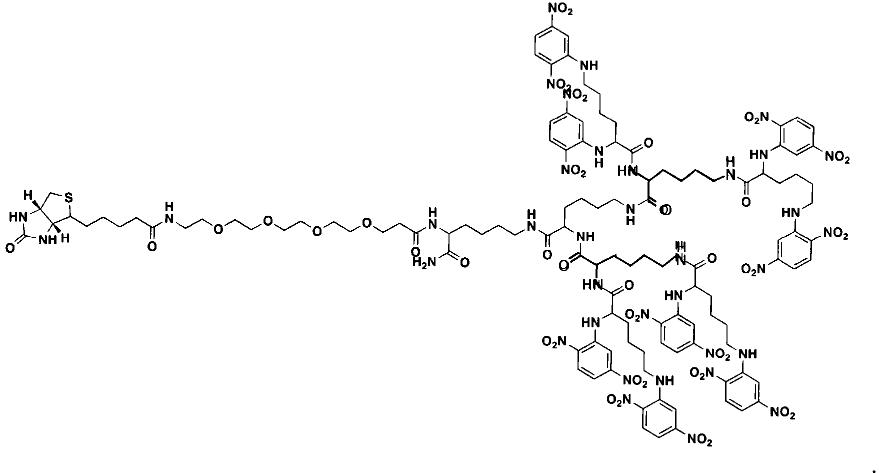

All DNP linkers were synthesized on the resin, starting with an ivDde protected Fmoc-Lysine amino acid. Fmoc group of the Lys was removed and then Biotin-EG4-COOH was attached to the linker. Once the ivDde protected group cleaved using 2% Hydrazine solution, resin was divided into 4 different synthesis vials; thus, mono **(Structure** 6), dual (**Structure 7**), quad (**Structure 8**), and octo-DNP (**Structure 9**) linker synthesis were followed in each resin vial. First vial was only received 4 molar access of 1-Fluoro-2,4-dinitrobenzene molecule, and reaction was carried for 1 h at room temperature while agitating. In the second vial, first Fmoc-Lys(Fmoc)-OH was attached to the linker using HBTU chemistry and then 1-Fluoro-2,4-dinitrobenzene molecule was coupled to the amine groups of the Lys residue. In the third vial, Fmoc-Lys-Fmoc-OH addition to the resin was followed twice using HBTU chemistry, and then Fmoc groups were cleaved and leaved four primary amine groups on the Lys residues, which were used to couple 1-Fluoro-2,4-dinitrobenzene molecule to the linker. In the last vial, same Fmoc-Lys-Fmoc-OH addition protocol was followed three times in a row, and the last cleavage of Fmoc groups of the Lys residues left eight primary amine groups on the resin that were used for attaching eight 1-Fluoro-2,4-dinitrobenzene molecule for each linker. After synthesis, all linkers were cleaved from the resin using TFA cleavage cocktail (95% TFA, 2.5% H₂O, and 2.5% TIS), and organic solvents were removed from the vials using a rotavapor. Purification and characterization of these linkers are still in progress.

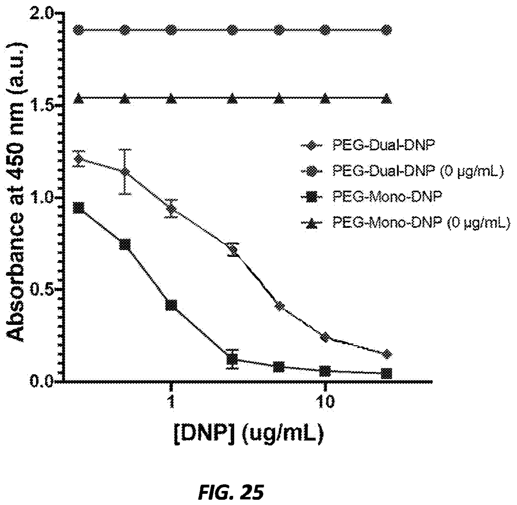

Monoclonal Anti-Dinitrophenyl antibody produced in mouse (D8406) and 2,4-Dinitrophenol (D198501 was purchased from Sigma. The anti-DNP antibody was directly conjugated with HRP using a commercial kit e.g., LIGHTENING-LINK^{™} Antibody Labeling Kits available from Novus Biolgicals. Once again ELISA plate coated with streptavidin was used as described in **FIG. 19****.** After blocking, the plates were incubated with the respective linker. Different concentrations of DNP with anti-DNP antibody was co-incubated for 45 minutes at room temperature in the dark. After incubation, the wells were washed 3 times with 200 µL PBST and incubated with 150 µL TMB for 10 minutes. Finally, 150 µL of stop solution was added and absorbance measurement was observed. **FIG. 25** is a plot showing ELISA results with Mono-DNP linker (blue) versus PEG-Dual- DNP linker (red). As with the dual histadine linkers, a clear difference could be seen between single and dual DNP linker. There is nearly a 30% increase in the signal intensity from single to dual DNP molecule. Moreover, a broader dynamic range is also observed with dual DNP.

### IX. Histamine Assay Protocols using a solid support

Histamine detection assay could be performed in two formats. The first format includes the immobilization of histamine conjuagtes to the solid support while the second format includes the immobilization of anti-histamine antibody on the solid support. For the immobilization of histamine conjugates the immobilization sample was prepared in 0.2 M carbonate-bicarbonate buffer (pH 9.4). Immobilisation samples herein refers to BSA-histamine, Streptavidin, and not limited to BSA-Histidine Samples. A high binding polystyrene 96 well ELISA plate was coated with 100 microliters of immobilization sample and the plates were incubated overnight at 4° C. Each well was then washed thrice with 0.01M phosphate-buff ered saline with 0.05% Tweeen 20 (pH 7.4). To each well was then added 250 microliters 1% BSA solution prepared in 0.01M phosphate-buffered saline with 0.05% Tweeen 20 (pH 7.4) for 1 hour at ambient temperature. Each well was then washed three times with 0.01M phosphate-buffered saline with 0.05% Tweeen 20 (pH 7.4). For Strepatvidin coated plates, to each well was then added 100 microliters of linkers prepared in 0.1 % BSA in 0.01M phosphate-buffered saline with 0.05% Tweeen 20 (pH 7.4) as described in the other sections for 2 hours at ambient temperature on a shaker at 260 rpm. Each well was then washed three times with 0.01M phosphate-buffered saline with 0.05% Tweeen 20 (pH 7.4). The plate was then incubated with 50 microlitres of different concentration of free histamine and co-incubated with anti-histamine antibody for 1 hour at ambient temperature. at 25° C. for thirty minutes. Anti-histamine antibody was prepared in 0.1 % BSA in 0.01M phosphate-buffered saline with 0.05% Tweeen 20 (pH 7.4). After the incubation, each well was washed three times with 0.01M phosphate-buffered saline with 0.05% Tweeen 20 (pH 7.4). The plates were then incubated with a secondary antibody labelled with HRP prepared in 0.1 % BSA in 0.01M phosphate-buffered saline with 0.05% Tweeen 20 (pH 7.4) in for 1 hour at ambient temperature. After the incubation, each well was washed three times with 0.01M phosphate-buffered saline with 0.05% Tween 20 (pH 7.4). Each well was then incubated with TMB turbo (150 microlitres) for 30 minute (measurement at a wavelength of 650 nm) followed by addition of 150 microlitres of 0.16 M Sulphuric acid to stop the solution in some examples where measurements are taken at a wavelength of 450 nm. For refined histamine assay, the anti-histamine antibody was directly conjugated to HRP using acommercial conjugation kit as previously described and hence the secondary antibody step was not included. Independent incubation time is described in each section.

### X-Comparison of PEG-mono-Histidine with PEG-mono-glutamate Histidine and PEG-mono-Phenyl Histidine

In a previous section, an assay developed using different valency of linkers was examined. In this section, a linker design with neutral **Structure 12** or charged **Structure 13** groups is examined. The study was performed to highlight the effect adding bulky **Structure 12** or charged **Structure 13** group on sensitivity. Thus **Structure 13** has a glutamate group with a single histidine, or a gluatamine-histadine small molecule attached to the PEG chain; while **Structure 12** has a phenyl group with single histidine, or a histidine-phenylalanie small molecule attached to a PEG chain. Both linkers were conjugated with biotin-PEG linker in solution. Briefly, histamine linkers were synthesized as follow. Histidine linkers were synthesized on the Nova PEG Rink Amide LL resin, starting with an ivDdE protected FMOC-Lysine amino acid, dissolved in DMF with addition of DIEA, and activated with HBTU and then conjugated to the resin with 2 h incubation under agitating condition at room temperature. FMOC group of the Lys was removed using 20% Piperidin. Biotin-EG4-COOH dissolved in DMF with addition of DIEA and HBTU was attached to the linker with a 1 h incubation while agitating at RT. Then, ivDdE protection of Lys was cleaved using 2% Hydrazine solution. To synthesize the phenyl version of the linker, FMOC-Phe-OH amino acid addition to linker was carried for 1 h at room temperature while agitating (**Structure 12**). Similarly, to synthesize the glutamate version of the linker (**Structure 12**), FMOC-Glu(OtBu)-OH molecule was added to the reaction after ivDdE cleavage by 2% Hydrazine solution. Lastly, FMOC-His(Trt)-OH aminoacid was added to the linker using the same reaction conditions. After synthesis, all linkers were cleaved from the resin using TFA cleavage cocktail (95% TFA, 2.5% H2O, and 2.5% TIS), and organic solvents were removed from the vials using a rotavapor. The final product was purified using a C18 column on RP-HPLC system and characterized by using mass spectroscopy (MALDI). The Biotin-PEG-phenyl-histadine, Structure 12 molecule has a chemical formula C₄₂H₆₆N₁₀O₁₀S with an exact mass of 902.468, and a molecular weight of 903.110. The Biotin-PEG-glutamate-histadine, Structure 13 molecule has a chemical formula C₃₈H₆₄Ni₁₀O₁₂S with an exact mass of 884,443 and molecular weight of 885.048.

### Struture 12; Biotin-PEG-phenyl-histadine

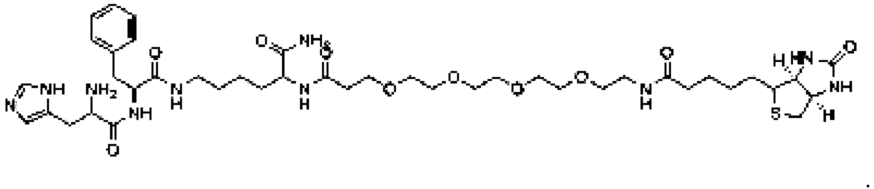

### Structure 13; Biotin-PEG-glutamate-histadine

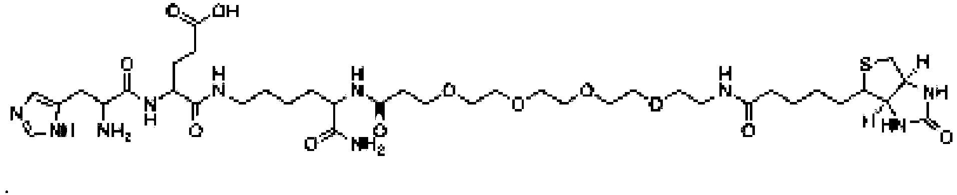

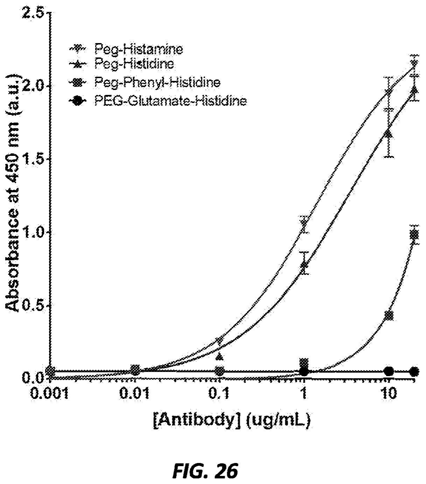

**FIG. 26** is a plot of a comparison of PEG-mono histamine with PEG-mono histidine using streptavidin coated ELISA plates. As illustrated, the data presents the affinity of anti-histamine antibody conjugated to HRP to different histamine conjugate linkers. As in the previous studies, streptavidin modified plates were used, here to study PEG-Mono-Histamine, PEG-mono-Histidine, PEG-phenyl-histidine and PEG-glutamate-histidine. The assay format was as per **FIG. 19** without free histamine in the solution. The modified plates were then exposed to different concentrations of anti-histamine antibodies conjugated with HRP for 5 minutes. **FIG. 26** clearly shows a high binding affinity of anti-histamine antibody with PEG-Mono-Histamine compared to PEG-mono-histidine linker. The affinity is decreased significantly when tested with PEG-phenyl-histidine and nearly no binding was observed with PEG-glutamate-histidine with the antibody concentration tested.

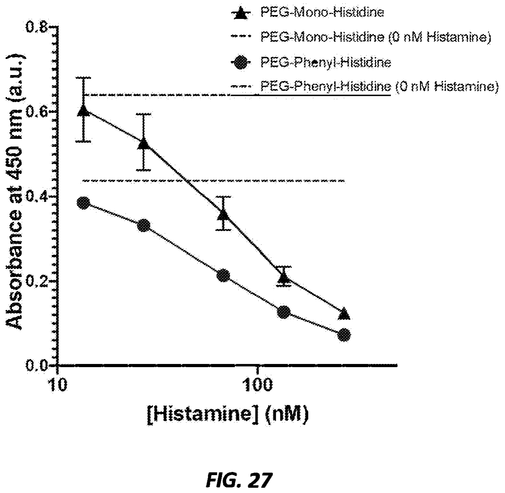

Further testing compared the performance of 5-minute histamine assay with PEG-mono-histidine and PEG-phenyl-histidine as illustrated illusted in the plotted data of **FIG. 27****.** Because of lower affinity of anti-histamine antibody against PEG-phenyl linker, clearly a lower starting signal was obtained as compared to PEG-mono-histidine. However, no significant difference was observed otherwise on the sensitivity of the assay.

### XI-Development of surface chemistry for ELISA plate to detect small molecules like histamine in complex matrices like human plasma

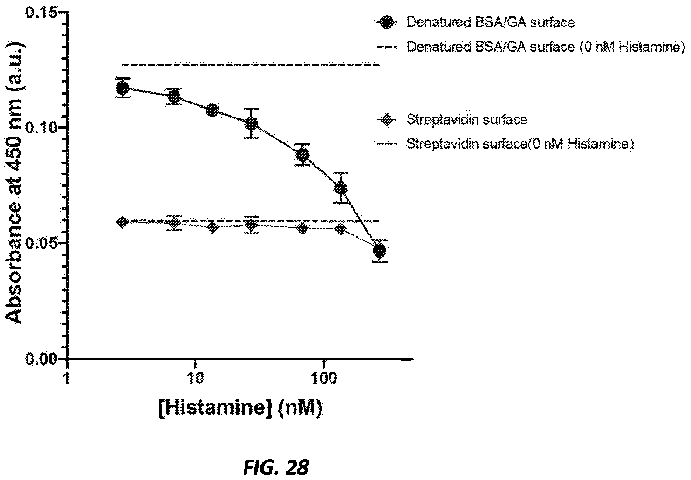

In order for the assay design to be used for clinical applications for the rapid quantification of allergy severity, using clinical samples (e.g. human plasma, serum) is one of the most critical aspects. An ELISA plate was prepared using conventional means as shown in **FIG. 19** using PEG-dual-histidine. In an alternative approach, an ELISA plate was prepared using the BSA-glutaraldehyde surface chemistry as previously described in international Application No. PCT/US2018/044076, the content of which is herein incorporated by reference. In contrast to the previously described sytem, no nanoparticles were used. Therfore BSA can be denatured and combinded with glutaraldehyde and used to coat ELISA wells. This is then activated using and (1-ethyl-3-(3-dimethylaminopropyl)carbodiimide hydrochloride)/Dicylohexylcarbodiimide (EDC/NHS) to link to streptavidin which allows attachment of biotin-PEG-dual-hitadine. The developed platform was tested with spiked human plasma samples and the results are presented in **FIG. 28****.** It can be clearly seen full blocking of the ELISA plate when human plasma samples are used for histamine detection. However, with BSA-glutaraldehyde surface chemistry, a clear detection of histamine was observed in under 10-minute assay. As per to date, no method exists to detect histamine in less than 10 minutes. In conclusion, with incorporation of a BSA-glutaraldehyde coating before immobilization of streptavidin for linker attachment, a rapid and sensitive histamine assay was developed for spiked human plasma samples.

## Claims

1. A method for detecting presence of an analyte in a sample, the method comprising:
(i) contacting a sample suspected of comprising an analyte with a compound comprising:
(a) a substrate binding domain, wherein the compound is linked to a substrate via the substrate binding domain, wherein the substrate is any of a solid, semi-solid, or polymer;
(b) a branching domain comprising a plurality of small molecules each small molecule linked to a branch of a branch-point, wherein the small molecules have a molecular weight of less than 1,000 Da; and
(c) a linker linking the substrate binding domain and the branching domain and
(ii) detecting binding of an analyte binding molecule to the compound.

2. The method of claim 1, wherein:
(a) the analyte binding molecule is an antibody; or
(b) said detecting of step (ii) comprises producing a chromogenic, fluorescence or electrochemical signal; or
(c) the analyte binding molecule comprises a detectable label; or
(d) said detecting step comprises contacting the sample from (i) with a molecule capable of binding with the analyte binding molecule and comprises a detectable label; or
(e) the analyte is histamine or dinitrophenol.

3. The method of claim 1, wherein the substrate is an electrode, and wherein the compound is linked to the electrode surface by the substrate binding domain and the analyte binding molecule includes an electroactive component, and wherein
(i) the analyte binding molecule is detected by the electrode when the electroactive component is proximate to the electrode, optionally wherein:
the linker length is greater than 5 Å and less than 200 Å,
the electrode detects the analyte binding molecule by direct redox reaction with the electroactive component or by a sacrificial redox active species, or
the analyte binding molecule is an antibody specific to the analyte, and the electroactive component is a biotinylated detection antibody conjugated to streptavidin-polyHRP and the electrode detects the sacrificial redox active agent 3,3',5,5'-Tetramethylbenzidine (TMB).

4. A method for selecting a ligand capable of binding a small molecule, the method comprising:
(i) contacting a test ligand with a compound comprising:
(a) a substrate binding domain, wherein the compound is linked to a substrate via the substrate binding domain, wherein the substrate is any of a solid, semi-solid, or polymer;
(b) a branching domain comprising a plurality of small molecules each small molecule linked to a branch of a branch-point, wherein the small molecules have a molecular weight of less than 1,000 Da; and
(c) a linker linking the substrate binding domain and the branching domain; and
(ii) detecting binding of the test ligand with the compound the presence and in the absence of the small molecule, and
selecting the test ligand having reduced binding in the presence of the small molecule.

5. The method of claim 4, wherein:
(a) the test ligand is selected from the group consisting of antibodies, adnectins, ankyrins, antibody mimetics and other protein scaffolds, aptamers, nucleic acid (e.g., an RNA or DNA aptamer), proteins, peptides, oligosaccharides, polysaccharides, lipopolysaccharides, cellular metabolites, cells, viruses, subcellular particles, haptens, pharmacologically active substances, alkaloids, steroids, vitamins, amino acids, avimers, peptidomimetics, hormone receptors, cytokine receptors, synthetic receptors, sugars and molecularly imprinted polymer; or
(b) the test ligand is an antibody; or
(c) said detecting of step (ii) comprises producing a chromogenic, fluorescence or electrochemical signal; or
(d) the ligand comprises a detectable label, optionally the detectable label is a chromogenic, fluorescent or redox active group, or
(e) said detecting step comprises contacting the ligand from (i) with a molecule capable of binding with the ligand and comprises a detectable label.

6. The method of any one of claims 1-5 wherein:
i. the substrate is a microparticle or a microbead, a nanotube, a microtiter plate, an electrode, a medical apparatus, a microchip, a filtration device, a membrane, a diagnostic strip, a dipstick, an extracorporeal device, a microscopic slide, a hollow fiber, a hollow fiber cartridge, an electrode surface or any combinations thereof; or
ii. the substrate is a microparticle or a microbead, a microtiter plate, an electrode, a membrane, a diagnostic strip, a dipstick, an ELISA plate, or a microscopic slide.

7. The method of claim 6, wherein the substrate is an electrode, a diagnostic strip, a dipstick, or an ELISA plate.

8. The method of claim 6 or 7, wherein a surface of the substrate is coated with a proteinaceous material, wherein the proteinaceous material can optionally be reversibly or non-reversibly denatured and/or cross-linked, optionally, the proteinaceous material is denatured BSA which is cross-linked with glutaraldehyde.

9. A method for raising antibodies specific to a small molecule, the method comprising contacting T cells with a compound comprising:
(a) a substrate binding domain wherein the compound is linked to a substrate via the substrate binding domain, wherein the substrate binding domain is a reactive group or one member of a binding pair;
(b) a branching domain comprising a plurality of small molecules each small molecule linked to a branch of a branch-point, wherein the small molecules have a molecular weight of less than 1,000 Da; and
(c) a linker linking the substrate binding domain and the branching domain.

10. The method of any one of claims 1-9, wherein:
i. the linker has a length between 5 Å and less than 200 Å; and/or
ii. the linker comprises a polyethylene glycol (PEG) having a molecular weight of less than 2,000 Da, or the linker comprises a PEG having from 2 to 45 repeat units.

11. The method of any one of claims 1-10, wherein the small molecules independently have:
i. a molecular weight of higher than 50 g/mol; and/or
ii. a molecular weight of between 50 and 600 g/mol.

12. The method of any one of claims 1-11, wherein the small molecule is selected from the group consisting of amino acids, amino acid dimers, nucleosides, saccharides, steroids, hormones, drugs, or conjugates thereof.

13. The method of any one of claims 1-12, wherein the small molecules are histidine, a histidine-phenylalanine dimer, or dinitrophenol (DNP).

14. The method of any one of claims 1-13, wherein the branch-point comprises at least one lysine, and optionally wherein:
(i) at least one small molecule is linked to the alpha-amino group the at least one lysine and at least one small molecule is linked to the epsilon-amino group of the at least one lysine,
(ii) the branch-point comprises a first lysine linked to a second lysine, and wherein the carboxyl group of the first lysine is linked to the epsilon-amino group of second lysine, or
(iii) the branch-point comprises a first lysine, a second lysine and a third lysine, and wherein the carboxyl group of the first lysine is linked to the epsilon-amino group of the second lysine, and the carboxyl group of the third lysine is linked to the alpha-amino group of the first or second lysine.

15. The method of any one of claims 1-14, wherein the branch-point is selected from the group consisting of: 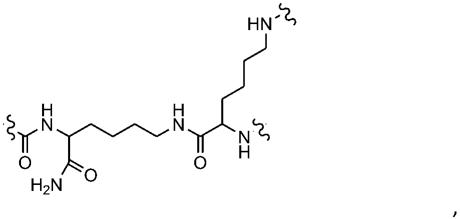 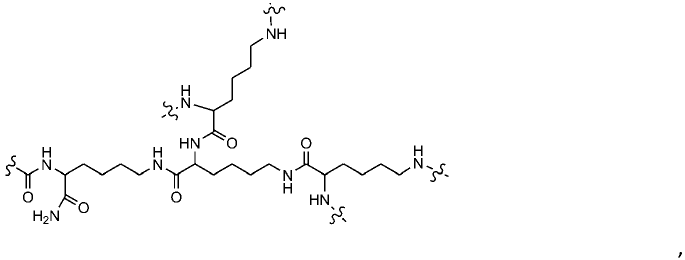 and

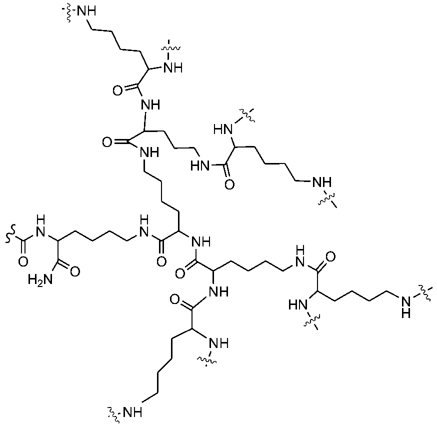

16. The method of any one of claims 1-15, wherein the branching domain:
(i) comprises from 2 to 20 small molecules linked to the branch-point; or
(ii) is selected from the group consisting of: 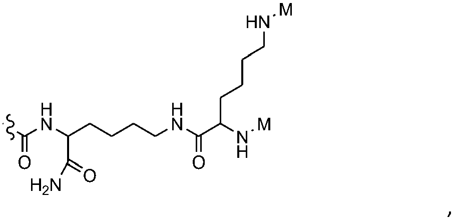 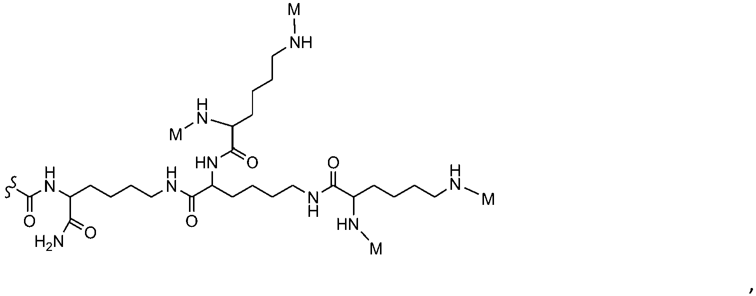 and 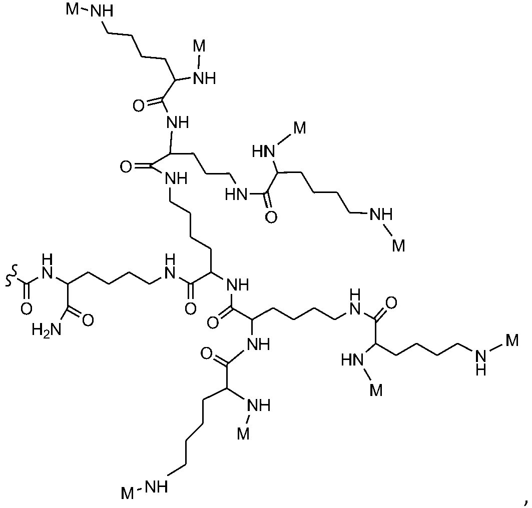 wherein each M is a small molecule; or
(iii) is selected from the group consisting of: 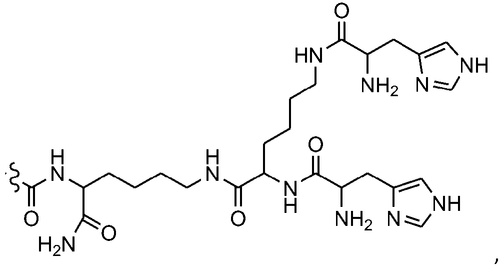 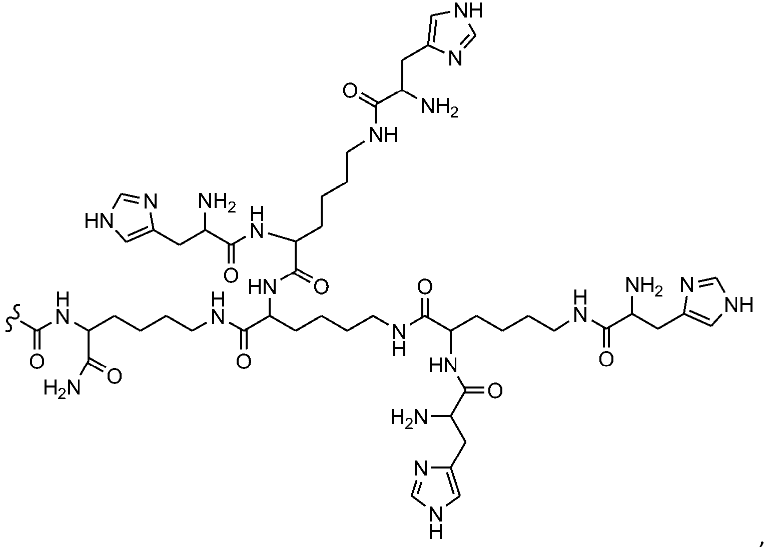 and 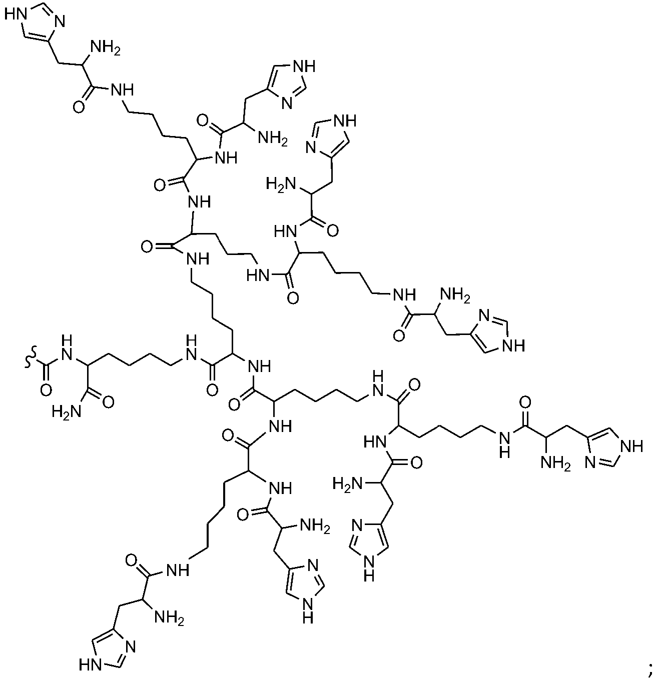 or
(iv) is selected from the group consisting of: 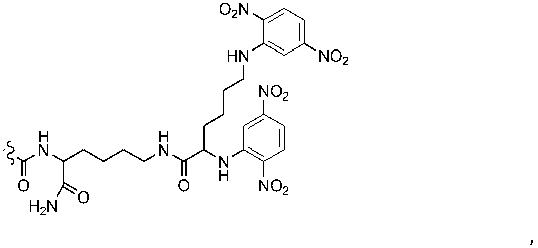 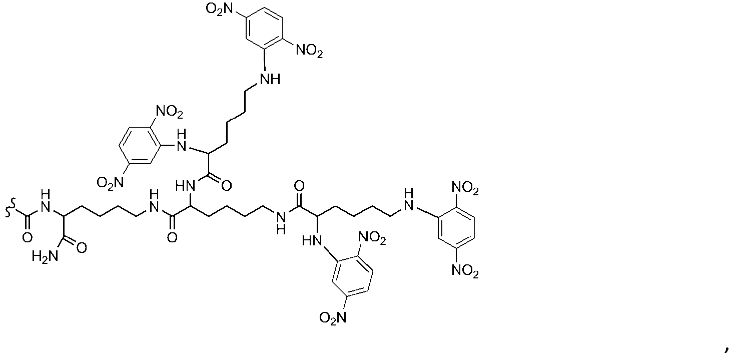 and 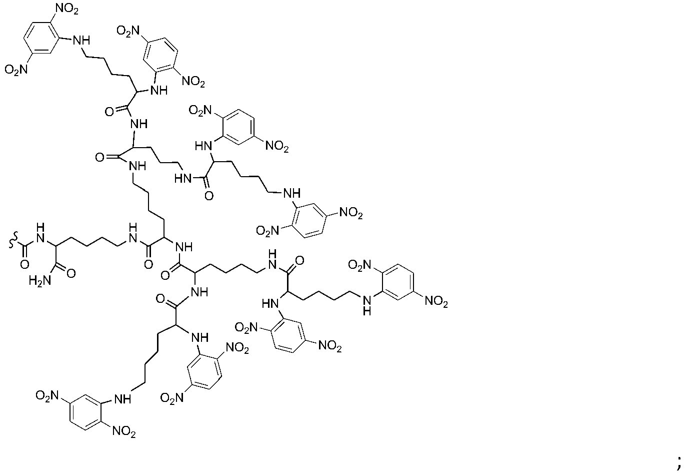 or
(v) comprises: 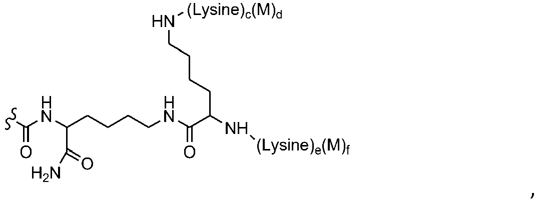 wherein:
d + f ≥ 2 (e.g., between 2 and 100), d ≥ c, and e ≥ f, wherein c, d, e and f are integers and each M is a small molecule, optionally wherein M is histidine or dinitrophenol;
or
(vi) has the formula C(x)ₐM_{b},
wherein:
C is a sub unit of the branching domain having a maximum of possible x branches, and the branch-point comprises one or more sub unit C and at least one subunit C is attached to the linker through a branch;
M is a small molecule attached to the subunit C through a branch;
a is an integer ≥ 1; and
b is an integer ≥ 2, provided that b ≤ (a)(x-2) +1.

17. The method of any one of claims 9-16, wherein the substrate binding domain comprises a reactive group or one member of a binding pair, optionally wherein:
i. the reactive group is selected from the group consisting of alkyl halide, aldehyde, amino, bromo or iodoacetyl, carboxyl, hydroxyl, epoxy, ester, silane, thiol, and the like; or
ii. the binding pair is biotin-avidin, biotin-streptavidin, complementary oligonucleotide pairs capable of forming nucleic acid duplexes, a thiol-maleimide pair, a first molecule that is negatively charged and a second molecule that is positively charged.

18. The method of any one of claims 9-17 wherein the substrate binding domain comprises a thiol group or a biotin molecule.
